# EUROPEAN PATENT APPLICATION

(11) **EP 2 157 088 A1**
(43) Date of publication of application: **24.02.2010**
(21) Application number: 07834942.0
(22) Date of filing: 11.04.2007
(51) Int. Cl.: C07D 403/06, C07D 209/18, C07D 209/08, C07D 209/24, C09B 23/08, G01N 33/533

(54) **INDICYANINE DYES AND THE DERIVATIVES THEREOF FOR ANALYSING BIOLOGICAL MICROMOLECULES**

(71) Applicant: UCHREZHDENIE ROSSIISKOI AKADEMII NAUK INSTITUT MOLEKULYARNOI BIOLOGII IM. V.A. ENGELGARDTA RAN (IMB RAN), Moscow 119991 (RU)
(72) Inventor: CHUDINOV, Alexandr Vasilievich, Moscow, 127549 (RU); KUZNETSOVA, Viktoria Evgenievna, Moscow, 129329 (RU); ZASEDATELEV, Alexandr Sergeevich, Moscow, 117418 (RU); BARSKY, Viktor Evgenievich, Moscow, 115167 (RU)
(74) Representative: HOFFMANN EITLE
(86) International application number: PCT/RU2007/000172
(87) International publication number: WO 2008/127139

(57) **Abstract**

The invention relates to two series of synthesised indolenine pentamethine dyes (37 compounds) comprising a reactive group in the third and fifth positions of the indolenine cycle, respectively. A method for producing, extracting and purifying the dyes and the precursors thereof, consisting in determining the absorption and fluorescence maxima, molecular extinction factors, fluorescence quantum yields, relative fluorescence effectiveness at excitation on a wavelength of 635 nm (detection of 670 nm) and 655 nm (detection of 690 nm), relative light stability and comparative sensitivity of detection of marked oligonucleotides on biochips of the claimed dyes, is disclosed. The use of the claimed dyes in the form of fluorescent marks for oligonucleotide and protein chips is also disclosed.

## Description

### Field of the Invention

The present invention relates to fluorescent indocyanine dyes and derivatives thereof for analyzing biological macromolecules. The invention also relates to a method for producing compounds titled.

### Background of the Invention

Fluorescent labeling is one of methods for revealing the results of biological analysis in immunologic and hybridization assays, specifically, in the engineering of biological microchips, in microscopic cytometry, in flow cytometric sorting, etc. For all these methods, the compound analyzed is firstly detected by using a complementary compound. Thereafter, the amount of specific complexes formed is measured by intensity of label fluorescence.

A fluorophore nucleus of polymethine dyes is known to consist of two heterocyclic moieties connected with a conjugated polymethine chain of atoms. Absorption and fluorescence maxima of fluorophore strongly depend on the length of polymethine chain, the nature of heterocyclic moieties, and to a lesser extent, on the nature of substituent and the site of binding thereof to fluorophore. The molar extinction coefficient and fluorescence quantum efficiency, i.e. an ability to absorb energy and to fluoresce, strongly depend on the molecular environment of fluorophore, the type of substituent, and the site of binding thereof to fluorophore.

Having been activated, dyes with reactive chemical groups are capable to transform into active groups and to be covalently bound with other molecules, are used in biological assay for fluorescent labeling biomolecule, proteins, nucleic acids etc. For this purpose, carboxyl group (-COOH) being bounded with the amino group of biomolecule is used more frequently.

Subject to statement of problem and record of analysis results, the performance requirements for fluorescent label are somewhat varied. The general requirements are as follows:
1. A label should not block the formation of specific complexes of analyzed compound and fluorescent-labeled probe, in maximal number;
2. A label must provide the instrument recording the number of specific complexes formed, with maximum sensitivity and accuracy;
3. The method for producing labeled preparations being used for analysis should be reproducible, with the procedure being not complicated;
4. During a labeling process, preparation characteristics should remain at most, and degradation should be minimal.

The polymethine dyes are indole derivatives (indocyanine dyes), being characterized with high efficiency, brightness of fluorescence, in combination with satisfactory chemical stability and light resistance.

For binding biomolecules, the reactive carboxyl group makes a component of molecule of the indocyanine dye. There is strong distinction between properties of biomolecules labeled with various fluorescent labels based on a sole fluorophore. The nature of substituent and the site of binding thereof with the fluorophore depend on solubility, the formation of molecular aggregates, and brightness of fluorescence for labeled biomolecules and specific complexes thereof. An orientation of the fluorophore label with respect to labeled biomolecule and the length of polymethylene chain moving the fluorophore off the biomolecule substantially influence the nonspecific binding of labeled biomolecules. As a result, substituent being included in the molecule of fluorescent label has a significant effect on the total bioassay sensitivity.

It is impossible to predict the nature of substituent and the sites of binding thereof with fluorophore for the purpose of producing the most efficient fluorescent label. For the purpose of searching new efficient fluorescent labels, various substituents are inserted into the known fluorophores, and there are changed sites for attaching a reactive carboxyl group to a heterocyclic moiety of fluorophore and the length of polymethylene chain moving the carboxyl group off the fluorophore.

Indocyanine dyes are prepared by condensation of two identical or different, indolenynes and polymethine precursor (Scheme 1). Next, the procedure of isolation and purification of the desired product from a reaction mixture of complex composition follow.

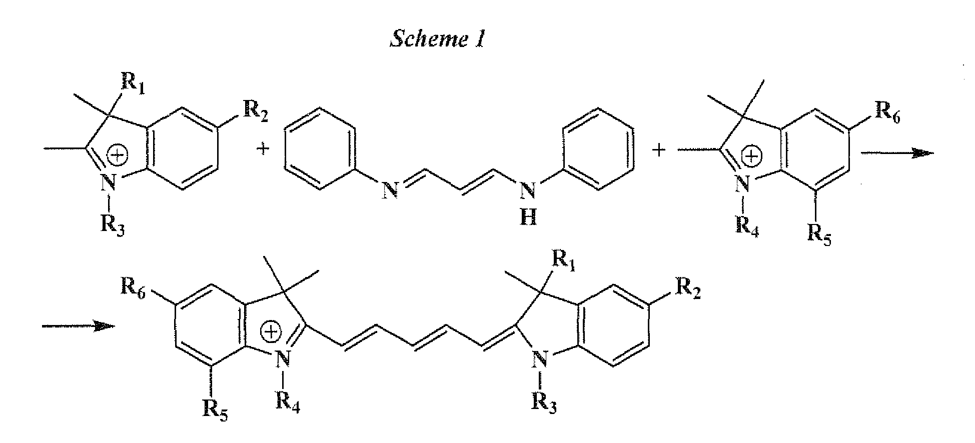

There are well known indolenynes having various non-reactive substituents, namely alkyl-, sulfo-, sulfoalkyl, and fused aryl groups. The polymethine precursors are also known.

The new carboxyl containing indolenyne each presents the basis for producing a novel series of indocyanine dyes having various substituents and different properties respectively.

References (Mader O. et al, Bioconjugate Chem, 2004, vol. 15, pp.70-78*;* Mank A.J.G. et al., Anal. Chem.,1995, vol. 67, pp. 1742-1748*;* Mujumdar R.B., et al., Biocojucg. Chem, 1993, vol. 4, No.2. pp.105-111*)* disclose the indocyanine dyes, wherein reactive carboxyl group being attached to 1-position of indolenyne moiety, - to heterocyclic nitrogen atom via polymethylene chain of atoms (R₃= -(CH₂)₅COOH, wherein R₁, R₂, R₄, R₅, R₆ are different alkyl-, sulfo-, sulfoalkyl, and fused aryl substituents).

References (Mank A.J.G. et al., Anal. Chem., 1995, vol. 67, pp. 1742-1748*;* Southwick et al., Cytometry, 1990; 11(3): 418-30*;* Lin Y. Et al., Bioconjuc. Chem., 2002, vol. 13, pp. 605-610*)* describe indocyanine compounds, wherein a reactive carboxyl group being attached to position 5 of indolenyne moiety directly or via one methylene group (R₂=-COOH or -CH₂COOH, wherein R₁, R₃, R₄, R₅, R₆ are different alkyl-, sulfo-, sulfoalkyl, and fused aryl substituents).

References *(*WO 02/26891 A1*;* EP 1211294 A1*)* disclose indocyanine dyes, wherein a reactive carboxyl group being attached to position 3 of indolenyne moiety via pentamethylene chain of atoms ( R₁= -(CH₂)₅COOH, wherein R₁, R₂, R₄, R₅, R₆ are different alkyl-, sulfo-, sulfoalkyl, and fused aryl substituents).

The abovementioned dyes, however, have some drawbacks. Specifically, in case the reactive group R₃ of dye is attached to 1-position of indolenyne moiety, then it is located on the same plane as the substituent R₄ and experiences a steric hindrance caused by this group. Therefore, a probe (biomolecule) being attached to carboxyl group, as located in substituent R₃, will also experience a steric hindrance caused by substituent R₄ that will interfere with formation of maximal number of specific complexes obtained from the compound analyzed and fluorescent-labeled probe.

Analogous drawbacks are appropriate for dyes having the reactive carboxyl group R₂ attached to 5-position of indolenyne moiety directly or via one methylene group (R₂=-COOH or -CH₂COOH)). The probe being attached to carboxyl group as located in substituent R₂ will be situated in the close proximity to the spatial fluorophore that will also interfere with formation of maximal number of specific complexes obtained from the compound analyzed and fluorescent-labeled probe.

Dyes with a reactive group R₁ as attached to 3-position of indolenyne moiety via pentamethylene chain of atoms (R₁= -(CH₂)₅COOH) also have some drawbacks. Pentamethylene chain moving the fluorophore off the reactive carboxyl group is strongly hydrophobic that will cause a twisting this chain in aqueous solutions, wherein bioassay being performed, As a result, the probe and fluorophore molecule will be situated in the close proximity that will also interfere with formation of maximal number of specific complexes obtained from the compound analyzed and fluorescent-labeled probe.

### Brief Description of Drawings

***Fig. 1******.*** Normalized absorption and fluorescence spectra: ---- a dye of formula **(I)** (R₁=-CH₃, R₃= -(CH₂)₅COOH, =-SO₃⁻; R₄,=-C₂H₅; R₂=R₅=R₆=H) and ---- dye **(A10).**
***Fig. 2******.*** Molar extinction coefficient for dyes in methanol and PBS.
***Fig. 3******.*** Fluorescence quantum efficiency of dyes in methanol and PBS.
***Fig. 4******.*** Fluorescence relative efficiency of dyes. Excitation and record made in absorption and fluorescence maxima.
***Fig. 5******.*** Fluorescence relative efficiency of dyes. Excitation at 655 nm. Record at 690 nm.
***Fig. 6******.*** Fluorescence relative efficiency of dyes. Excitation 655 nm. Record 670 nm.
***Fig. 7******.*** Time dependence of fluorescence intensity under irradiation of dyes over biochip with semiconductor laser light source (655 nm / 690 nm, 40 mW): **1** - dye **(B1); 2** - dye **(A10); 3** - dye **(B6); 4** - dye of formula (**I**) (R₁= -CH₃, R₃= -(CH₂)₅COOH, = -SO3⁻; R₄= -C₂H₅; R₂=R₅=R₆=H); **5** - dye (**B5**); **6** - dye of formula (**I**) (R₁= -CH₃, R₂= -SO3⁻, R₃= -(CH₂)₅COOH, R₄= -(C₂H)₄SO3⁻, R₅=R₆= -H); **7** - dye of formula (**I**) (R₁= -CH₃, R₃= -(CH₂)₅COOH, R₄= -(C₂H)₄SO₃⁻, R₂-R₅R₆= -H); **8** - dye **(B4).**
***Fig. 8******.*** Determination of relative sensitivity for detection of labeled synthetic oligonucleotides using a portable fluorescent analyzer of biochip's images with a semiconductor laser light source (655 nm / 690 nm, 40 mW): **1** - dye of formula (**I**) (R₃= -(CH₂)₅COOH, R₁=R₅= -CH₃, R₂=R₆=-SO₃⁻; R₄= -C₂H₅); **2** - dye of formula (**I**) (R₁= -CH₃, R₃= -(CH₂)₅COOH, R₄= -C₂H₅; R₂=R₅=R₆= -H); **3** - dye **(A10); 4** - dye **(B5).**
**Fig. 9****.** Detection of tuberculosis mycobacterium mutation (Leucine 531) over microchips using dye of formula (**I**) (R₁= CH₃, R₃= -(CH₂)₅COOH, -SO₃⁻ R₄= C₂H₅; R₂=R₅=R₆= H) (**a**) and dye **(A10) (b).**
***Fig. 10***. Photography and Scheme of microchip after performing analysis, wherein: **1** -protein A concentration in a gel (0.4 mg/ml), **2** - protein A concentration in a gel (0.2 mg/ml), **3** - protein A concentration in a gel (0.1 mg/ml), **4** - protein A concentration in a gel (0.05 mg/ml), **5** - protein A concentration in a gel (0.025 mg/ml), **6** - protein A concentration in a gel (0,013 mg/ml), **7** - protein A concentration in a gel (0.006 mg/ml), **8** - a blank gel.
***Fig. 11******.*** Concentration dependence of fluorescence normalized signal for protein A immobilized in a gel: **1** - dye of formula **I** (R₁= -CH₃, R₂= R₅= R₆ = -H; R₃= -(CH₂)₅COOH, R₄,= -C₂H₅), **2** - dye **(A10).**

### Summary of the Invention

An object of the invention is to provide indocyanine dyes comprising two ring systems being bound with a polymethine linker bond and containing a carboxyl group attached to a heterocyclic moiety via polymethylene chain, for utilization thereof as fluorescent labels in biological materials. Provided in the present invention, such compounds have general formula **(I):** 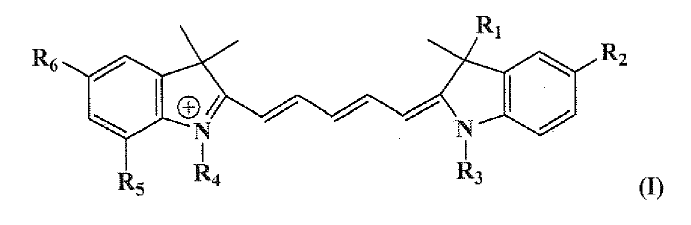 wherein:
R₁ is -CH₃; -(CH₂)₄COOH;
R₂ represents -CH₃; -(CH₂)₂COOH; -H; -SO₃⁻;
R₃ represents -C₂H₅; -(CH₂)₄SO₃⁻;
R₄ represents -C₂H₅; -(CH₂)₄SO₃⁻;
R₅ represents -H; -CH₃;
R₆ represents -H; -CH₃; -SO₃⁻.

Specifically, compounds being arbitrary considered as compounds of group A correspond to general formula (A) 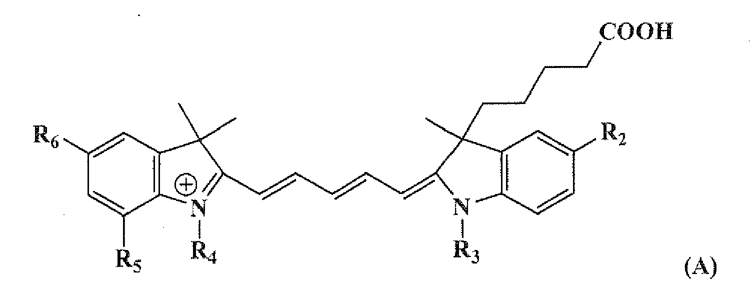 wherein:
R₁ is -(CH₂)₄COOH;
R₂ is -H; -CH₃; -SO₃⁻;
R₃ represents -C₂H₅;-(CH₂)₄SO₃⁻
R₄ represents -C₂H₅; -(CH₂)₄SO₃⁻;
R₅ represents -H; -CH₃;
R₆ represents -H; -CH₃; -SO₃⁻;
as well as compounds being arbitrary considered as compounds of group **B** correspond to general formula **(B)** 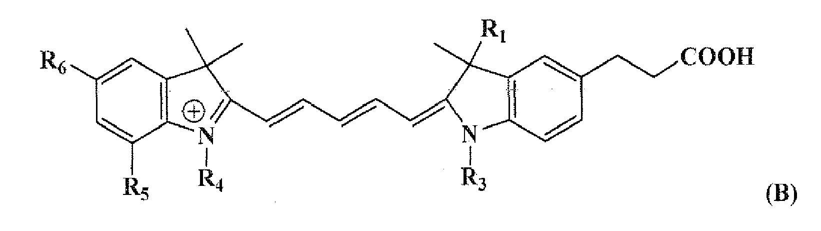 wherein:
R₁ is -CH₃;
R₂ represents -(CH₂)₂COOH;
R₃ represents -C₂H₅; -(CH₂)₄SO₃⁻;
R₄ represents-C₂H₅; -(CH₂)₄SO₃⁻;
R₅ represents -H;
R₆ represents -H; -CH₃; -SO₃⁻

Indocyanine dyes **(A)** proposed are characterizing in that the reactive carboxyl group thereof is attached to 3-position of the indolenyne moiety via tetramethylene chain of atoms (R₁= -(CH₂)₄COOH, wherein R₂, R₃, R₄, R₅, R₆ are alkyl-, sulfo-, and sulfoalkyl substituents).

Indocyanine dyes **(B)** proposed are characterizing in that the reactive carboxyl group thereof is attached to 5-position of the indolenyne moiety via dimethylene chain of atoms (R₂= -(CH₂)₂COOH, wherein R₁, R₂, R₃, R₄, R₅, R₆ are alkyl-, sulfo-, and sulfoalkyl substituents).

There are some features distinguishing dyes **(A)** from dyes being disclosed in references above, as follows: 1) the reactive carboxyl group is separated from indolenyne moiety with four methylene groups, instead of five groups as in analogs above; 2) method for producing an intermediate - ketoacid being utilized for synthesis of the corresponding indolenyne, differs from the method being disclosed in references *(*WO 02/26891 A1*;* EP1211294 A1).

The specified structural distinctions of proposed dyes **(A)** result in series of unexpected properties of the compounds according to the invention. The compounds according to the invention are featured by:
- improved light resistance, and
- increased chemical and thermal stability when used as fluorescent labels for biomolecules in the technology of biological microchips having both nucleotide and protein nature.

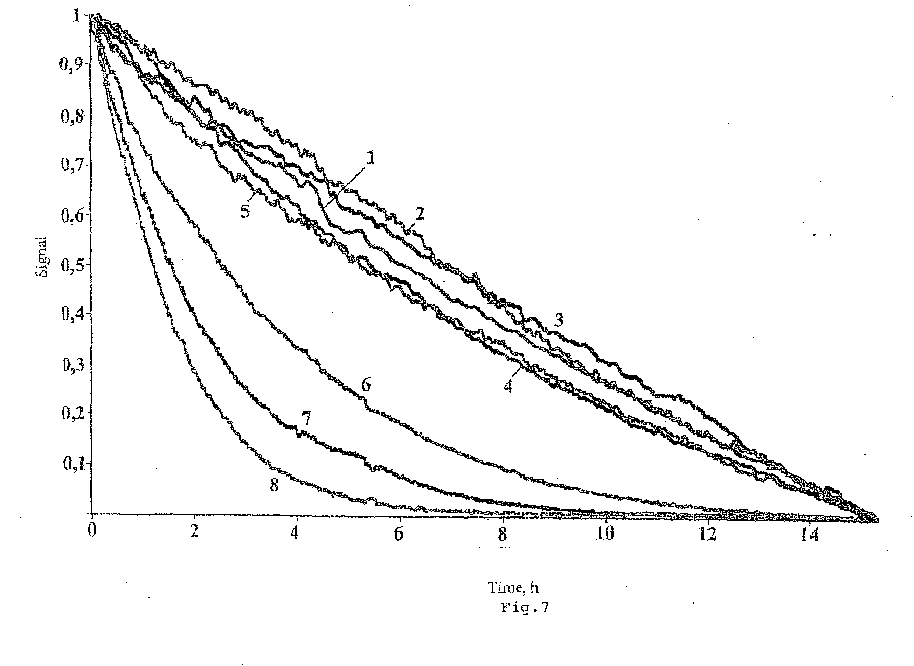

On continuous irradiation in a fluorescent microscope using a wavelength of 655 nm, a half lifetime of dyes of formula **(I)** is shown to differ considerably: the half lifetime (LT₅₀) for dye **(A10)** is 7 h, for dye of formula **(I)** (R₁= -CH₃; R₂= -SO₃⁻; R₃= -(CH₂)₅COOH, R₄=-(CH₂)₄SO₃⁻; R₅= R₆= -H), the LT₅₀ is 2.5 h, for dyes of formula (**I**) (R₁=-CH₃; R₂-R₅=R₆=-H, R₃= - (CH₂)₅COOH, R₄= -(CH₂)₄SO₃⁻), the LT₅₀ is 1.5 h (Fig. 7).
- higher sensitivity for recording fluorescence when used as fluorescent labels of biomolecules in the technology of biological microchips having both nucleotide and protein nature.

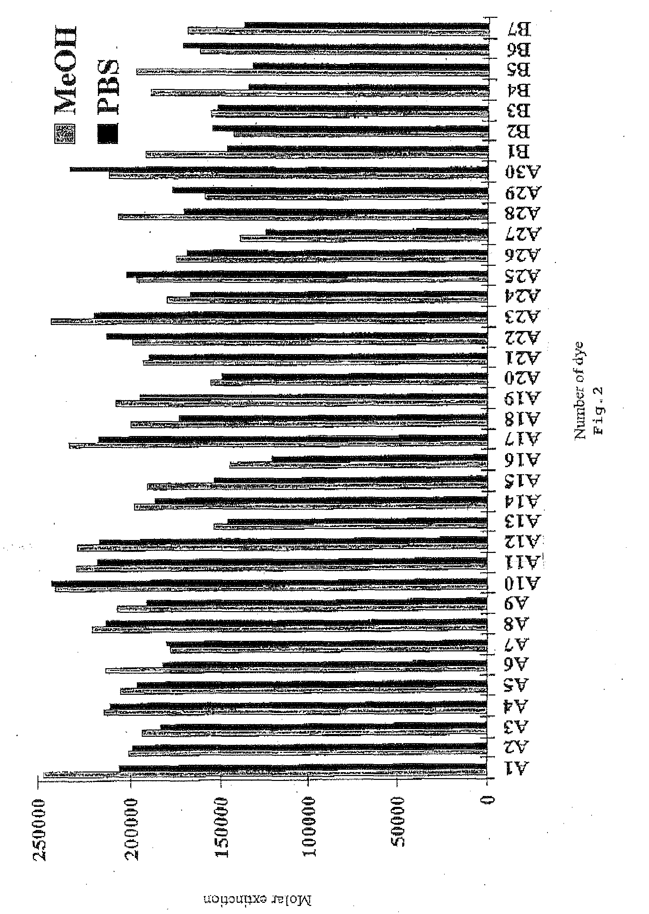

In methanol, the molar extinction coefficient (ε) of dyes **(A1), (A10),** and **(A23)** exceeds 240,000, in aqueous phosphate buffered saline (PBS), the ε-value of dyes **(A10)** and **(A30)** exceeds 230,000 (Table 1, Fig. 2), while dyes of formula **(I)** (R₁ = -CH₃, R₂ = R₆= -SO₃⁻; R₃ = -(CH₂)₅COOH, R₄= -C₂H₅; R₅= -H) in methanol and aqueous phosphate buffered saline have the ε-values of 167,000 and 174,000, respectively.

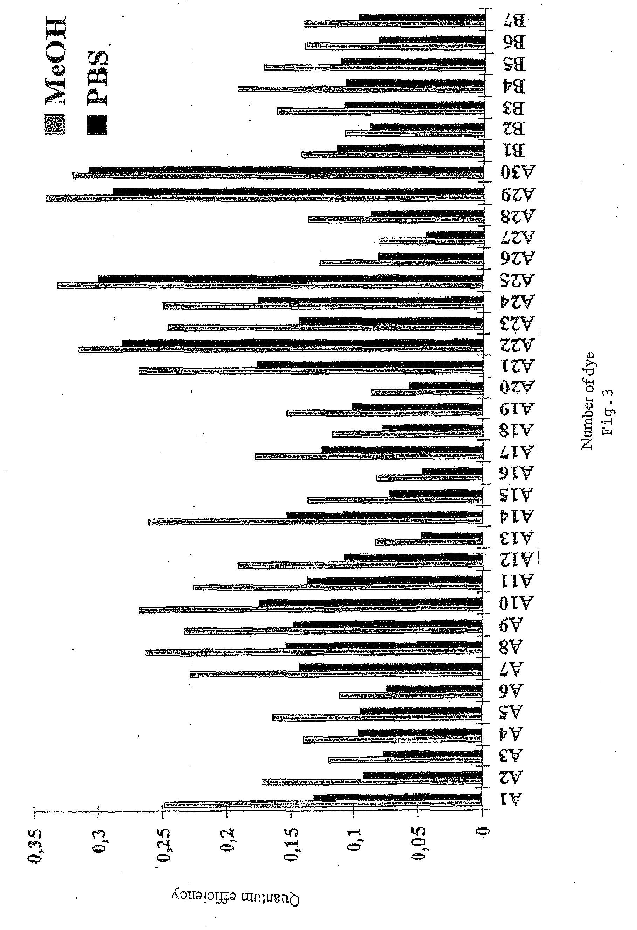

In methanol, the quantum efficiency of fluorescence (QE) for dyes **(A25)** and **(A29)** exceeds 0.33, in PBS, the QE for dyes **(A25)** and **(A30)** is more than 0.30 (Table 1, Fig. 3), whereas dyes of formula **(I)** (R₁= -CH₃, R₂= R₆= -SO₃⁻; R₃= -(CH₂)₅COOH, R₄= -C₂H₅; R₅= -H) in methanol and aqueous phosphate buffered saline have the QE-values of 0.32 and 0.27, respectively.

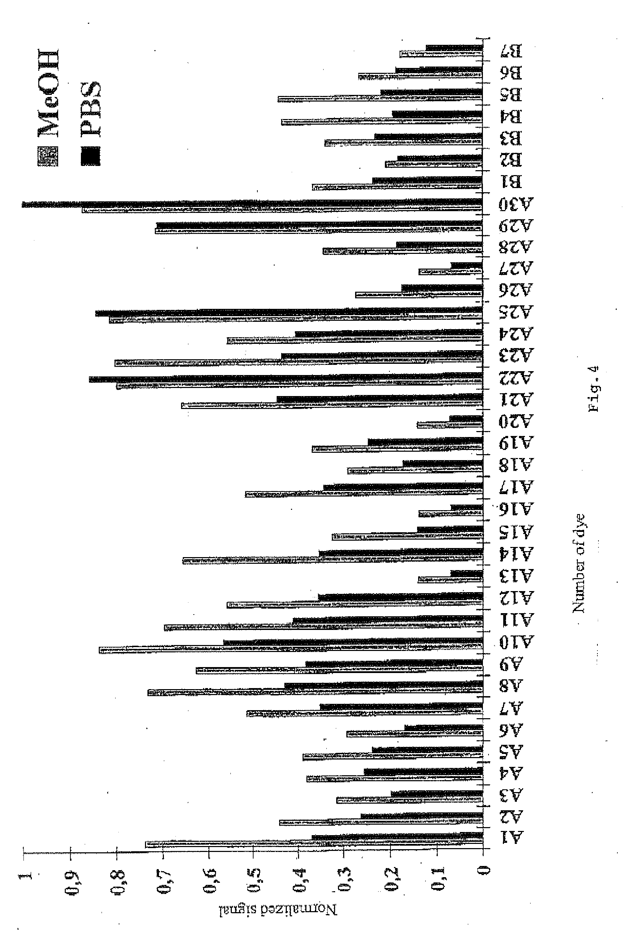

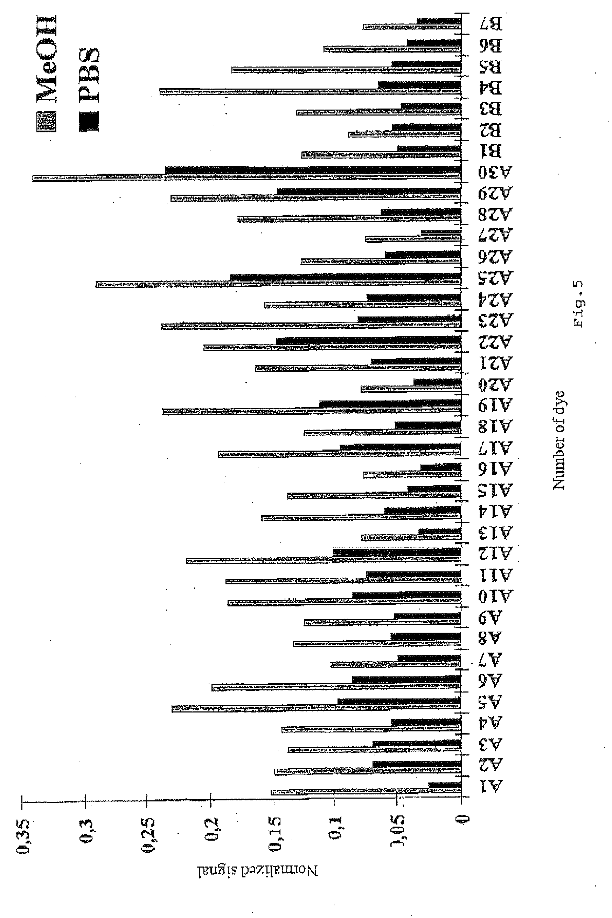

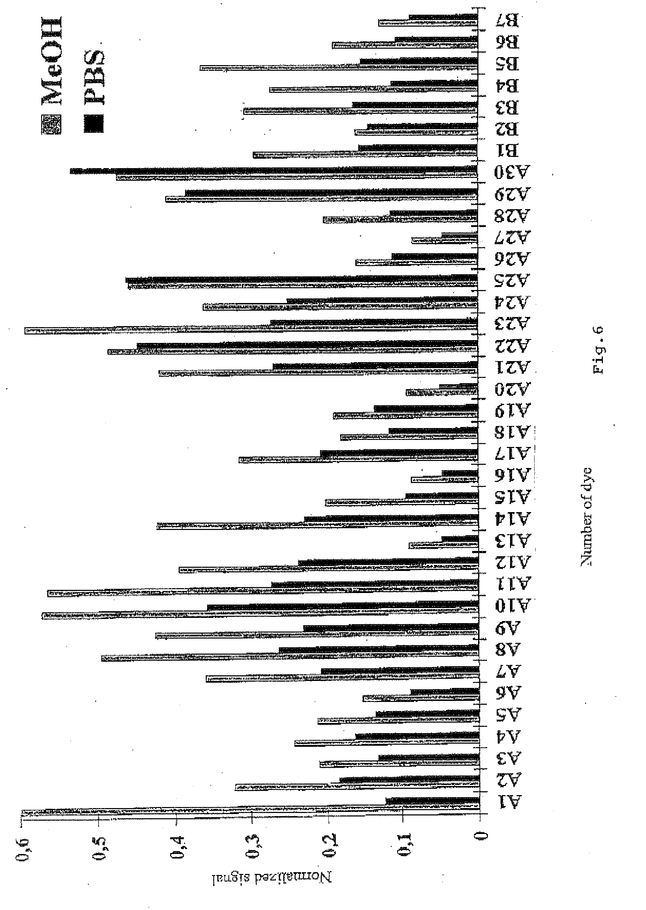

The relative efficiency of fluorescence for dyes proposed was determined, and dye **(A30)** in PBS showed the highest efficiency value on excitation and record in absorption and fluorescence maxima, and this value being taken as a unit (Table 1, Fig. 4-6). The data obtained were as follows:
- on excitation and record in absorption and fluorescence maxima, the relative efficiency of fluorescence exceeds 0.80 for dyes **(A10), (A22), (A23), (A25),** and **(A30)** in methanol and for dyes **(A22), (A25),** and **(A30)** in PBS (Table 1, Fig. 4), whereas for dyes of formula **(I)** (R₁ = -CH₃, R₂= R₆= -SO₃⁻; R₃= -(CH₂)₅COOH, R₄= -C₂H₅; R₅= -H) the values are 0.69 in methanol and 0.67 in PBS;
- on excitation at wavelength 655 nm and record at 690 nm, the relative efficiency of fluorescence exceeds 0.25 for dyes **(A25)** and **(A30)** in methanol and is higher than 0.18 for dyes **(A24)** and **(A30)** in PBS (Table 1, Fig. 5), whereas for dyes of formula **(I)** (R₁ = -CH₃, R₂ = R₆= -SO₃⁻; R₃= -(CH₂)₅COOH, R₄= -C₂H₅; R₅= -H) the values are 0.18 in methanol and 0.11 in PBS;
- on excitation at wavelength 635 nm and record at 670 nm, the relative efficiency of fluorescence exceeds 0.50 for dyes **(A1), (A11), (A23)** in methanol and is higher than 0.45 for dyes **(A22), (A25),** and **(A30)** in PBS (Table 1, Fig. 6), whereas for dyes of formula **(I)** (R₁= -CH₃, R₂= R₆= -SO₃⁻; R₃= -(CH₂)₅COOH, R₄= -C₂H₅; R₅= -H) the values are 0.44 in methanol and 0.38 in PBS.

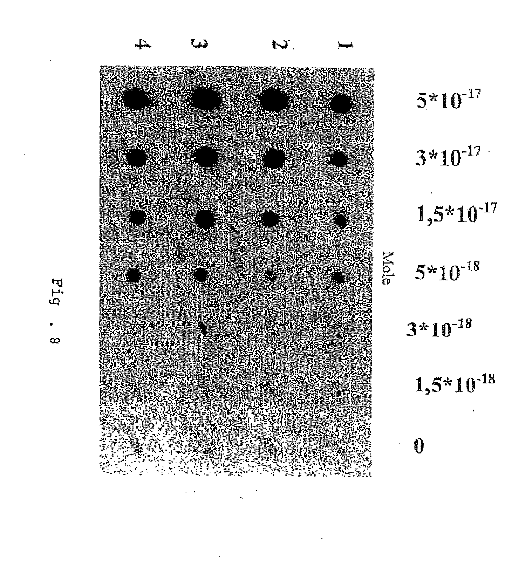

For oligonucleotides labeled with dye **(A10)** and situated at hydrogel cells of biological microchip, at the portable fluorescent analyzer of biochips' images using a semiconductor laser light source (655 nm / 690 nm, 40 mW), the sensitivity of record is 3*10⁻¹⁸ mole, and for dyes of formula **(I)** (R₁= -CH₃, R₂=R₆= -SO₃⁻; R₃= -(CH₂)₅COOH, R₄= -C₂H₅, R₅= -H) and (R₁= -CH₃, R₂= R₅= R₆= -H; R₃= -(CH₂)₅COOH, R₄= -C₂H₅), the sensitivity of record is 5*10⁻¹⁸ mole (Fig. 8).

In actual practice of bioassay, the properties of dyes **(A1) - (A30)** specified are cardinally important for fluorescent labeling.

There are some features distinguishing dyes **(B)** from dyes being disclosed in references above, as follows: 1) the reactive carboxyl group is separated from indolenyne moiety with two methylene groups, instead of one group (or the absence thereof) as in analogs above; 2) method for producing an intermediate - indolenyne comprising a carboxyl group and being utilized for synthesis of corresponding dyes, differs from the method being disclosed in references (Mank A.J.G. et al., Anal. Chem., 1995, vol, 67, pp. 1742-1748; Southwick et al., Cytometry, 1990;11(3):418-30*;* Lin Y. Et al., Bioconjuc. Chem., 2002, vol. 13, pp, 605-610).

The specified structural distinctions of proposed dyes **(B)** result in series of unexpected properties of compounds according to the invention. The compounds according to the invention are featured by:
- improved light resistance, and
- increased chemical and thermal stability when used as fluorescent labels for biomolecules in the technology of biological microchips having both nucleotide and protein nature.

On continuous irradiation in a fluorescent microscope using a wavelength of 655 nm, the half lifetime of dyes of formula **(I)** is shown to differ appreciably: for dye **(B6)** the LT₅₀ is 4.5 h, for dye of formula **(I)** (R₁= -CH₃; R₂= -SO₃⁻; R₃= -(CH₂)₅COOH, R₄= -(CH₂)₄SO₃⁻; R₅= R₆= -H), the LT₅₀ is 2.5 h, for dye of formula **(I)** (R₁ = -CH₃; R₂ = R₅ = R₆ = -H, R₃ = -(CH₂)₅COOH, R₄ = -(CH₂)₄SO₃⁻), the LT₅₀ is 1.5 h (Fig. 7).
- the ability to form specific complexes increases due to elongation of the polymethylene chain, and appropriate moving the label of fluorophore nucleus off the molecule being labeled;
- the sensitivity and accuracy of instrumental record of quantity of the specific complexes formed are improved.

For dye **(B5),** the molar extinction coefficient is about 200,000 (Table 1, Fig. 2). For dyes **(B4)** and **(B5)** in methanol, the quantum efficiencies of fluorescence are 0.19 and 0.17, respectively (Table 1, Fig. 3). For dyes of formula **(I)** (R₁= -CH₃, R₂= -COOH, R₃= R₄= -(CH₂)₄SO₃⁻, R₅= R₆= -H) and (R₁= -CH₃, R₂= -CH₂COOH, R₃= R₄= -(CH₂)₄SO₃⁻, R₅=R₆= -H) in acetonitrile, the quantum efficiencies of fluorescence are 0.12 and 0.16, respectively (Mank A.J. G. et al., Anal. Chem., 1995, vol. 67, pp. 1742-1748*).*

For oligonucleotides labeled with dye **(B5)** at the portable fluorescent analyzer of biochips' images using a semiconductor laser light source (655 nm / 690 nm, 40 mW), the sensitivity of record is 5***10⁻¹⁸ mole.

Subject to substituent at the heterocyclic moiety, dyes of formulae **(A)** and **(B)** proposed are distinguished by a spectral range of excitation and fluorescence, by the total electrical charge, and water solubility (Table 1). For example, monosulfonated (electrically neutral) compounds **(A1) - (A9), (B1) - (B3)** are partially dissolved in water; disulfonated (electronegative) compounds **(A10) - (A22), (B4) - (B6)** have good water solubility; trisulfonated (doubly electronegative) compounds **(A23) - (A29), (B7)** and tetrasulfonated compound (triply electronegative) **(A30)** have a very good water solubility.

Due to spectral properties in the near infrared range, indodicarbocyanine dyes of formulae **(A)** and **(B)** are optimal for labeling biomolecules, since the inherent fluorescence of a biomaterial is low, in the range of wavelength between 600 and 800 nm. For this dyes, it is economically sound use of inexpensive semiconductor lasers as a light source. In this case, the high fluorescence signal is observed.

The high-efficient detection of various mutations is available using the hybridization of DNA analyzed with a kit of oligonucleotides being immobilized in gel cells of the microchip. DNA analyzed is preliminary labeled with fluorescent dye. The hybridization results are recorded by the instrumentality of fluorescent microscope. In each cell of microchip, the intensity of net fluorescence signal is proportional to the content of duplex formed.

There are available the commercial semiconductor lasers having wavelengths of 655 nm and 635 nm and being suitable for pentamethine dyes of formula **(I).** In the use of laser having wavelength of 655 nm as a light source, the fluorescence is recorded by using a light filter with transmission of 690-730 nm. In the case of laser having wavelength of 635 nm, the fluorescence is recorded by using the light filter with transmission of 670 nm and higher.

Next, there are presented results on application of dyes for the proposed method of analysis. With a good yield, all dyes of formula **(I) (A1) - (A30)** and **(B1) - (B7),** as *N-*hydroxysuccinimide esters (for **(A)** R₁= -(CH₂)₄COOSu; for **(B)** R₂=-(CH₂)₂COOSu) and *para-*nitrophenyl esters fitted (for **(A)** R₁= -(CH₂)₄COO-p-NP; for **(B)** R₂= -(CH₂)₂COO-p-NP) label oligonucleotides comprising the phosphate group with aliphatic amine linker at 5'-terminal. On carrying out the reaction in the carbonate-bicarbonate buffer solution at pH 9.0-9.2, in a mixed water-organic solvent, the conjugate yield is 70 - 80%.

All dyes of formula **(I) (A1) - (A30)** and **(B1) - (B7),** being conjugated with oligonucleotides, are found not to inhibit the polymerase chain reaction (PCR). The quantity and composition of reaction products are similar for unlabeled and labeled oligonucleotides, but depend on other conditions of performing reaction only.

The sensitivity of recording the results of polymerase chain reaction with the use of primers, being labeled with dyes of formula **(I) (A1) - (A30)** and **(B1) - (B7),** is found to depend on spectral characteristics of dye and conformity thereof with the spectral characteristics of recording instrument (fluorescent microscope). Dyes of formula **(I) (A1) - (A30)** and **(B1) - (B7)** are characterized with the extremely high sensitivity of record. With the use of portable fluorescent microscope, being developed in 1MB RAS and having the semiconductor laser light source (655 nm, 40 mW; record at 690 nm), a lower threshold of sensitivity to oligonucleotides, being labeled with dye **(A10)** and situated in hydrogel cells of biological microchip, attains 3*10⁻¹⁸ mole. This value, however, is equal to 5* 10⁻¹⁸ mole for dyes of formula **(I)** (R₁ = -CH₃, R₂ = R₆ = -SO₃⁻; R₃= -(CH₂)₅COOH, R₄= -C₂H₅, R₅= -H) and (R₁= -CH₃, R₂=R₅=R₆= -H; R₃= -(CH₂)₅COOH, R₄= -C₂H₅) (Fig. 8).

The reproducibility of analysis results is found to depend on hydrofilic-hydrophobic properties of dyes. The reproducibility of analysis results varies in the order of increasing: non-sulfonated< monosulfonated< disulfonated< trisulfonated< tetrasulfonated dyes. The non-sulfonated dyes have the minimal reproducibility, but tri- and tetrasulfonated dyes have the maximal reproducibility.

The dyes of formula **(I)** are found not to affect the stability of all the rest of analysis components. For example, dye of formula **(A10)** does not affect the stability of all the rest components of operating mixtures and solutions of diagnostic testing module during storage thereof at room temperature in the course of 1 year.

All dyes of formula **(I) (A1) - (A30)** and **(B1) - (B7)** are found to possess the sufficient stability for performing a complete cycle of analysis without appreciable decomposition of dye.

Taking characteristics: sensitivity, stability, and minimal effect on biochemical properties of labeled oligonucleotides and proteins, and usability in the aggregate, all dyes of formula **(I) (A1) - (A30)** and **(B1) - (B7)** may be used as fluorescent labels in developing diagnostic testing modules.

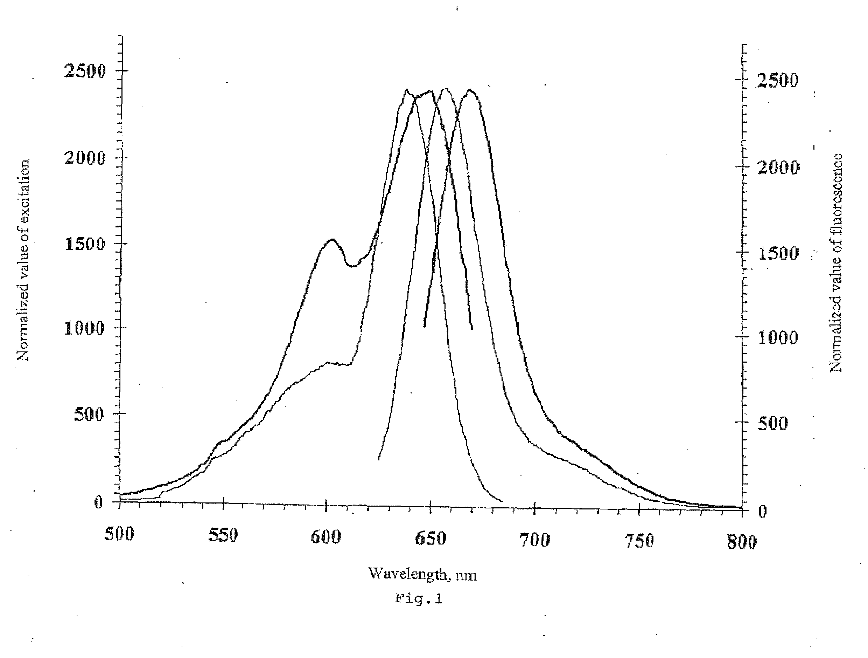

Table 1 presents the fluorescent characteristics of dyes **(I) (A1) - (A30)** and **(B1) - (B7)** proposed. An insertion of methyl group at 5- and 7-positions of indolenyne cycle produces a bathochromic shift in the absorption and fluorescent spectra. As to the rest sulfur containing dyes, the spectral characteristics thereof appear to be in the same range (between about λₘₐₓ 648 nm and λₑₘ 669 nm) (Fig. 1),

For all indodicarbocyanine dyes **(I) (A1) - (A30)** and **(B1) - (B7)** of the invention, there are determined the molar extinction coefficients in methanol and in aqueous phosphate buffered saline (PBS) (10 mM potassium phosphate buffer, sodium chloride concentration is 0.9%, pH 7.4). Table 1 presents data. In methanol, indodicarbocyanine **(A1, A8, A10-A12, A17, A23)** has the upper-range of molar extinction coefficient (ε >2.2*10⁻⁵). In aqueous phosphate buffered saline (PBS), dyes **(A10, A23, A30)** have the maximal ε-value (Fig. 2).

For all indodicarbocyanine dyes **(I) (A1) - (A30)** and **(B1) - (B7)** of the invention, there are determined the quantum efficiency of fluorescence in methanol and in aqueous phosphate buffered saline (Table 1, Fig. 3). The measurements are performed versus dyes of formula **(I)** (R₁= -CH₃, R₂= R₆= -SO₃⁻; R₃= -(CH₂)₅COOH, R₄= -C₂H₅, R₅= -H) whose quantum efficiency of fluorescence in PBS is 0.27 *(*Mader O., K. Reider, H.-J. Egelhaaaf, R, Fischer, R. Brock, Structure Properties of Pentamethine Indocyanine Dyes: Identification of New Dyes for Life Science Applications. Bioconjugate Chem, 2004, vol. 15, pp. 70-78*).* In methanol, dyes **(A10, A22, A25, A29, A30)** have the upper-range of quantum efficiency of fluorescence (Φ ∼ 0.3), but in PBS, dyes **(A10, A22, A25, A29, A30)** have the maximal Φ -value.

Term sensitivity of fluorescent analysis is defined by the absolute sensitivity of label that may be represented as the product of molar extinction coefficient by quantum efficiency of fluorescence (ε*Φ). Thus, there are determined the absolute sensitivity of indodicarbocyanines **(A1-A30, B1-B7)** in appropriate absorption and fluorescence maxima in methanol and in aqueous phosphate buffered saline. The values are normalized to the maximal value obtained for dye **(A30)** in PBS (Table 1). In methanol, dyes **(A10, A22, A23, A25, A30)** have the upper-range of absolute sensitivity, but in PBS, dyes **(A10, A22, A25, A29, A30)** have the maximal values of sensitivity (Fig. 4).

There are available two instruments having the commercial semiconductor laser light sources - 635 nm / 670 nm (20 mW), 655 nm / 690 nm (40 mW), and advantages of the latter being high-power beam and relatively low cost.

To determine the most appropriate dyes for instrument setting, there are measured the relative efficiency of fluorescence of dyes at wavelength of excitation and record of the instrument (655 nm. / 690 nm). The maximal values appear to be specific to dyes **(A25)** and **(A30)** in methanol, and to dyes **(A19, A22, A25, A29, A30)** in PBS (Table 1, Fig. 5).

There are also measured the relative efficiency of fluorescence at wavelength of excitation and record of the instrument (635 nm / 670 nm). The maximal values appear to be specific to dyes **(A1, A10, A11, A23)** in methanol, and to dyes **(A10, A22, A25, A29, A30)** in PBS (Table 1, Fig. 6).

Based upon data obtained, one may draw a conclusion as follows. The claimed dyes of formulae **(A)** and **(B)** are utilizable as fluorescent labels to perform the hybridization assay by means of biological microchips.

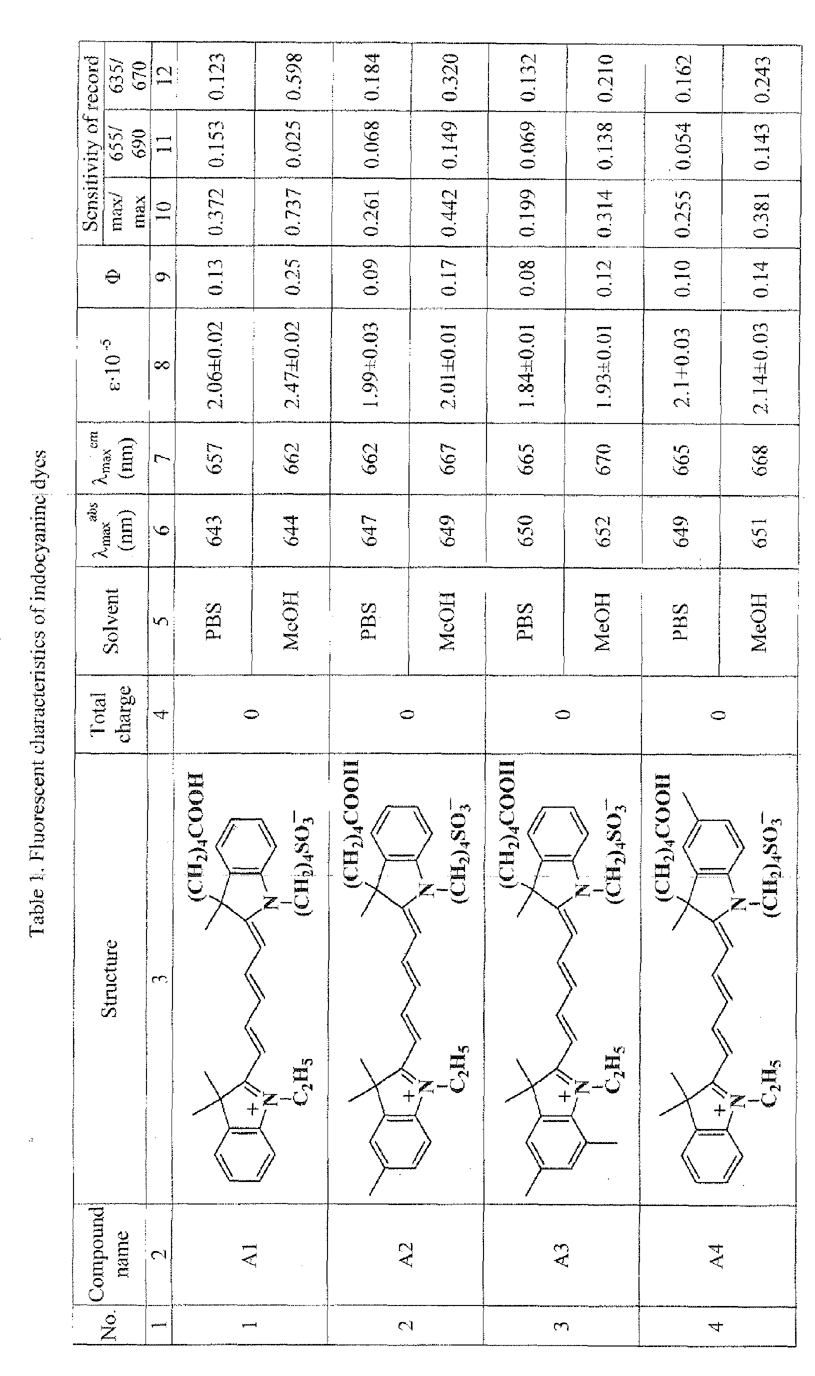

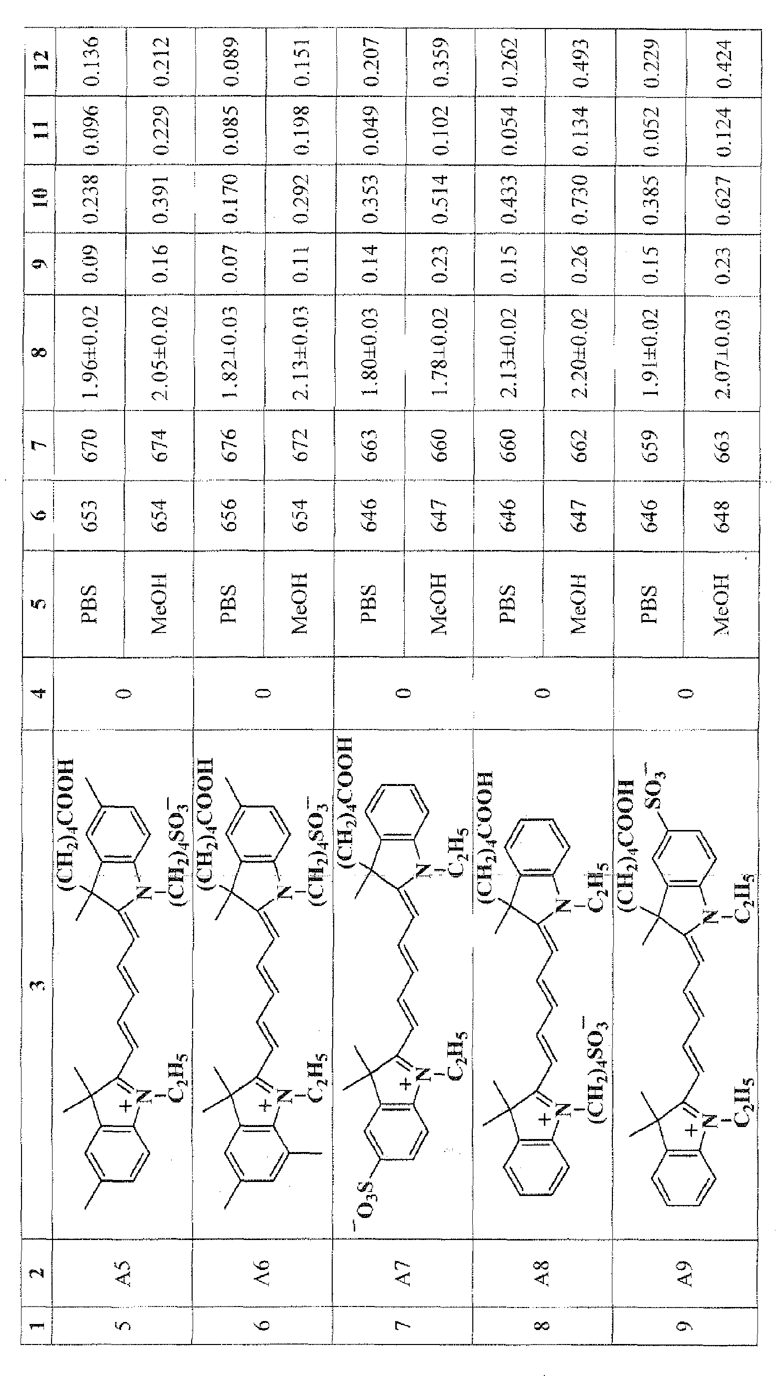

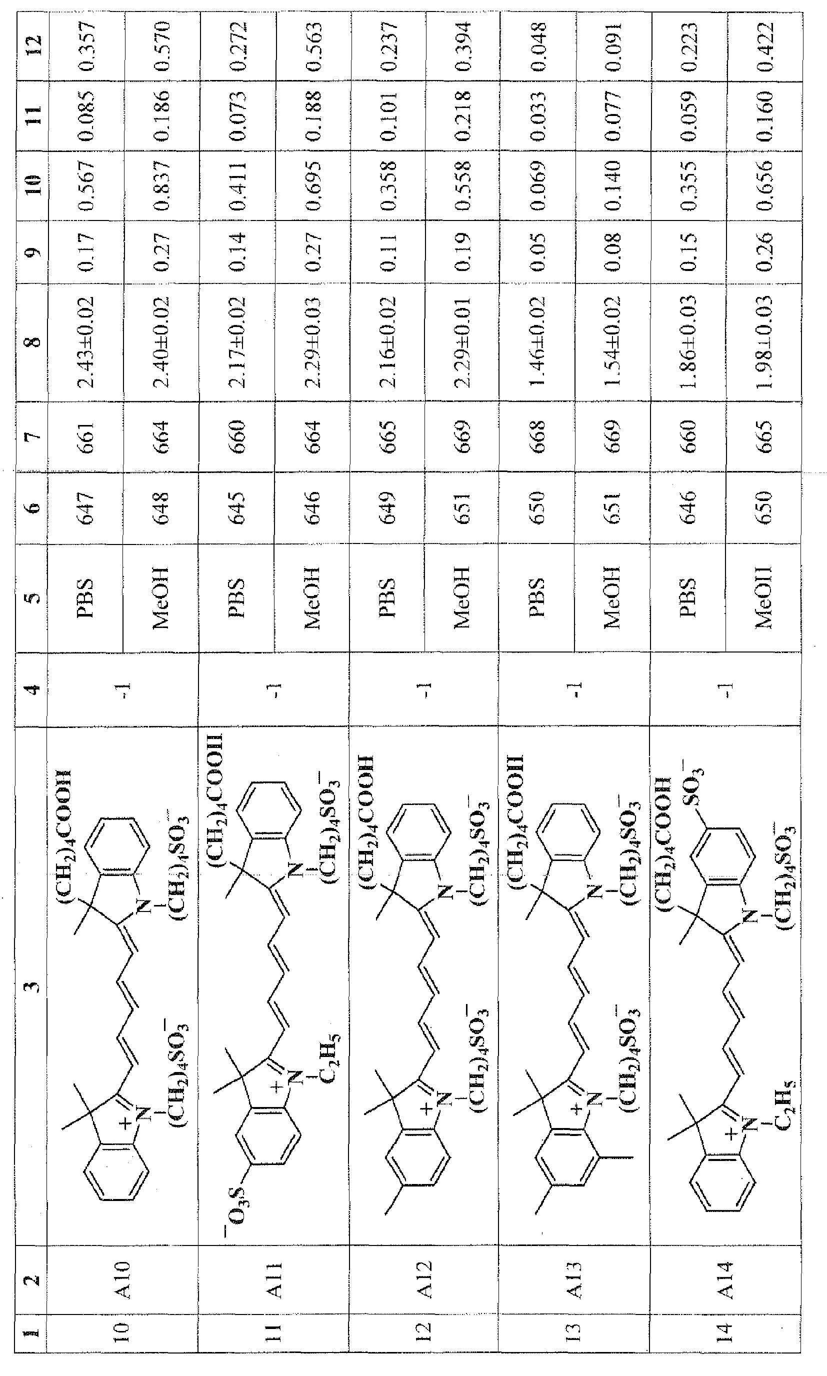

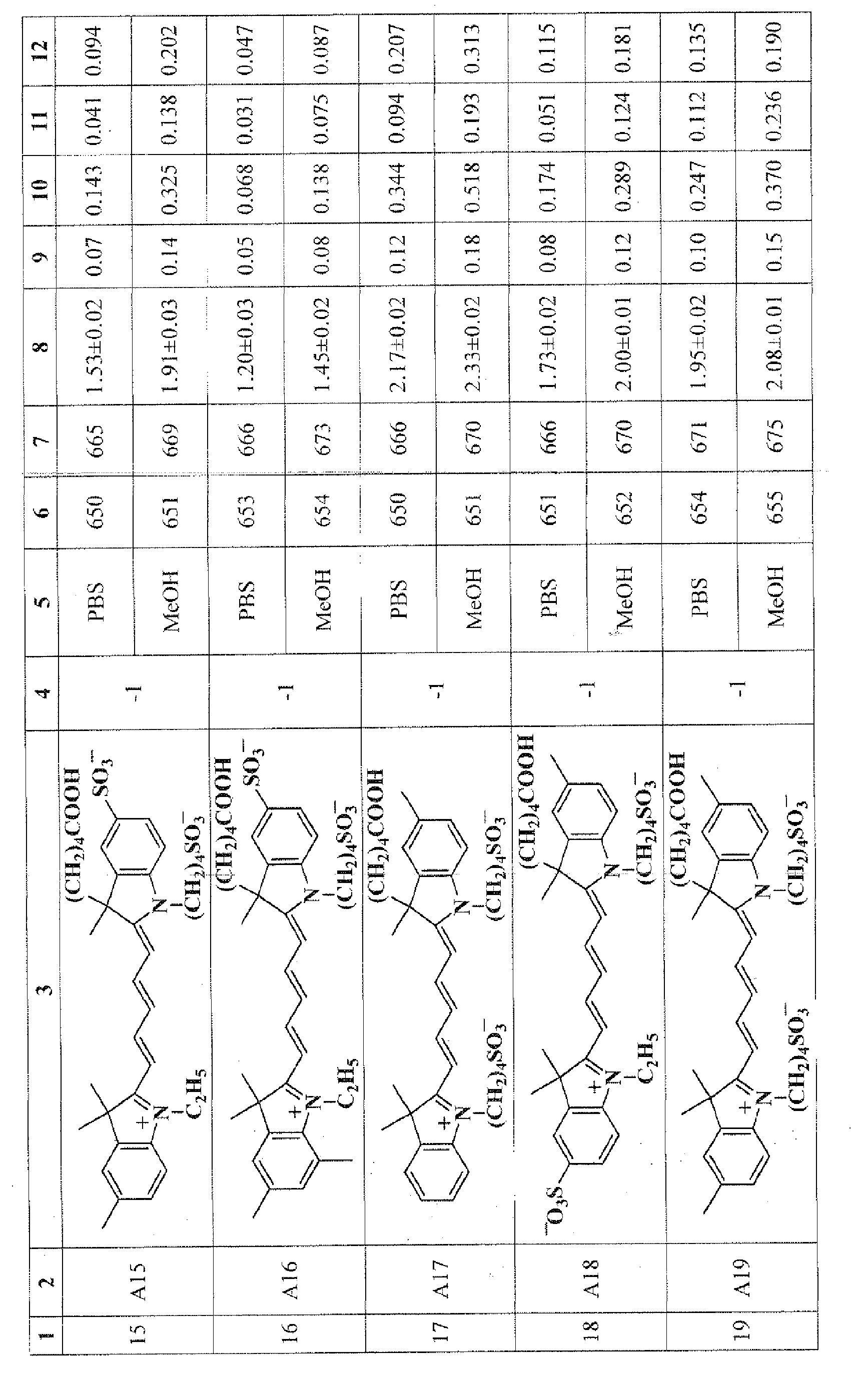

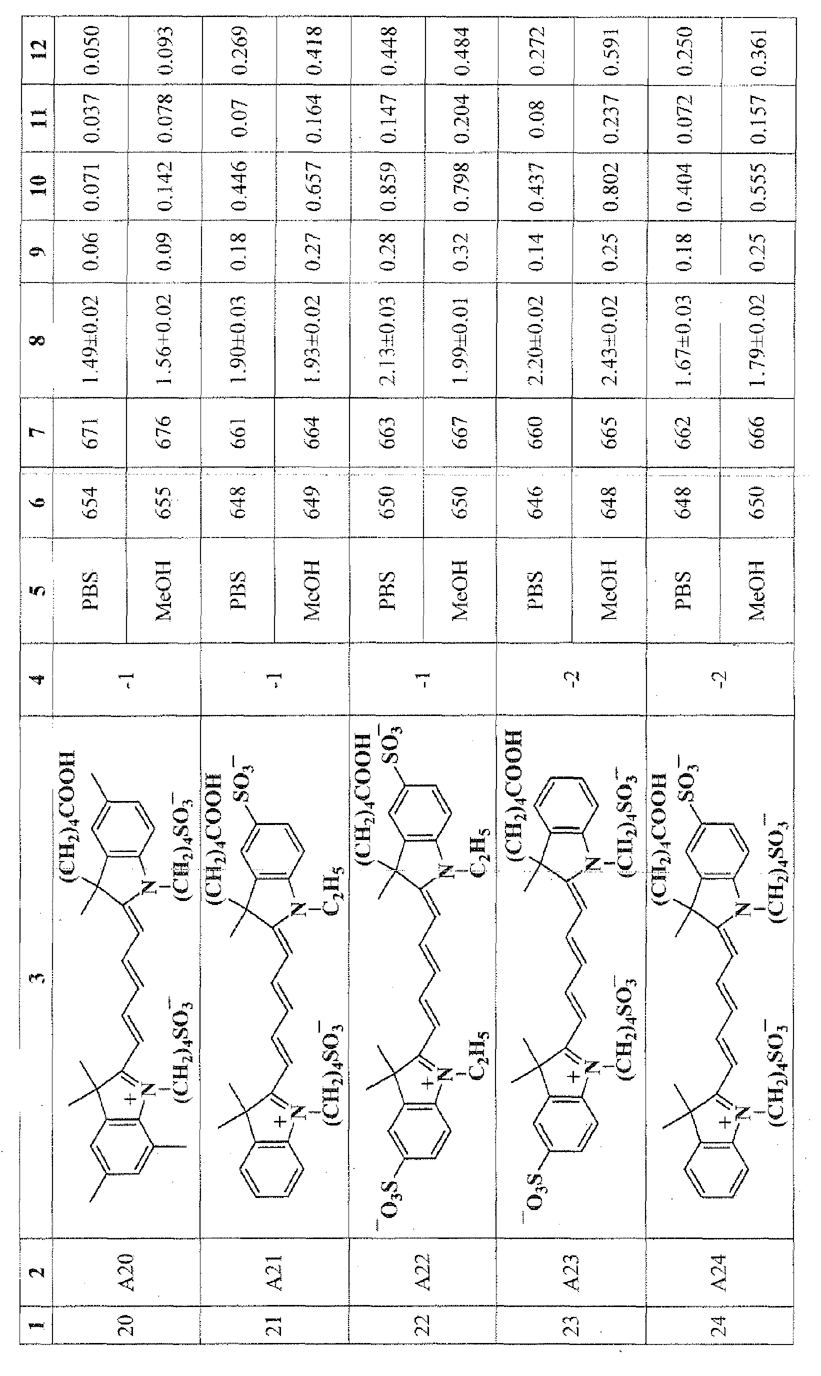

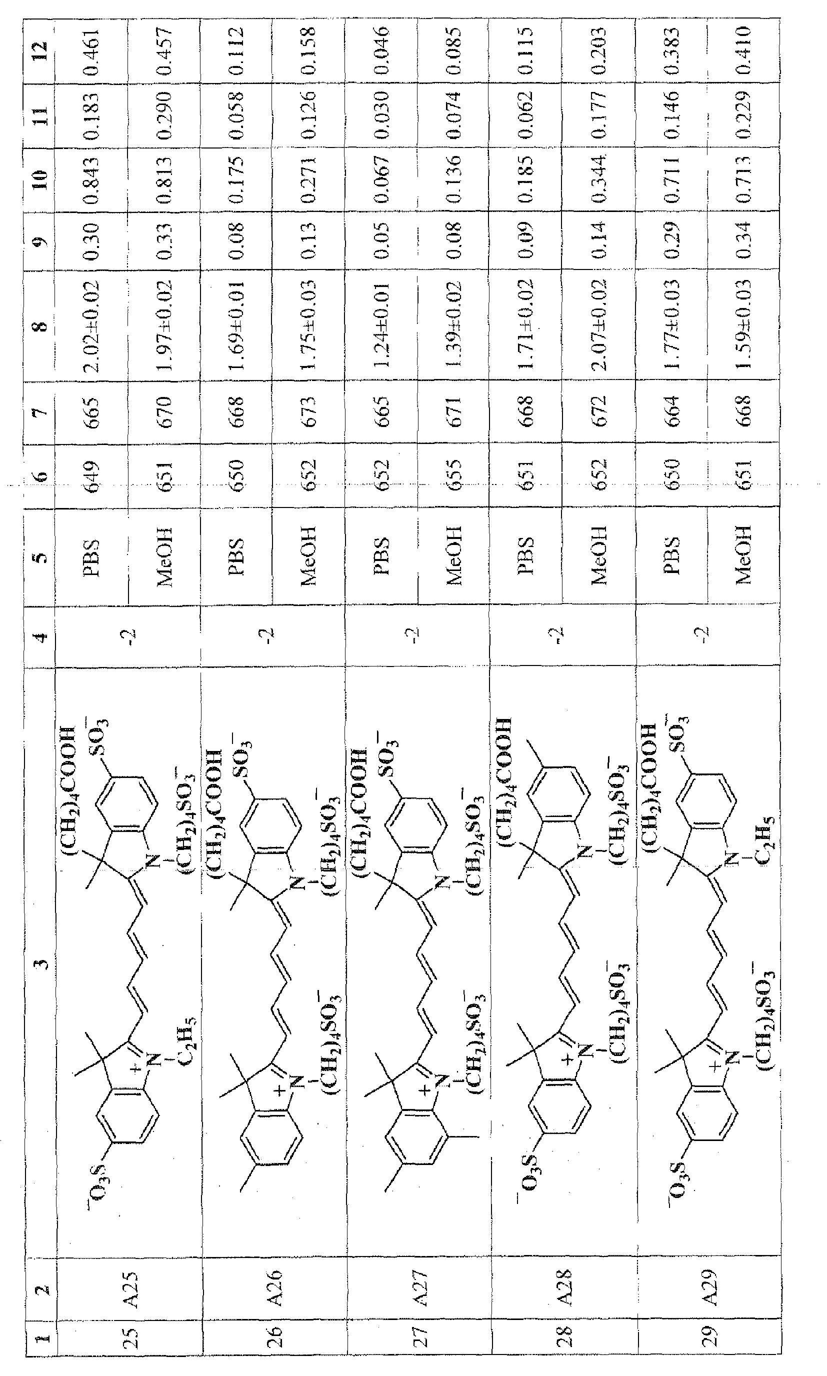

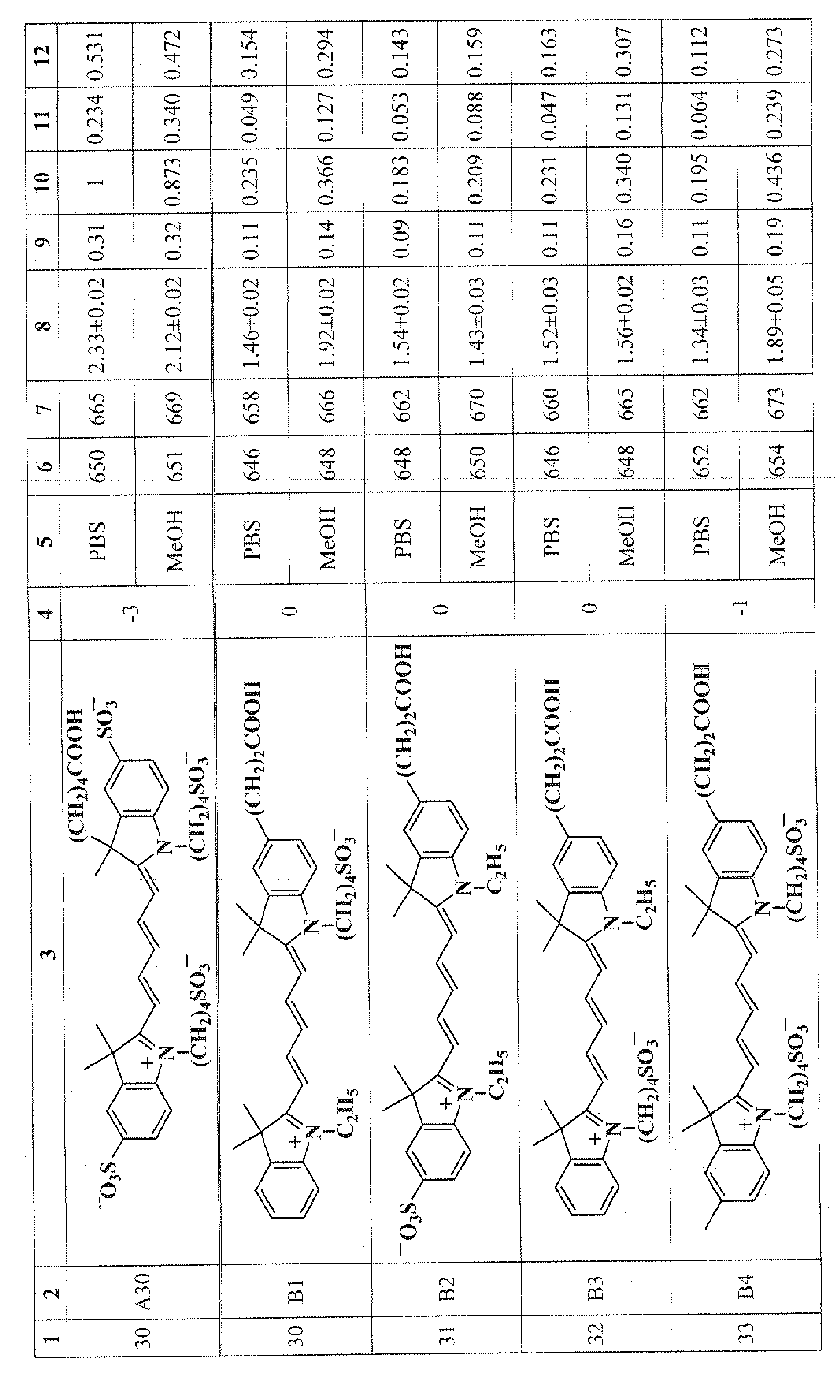

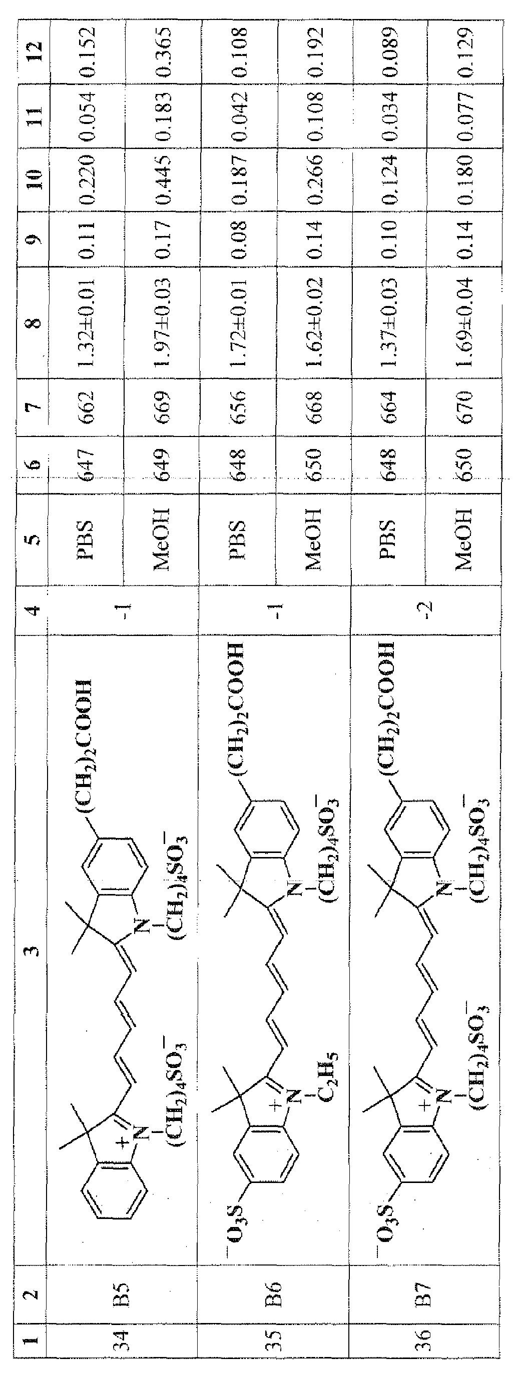

It is another object of the invention to provide method for producing compounds according to the invention (Scheme 2).

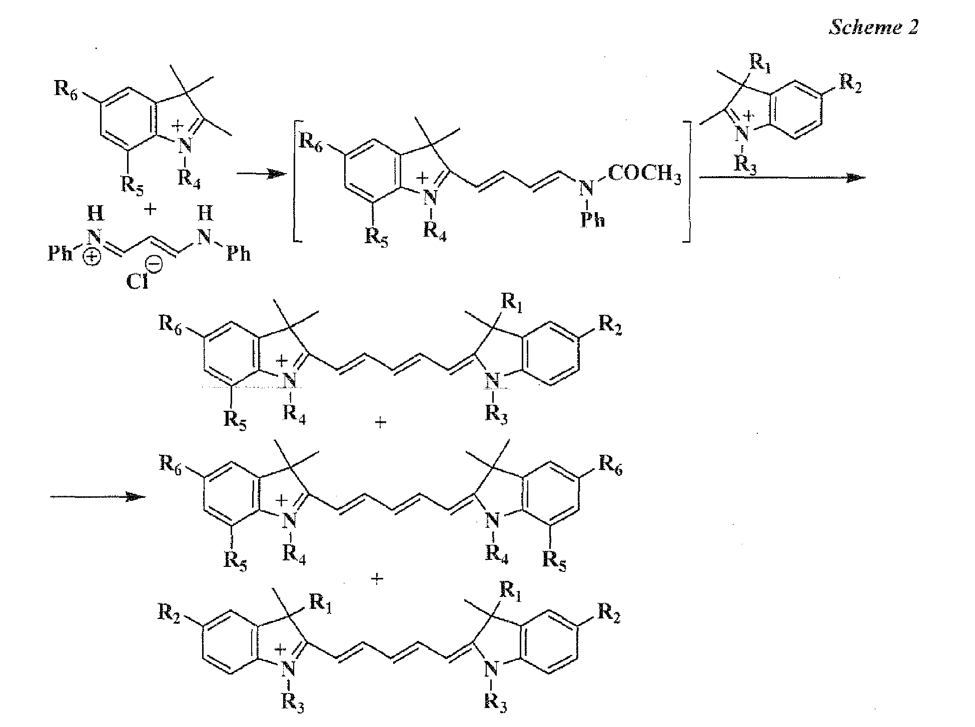

Synthesis of asymmetrical indodicarbocyanine dyes of formulae (**A**) and (**B**) is based upon the condensation reaction of indolenyne salts, comprising the active methylene group, with malonic dialdehyde hydrochloride (Tables 2-8). In the course of reaction, along with desired compound, there are also obtained two symmetrical by-products.

In the first step of the condensation reaction, the sole indolenyne was introduced. The reaction was monitored using a spectrophotometry; there was observed a disappearance of peaks with λ*ₘₐₓ* 237 and 280 nm, being a characteristic of indolenynes, and the appearance of new signal of λ*ₘₐₓ* 440 nm, being a characteristic of the intermediate compound. To complete the reaction, it was sufficient to heat the mixture at 118°C, for 1-3 h.

The second step of producing indodicarbocyanine was performed via two different methods - with the use of diisopropylethyl amine or anhydrous potassium acetate as a condensing agent.

All indodicarbocyanines synthesized were isolated using a reversed phase chromatography in the system of acetonitrile-0.05M TEAA, with gradient elution from 0 to 50% of acetonitrile. Next, there were prepared sodium salts, succinimide, or *para*-nitrophenyl esters thereof. The yield of indodicarbocyanine was between 4 and 72%, subject to the structure of compound.

Methods for producing various dyes differ by the order of reagents mixing, type of condensing agent, solvents ratio, temperature, and time period for reaction.
**Table 2. Synthesis scheme for indodicarbocyanine dyes of formula A wherein R₂= -H; R₃ = -(CH₂)₄SO₃⁻**

**Table 2**

| **Indolenyne** | **Carboxyindolenyne, dianil** | **Indodicarbocyanine** | **Yield** |
|---|---|---|---|
| 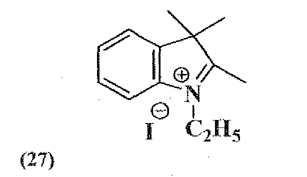 | | 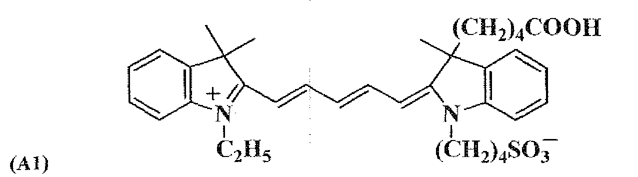 | 37.5% |
| | | | (a*) |
| 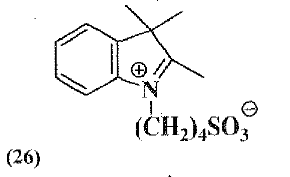 | 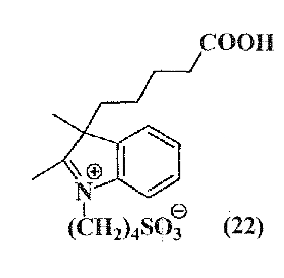 | 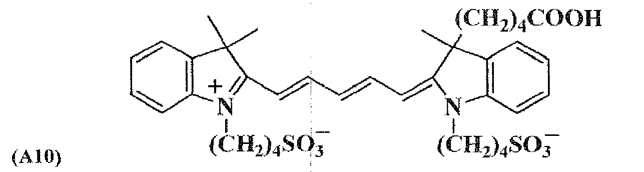 | 48% |
| | | | (c) |
| 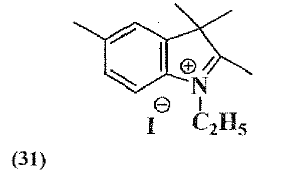 | | 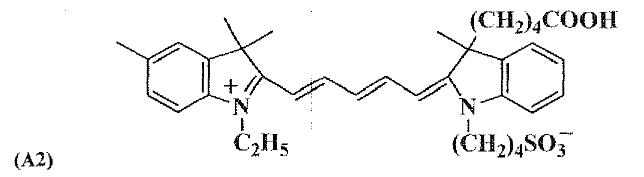 | 31.9% |
| | 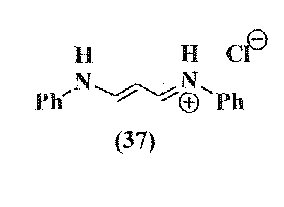 | | (a) |
| 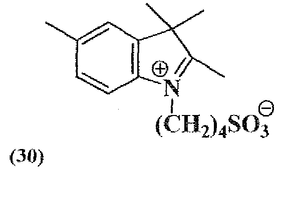 | | 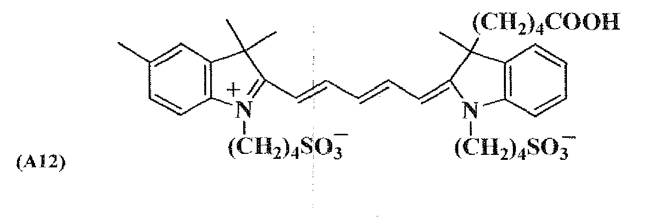 | 35.7% |
| | | | (a) |
| 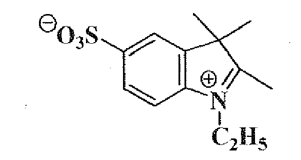 | | 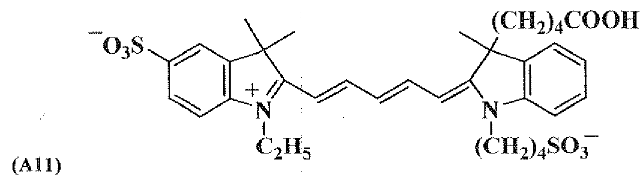 | 22.4% |
| | | | (c) |
| 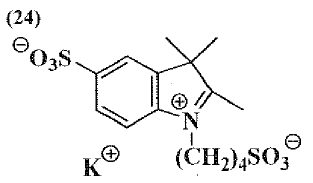 | 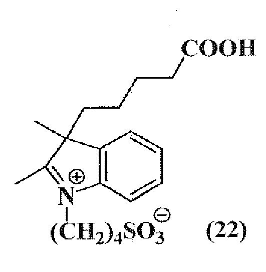 | 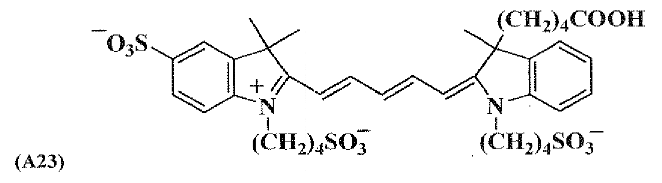 | 32.1% |
| | | | (c) |
| 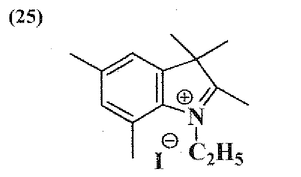 | 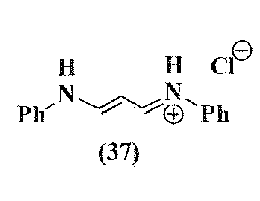 | 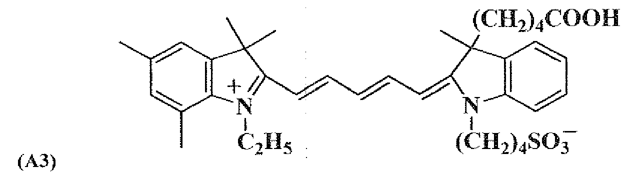 | 20.2% |
| | | | (a) |
| 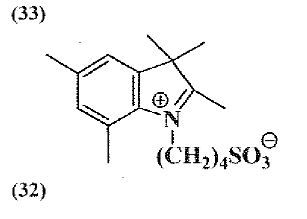 | | 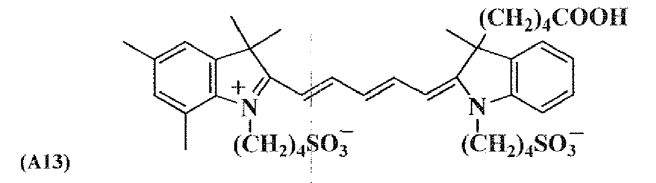 | 5.4% |
| | | | (c) |

| | | | |
|---|---|---|---|
| * Method for producing dyes: **(a)** 1^{st} step: Ac₂O, AcOH; 2^{nd} step: Ac₂O, DIPEA; **(b)** 1^{st} step: Ac₂O, AcCl; 2^{nd} step: Ac₂O, DIPEA; **(c)** 1^{st} step: Ac₂O, AcOH; 2^{nd} step: Ac₂O), AcOH, AcOK | | | |

**Table 3. Synthesis scheme for indodicarbocyanine dyes of formula A wherein R₂= -H; R₃ = -C₂H₅**

**Table 3**

| **Indolenyne** | **Carboxyindolenyne, dianil** | **Indodicarbocyanine** | **Yield** |
|---|---|---|---|
| 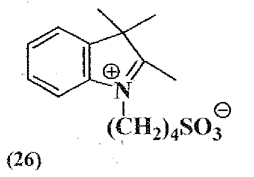 | 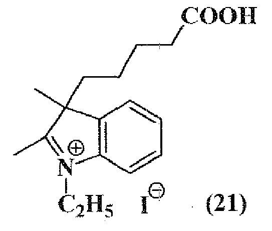 | 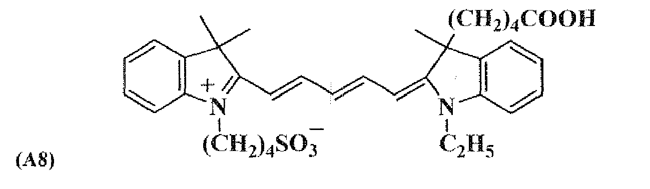 | 28% |
| | | | (b*) |
| 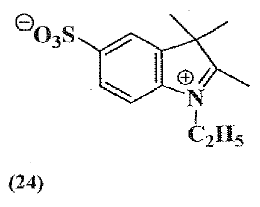 | 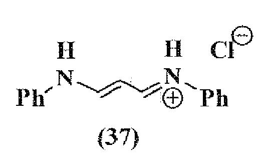 | 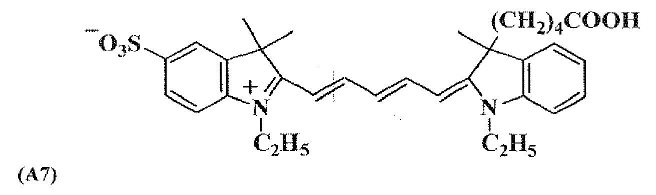 | 26.9% |
| | | | (b) |

| | | | |
|---|---|---|---|
| * Method for producing dyes: **(a)** 1^{st} step: Ac₂O, AcOH; 2^{nd} step: Ac₂O, DIPEA; **(b)** 1^{st} step: Ac₂O, AcCl; 2^{nd} step: Ac₂O, DIPEA; **(c)** 1^{st} step: Ac₂O, AcOH; 2^{nd} step: Ac₂O, AcOH, AcOK | | | |

**Table 4. Synthesis scheme for indodicarbocyanine dyes of formula A wherein R₂= -SO3⁻; R₃ = -(CH₂)₄SO₃⁻**

**Table 4**

| **Indolenyne** | **Carboxyindolenyne, dianil** | I**ndodicarbocyanine** | **Yield** |
|---|---|---|---|
| 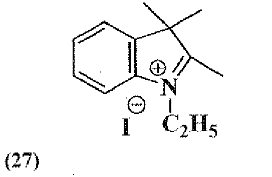 | | 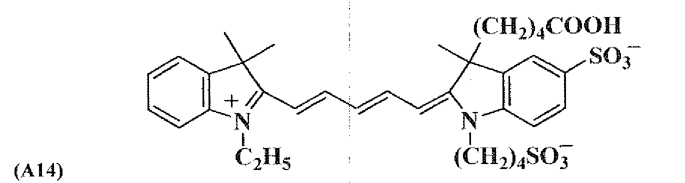 | 7.1% |
| | | | (c*) |
| 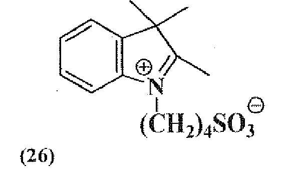 | 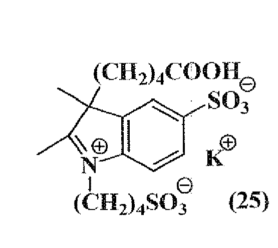 | 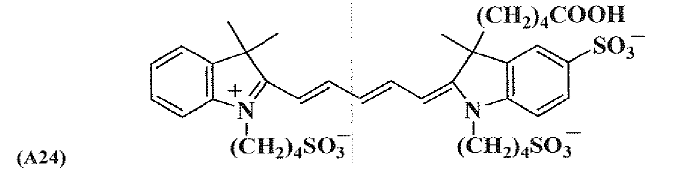 | 24.1% |
| | | | (c) |
| 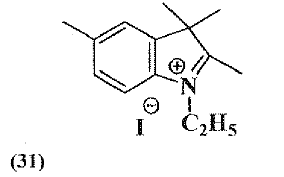 | 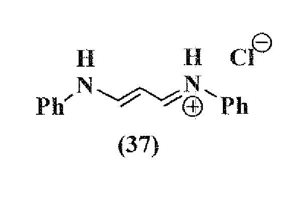 | 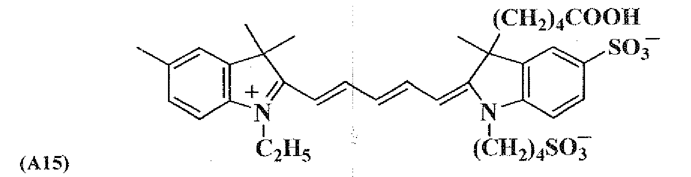 | 9.2% |
| | | | (c) |
| 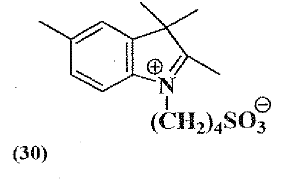 | | 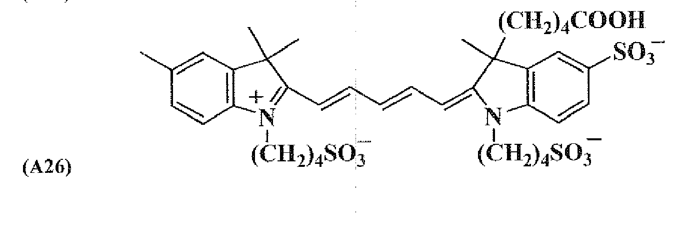 | 40.4% |
| | | | (c) |
| 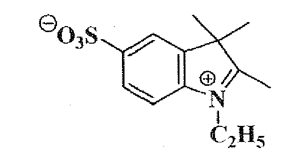 | | 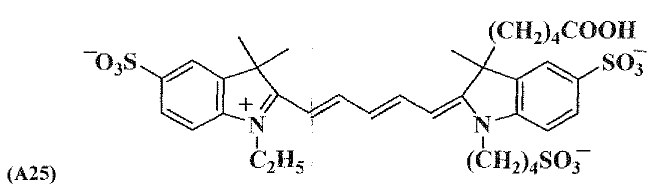 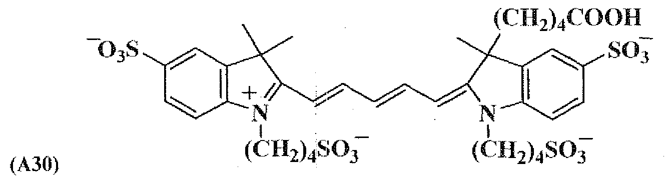 | 29.5% |
| | | | (c) |
| 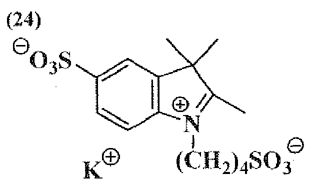 | 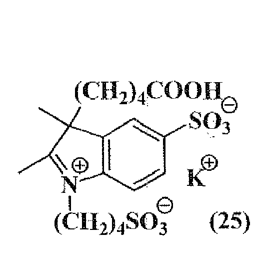 | | 41.4% |
| | | | (c) |
| 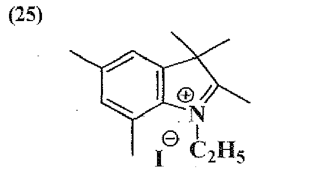 | 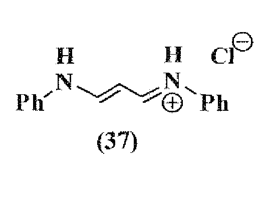 | 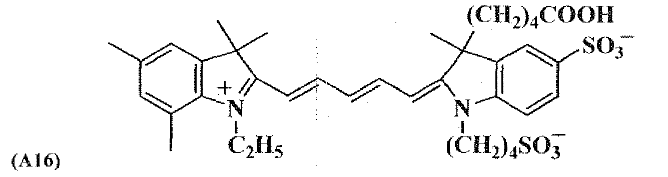 | 5.8% |
| | | | (c) |
| 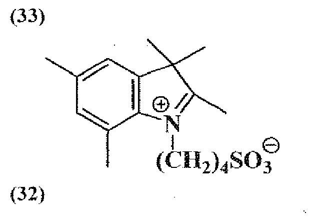 | | 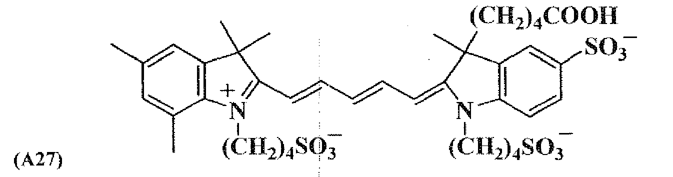 | 4% |
| | | | (c) |

| | | | |
|---|---|---|---|
| * Method for producing dyes: **(a)** 1^{st} step: Ac₂O, AcOH; 2^{nd} step: Ac₂O, DIPEA; **(b)** 1^{st} step: Ac₂O, AcCl; 2^{nd} step: Ac₂O, DIPEA; **(c)** 1^{st} step: Ac₂O, AcOH; 2^{nd} step: Ac₂O, AcOH, AcOK | | | |

**Table 5. Synthesis scheme for indodicarbocyanine dyes of formula A wherein R₂= -SO₃⁻; R₃ = -C₂H₅**

**Table 5**

| **Indolenyne** | **Carboxyindolenyne, dianil** | I**ndodicarbocyanine** | **Yield** |
|---|---|---|---|
| 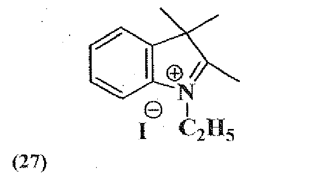 | | 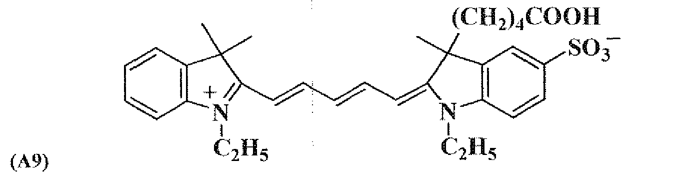 | 17.5% |
| | | | (c*) |
| 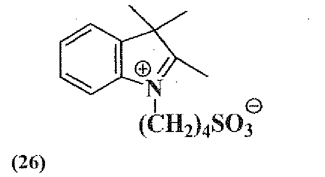 | 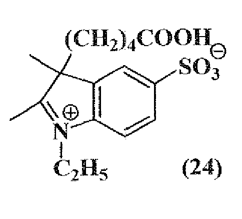 | 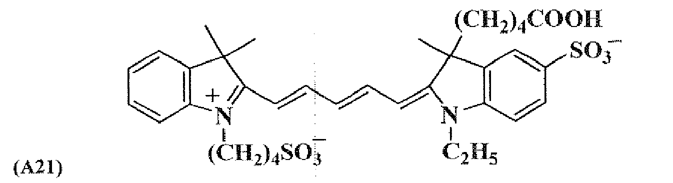 | 20.2% |
| | | | (c) |
| 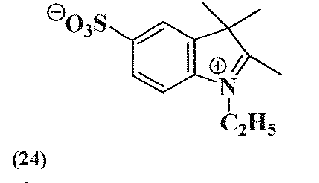 | 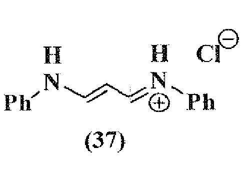 | 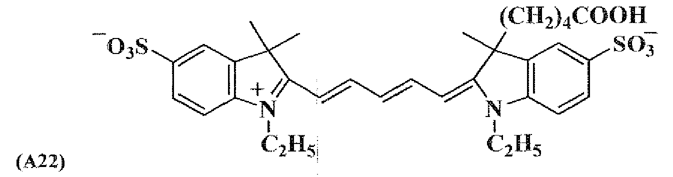 | 13.5% |
| | | | (c) |
| 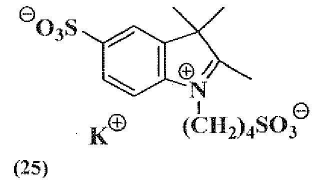 | | 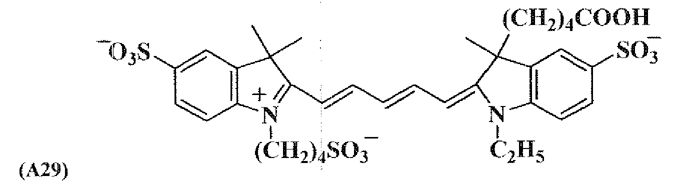 | 18.8% |
| | | | (c) |

| | | | |
|---|---|---|---|
| * Method for producing dyes: **(a)** 1 step: Ac₂O, AcoOH; 2 step: Ac₂O, DIPEA; **(b)** 1 step: Ac₂O, AcCl; 2 step: Ac₂O, DIPEA; **(c)** 1 step: Ac₂O, AcOH; 2 step: Ac₂O, AcOH, AcOK | | | |

**Table 6. Synthesis scheme for indodicarbocyanine dyes of formula A wherein R₂= CH₃; R3 = -(CH₂)₄SO₃⁻**

**Table 6**

| **Indolenyne** | **Carboxyindolenyne, dianil** | **Indodicarbocyanine** | **Yield** |
|---|---|---|---|
| 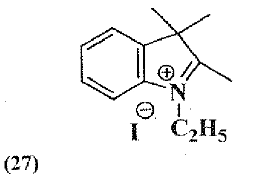 | | 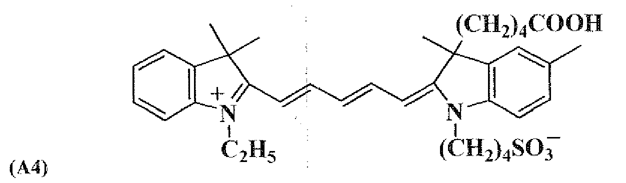 | 18.3% |
| | | | (a*) |
| 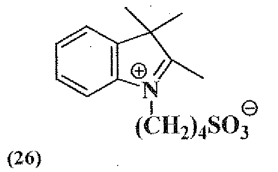 | 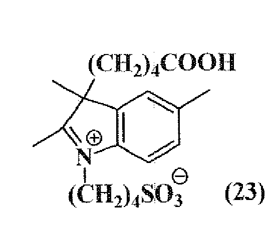 | 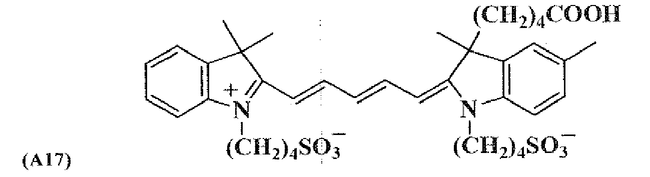 | 28.6% |
| | | | (a) |
| 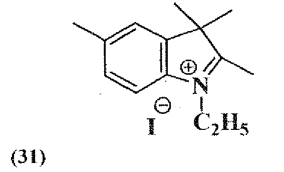 | 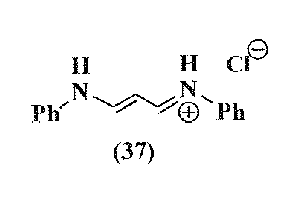 | 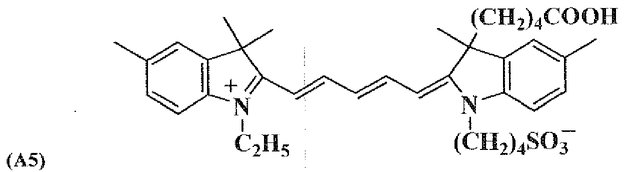 | 27.2% |
| | | | (a) |
| 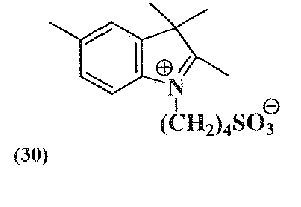 | | 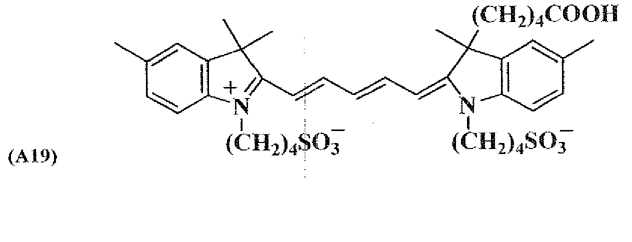 | 22.6% |
| | | | (a) |
| 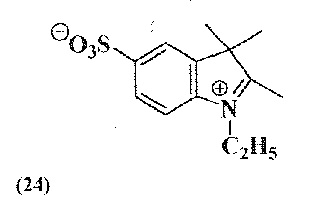 | | 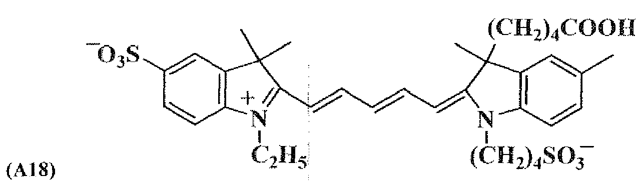 | 17.7% |
| | | | (c) |
| 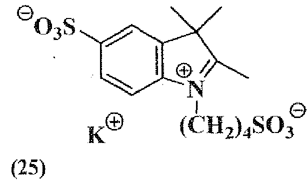 | 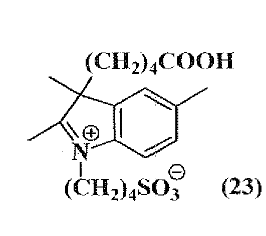 | 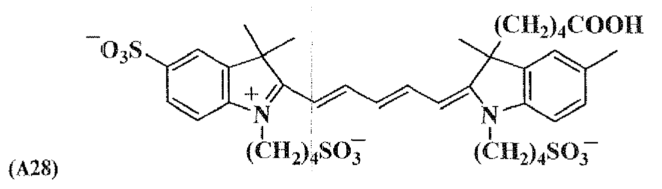 | 50.3% |
| | | | (c) |
| 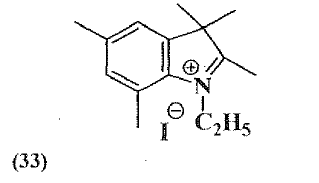 | 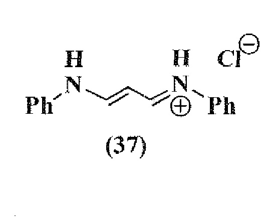 | 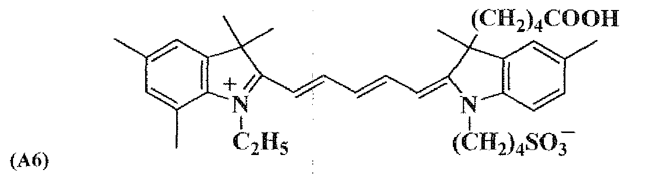 | 17.1% |
| | | | (a) |
| 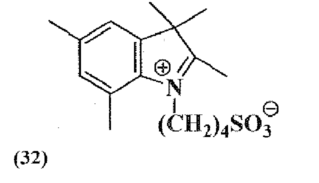 | | 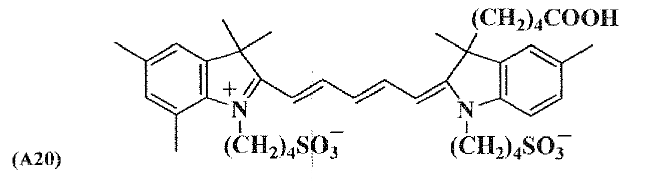 | 4.5% |
| | | | (c) |

| | | | |
|---|---|---|---|
| * Method for producing dyes: **(a)** 1 step: Ac₂O, AcOH; 2 step: Ac₂O, DIPEA; **(b)** 1 step: Ac₂O, AcCl; 2 step: Ac₂O, DIPEA; **(c)** 1 step: Ac₂O, AcOH; 2 step: Ac₂O, AcOH, AcOK | | | |

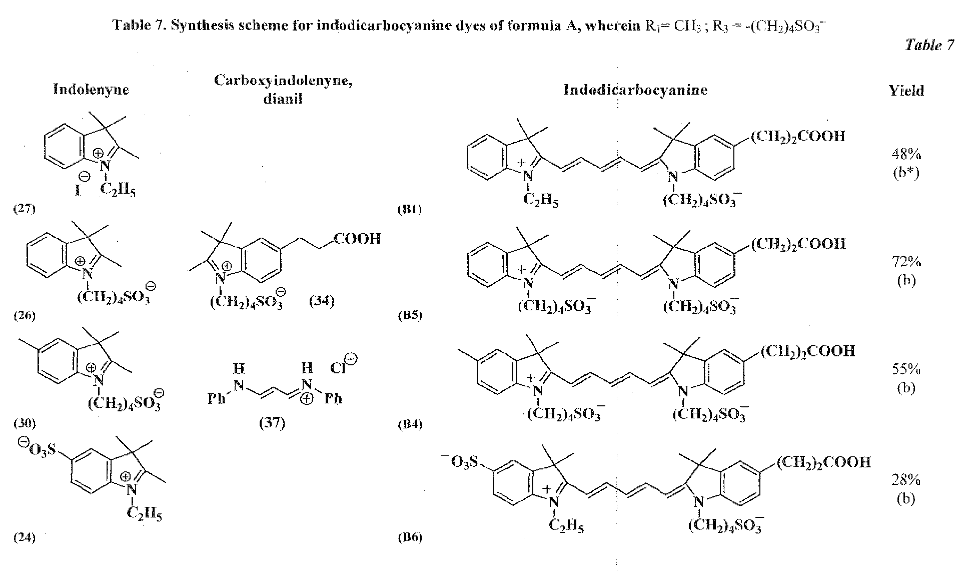

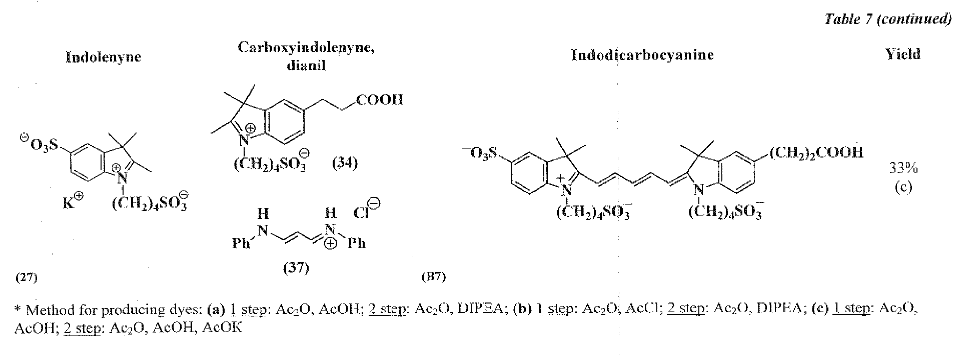

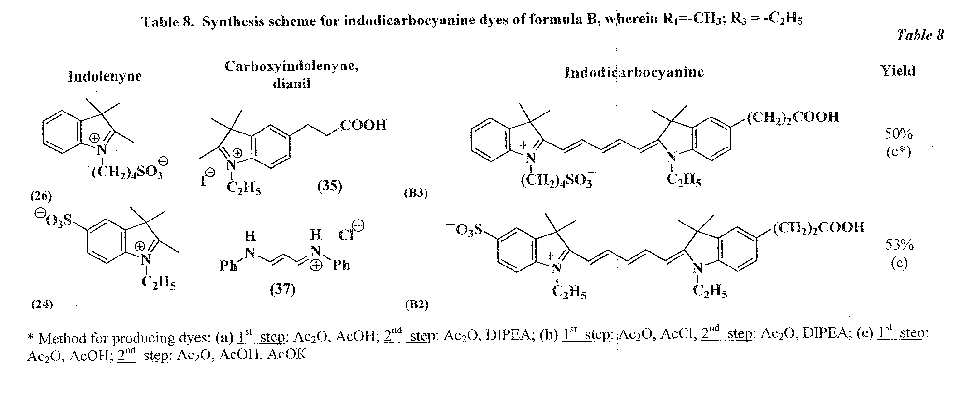

It is another object of the invention to provide method for producing semiproducts and precursors for compounds according to the invention (Schemes 3-19).

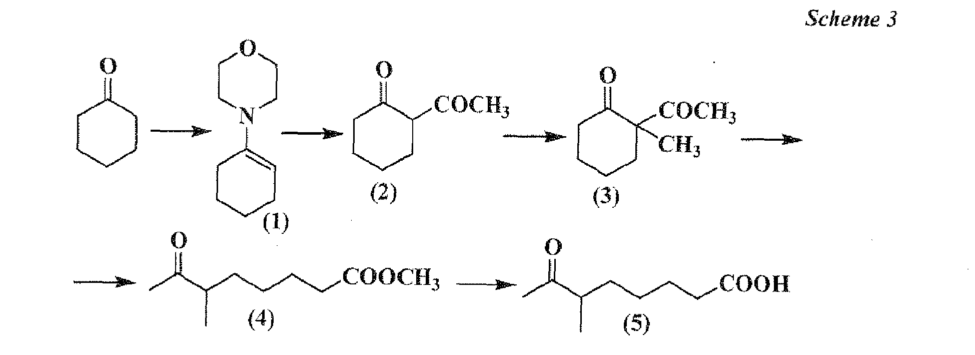

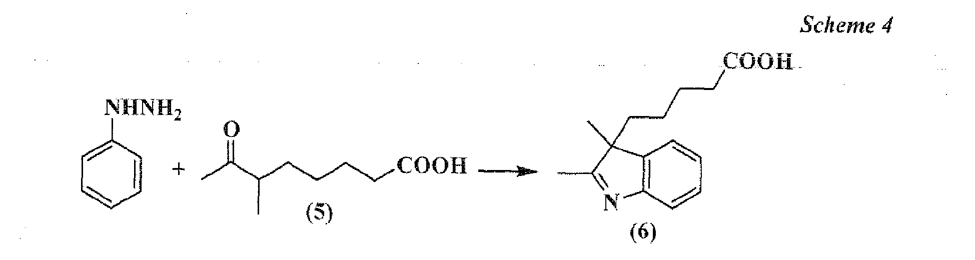

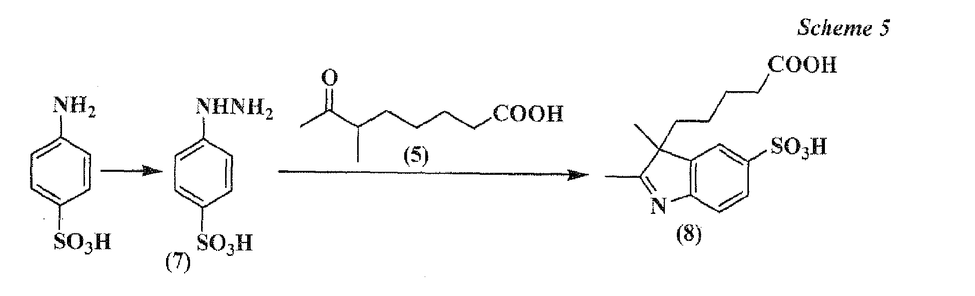

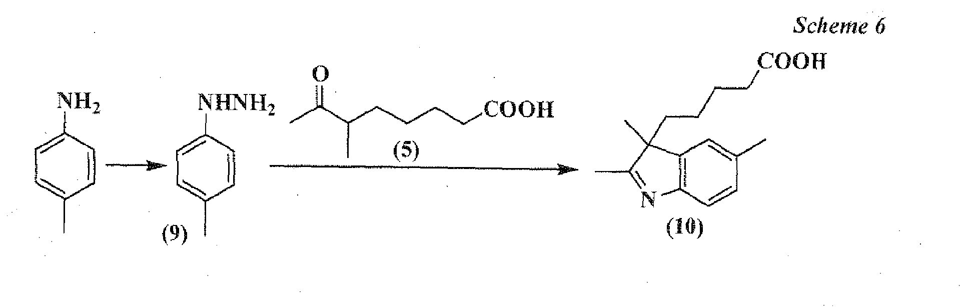

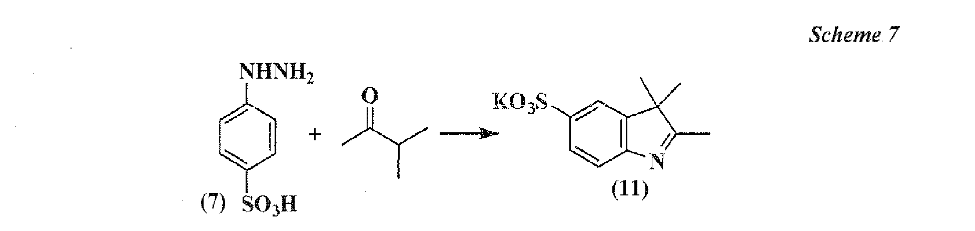

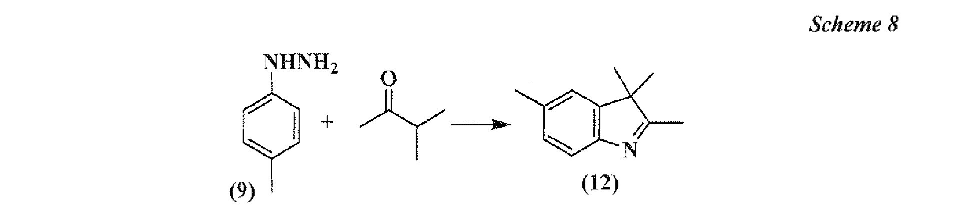

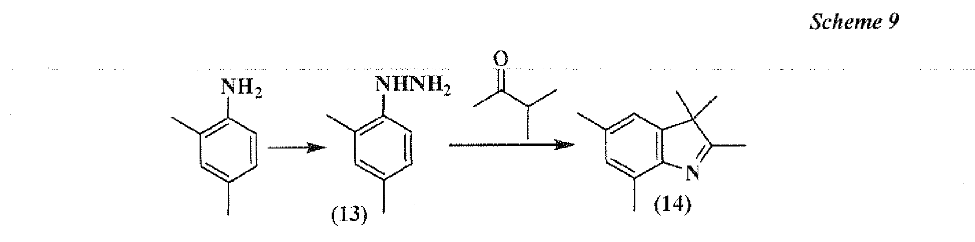

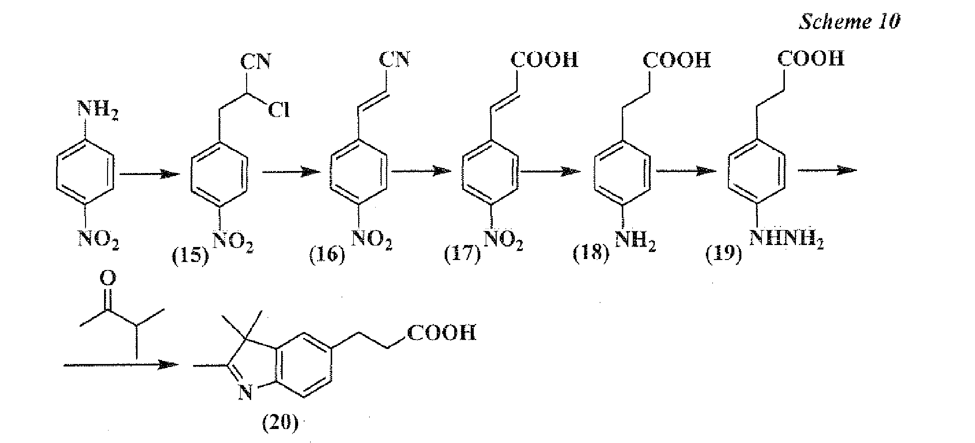

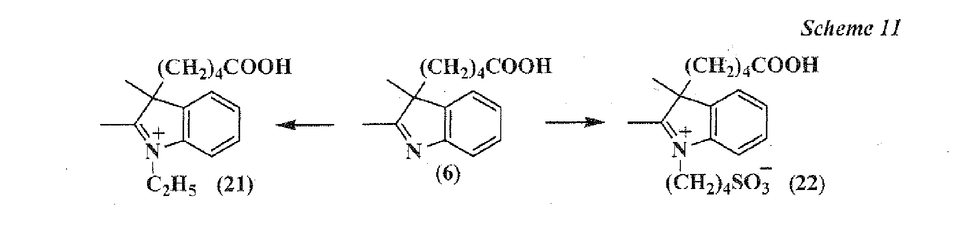

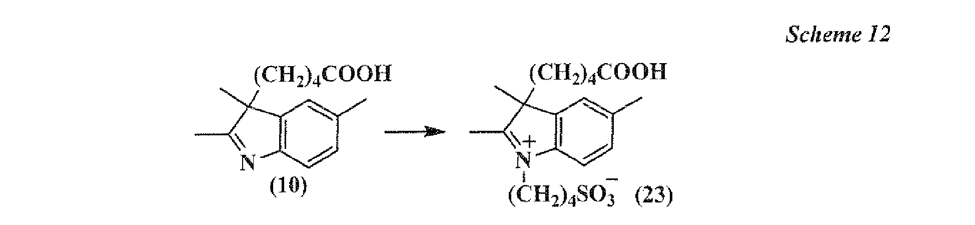

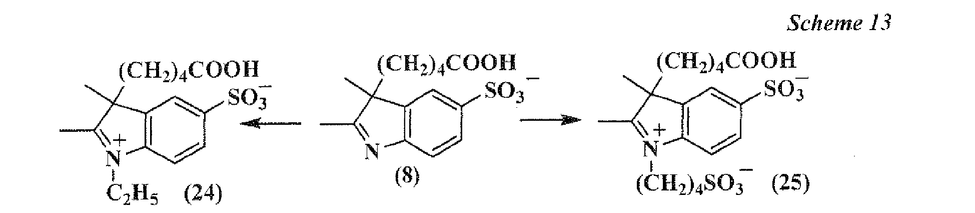

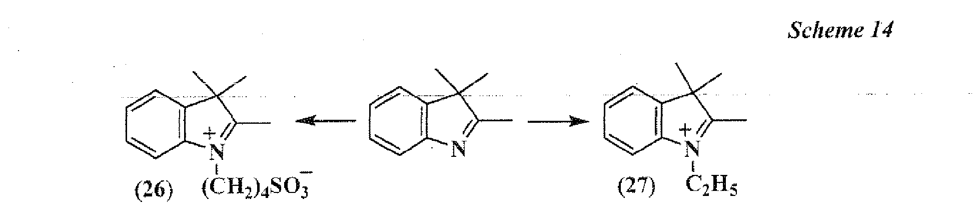

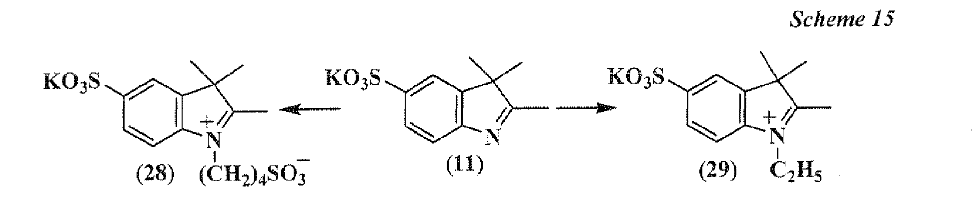

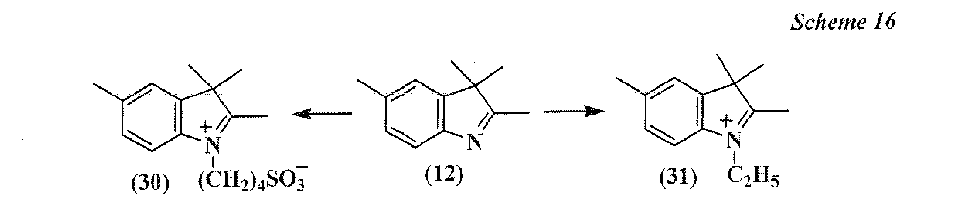

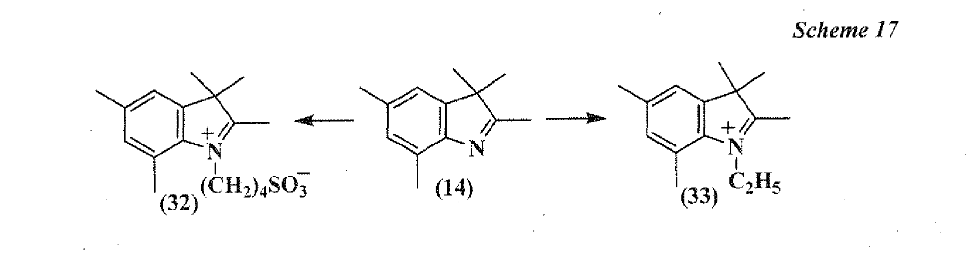

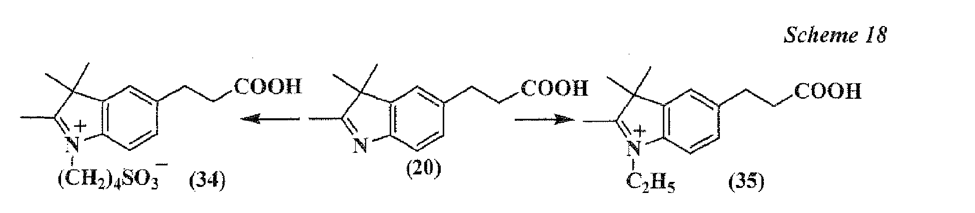

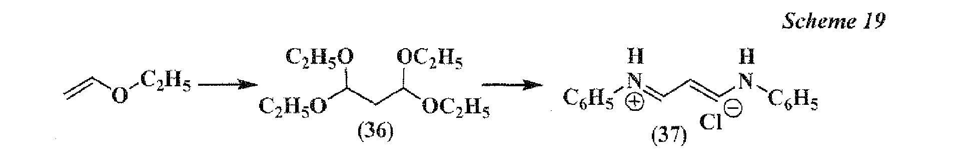

For synthesis of indolenyne (**6**) comprising the functional group at 3-position, the initial compound was 6-methyl-7-oxooctane acid (**5**) (Scheme 3). A cyclohexanone acetylation was performed by two methods: (a) the direct cyclohexanone acetylation with the use of acetic anhydride and boron trifluoride-diacetic acid complex and (b) the acetylation via 1-morpholinocyclohexene (**1**). 2-Acetyl-2-methylcyclohexanone was prepared by action of methyl iodide to the solution of 2-acetylcyclohexanone (**2**) in methyl alcohol. A methyl ester of ketoacid (**4**) was then synthesized using lithium methylate. Ketoacid (**5**) was prepared by hydrolysis of methyl ester with diluted hydrochloric acid. By condensation of phenyl hydrazines (**7, 9**) with 6-methyl-7-okcooctane acid (**5**), there were prepared respective indolenyne bases (**6, 8, 10**) (Scheme 4-6).

By condensation of various phenyl hydrazines with methylisopropyl ketone, there were prepared respective indolenynes (**11, 12, 14**) (Scheme 7-9).

For synthesis of indolenyne comprising the functional group at 5-position, the initial compound was *p*-carboxyethyl phenyl hydrazine hydrochloride (**19**) (Scheme 10). Starting from *p-*nitroaniline, nitrile (**15**) was prepared by action of the acrylonitrile solution in acetone to the diazonium salt being formed in the course of reaction. *p*-Nitrocinnaraic acid nitrile (**16**) was prepared by refluxing the aqueous-alcoholic solution of propionic acid nitrile (**15**) in the presence of sodium acetate. *p*-Nitrocinnamic acid (**17**) was prepared by acidic hydrolysis of respective nitrile. Further reduction of *p*-nitrocinxamic acid (**17**) resulted in aminophenyl propionic acid (**18**). *p*-Carboxyethyl phenyl hydrazine hydrochloride (**19**) was prepared by acid reduction of diazonium salt being formed in the course of reaction. Carboxyethyl derivative of trimethylindolenyne (**20**) was prepared from *p*-carboxy ethylphenyl hydrazine hydrochloride (**19**) and methylisopropyl ketone in glacial acetic acid, in the presence of potassium acetate, without isolation of hydrazone being formed in the course of reaction.

Sulfoalkylation of all indolenyne bases was performed in 1,2-dichlorobenzene or butyronitrile under action of 1,4-butansultone (Scheme 11-18). N-Alkyl derivatives were prepared by action of ethyl iodide to the indolenyne solution in acetonitrile.

Polymethine bridges were prepared as follows (Scheme 19). Malonic aldehyde tetraethylacetal (**36**) was prepared by reaction of ethylvinyl ether with triethyl ortho-formate in the presence of boron trifluoride-etherate. Compound (**37**) was further prepared under action of aniline hydrochloride.

It is another object of the invention to provide method for use of compounds of general formula (**I**) as fluorescent dyes for analysis of biological macromolecules. Said use consists in the following.

The hybridization assay using fluorescent-labeled primers is performed in the following way. By means of chemistry with automatic DNA-synthesizer, there are prepared oligonucleotides, primers containing the phosphate group with aliphatic amine linker at 5'-terminal of oligonucleotide. The structure of primer matches to the fragment structure of supposed DNA-molecule to be analyzed. Then, the oligonucleotide having an amine linker is labeled with one of dyes (**A**) or (**B**). Dyes are applied as appropriate active derivatives - N-hydroxysuccinimide or *para*-nitrophenyl esters. The reaction is carried out in the carbonate-bicarbonate buffer solution mixed with water-organic solvent. Thus obtained fluorescent labeled primers are applied in the polymerase chain reaction (PCR). In this case, the inhibiting effect of markers is not revealed. Provided the structure of analyzed DNA matches the structure of primer used, then amplification and accumulation of PCR product take place. The product of polymerase chain reaction, - amplicon comprises the fluorescent label. The fluorescent labeled amplicon is hybridized over microchip, Based on hybridization results of labeled oligonucleotide over microchips, it follows that dye comprising the sulfo-groups shows higher signal and discrimination effect as compared with hydrophobic dyes.

A microchip consists of supported cells made of hydrogel with immobilized oligonucleotide probes being inside the cells. The cell diameter is approximately 100 microns. Different cells comprise the oligonucleotide probes of various structures.

The binding of amplicon to stable complex, i.e. duplex, takes place in cells, the probe structure whereof matches that of amplicon.

The amplicon binding does not occur in that cells wherein the structure of probe mismatch that of amplicon. The results of analysis are estimated by intensity of fluorescence signals in cells of biochip. The intensive fluorescence signal is observed in cells wherein the fluorescent labeled amplicon binds with probe. To record signals, a use is made of fluorescent microscope equipped with computer having embedded software.

There was determined the relative sensitivity to detect the labeled synthetic oligonucleotides using a portable fluorescent analyzer of biochip's images with the semiconductor laser light source (Fig. 8). There were studied biochips with cells comprising various concentrations of immobilized oligonucleotides. Dye (**A10**) showed the highest recording sensitivity that exceeded sensitivity for dye of formula (**I**) (R₃= -(CH₂)₅COOH, R₁= R₅= -CH₃, R₂=R₆= -SO₃⁻; R₄,= -C₂H₅) by a factor of 1.6.

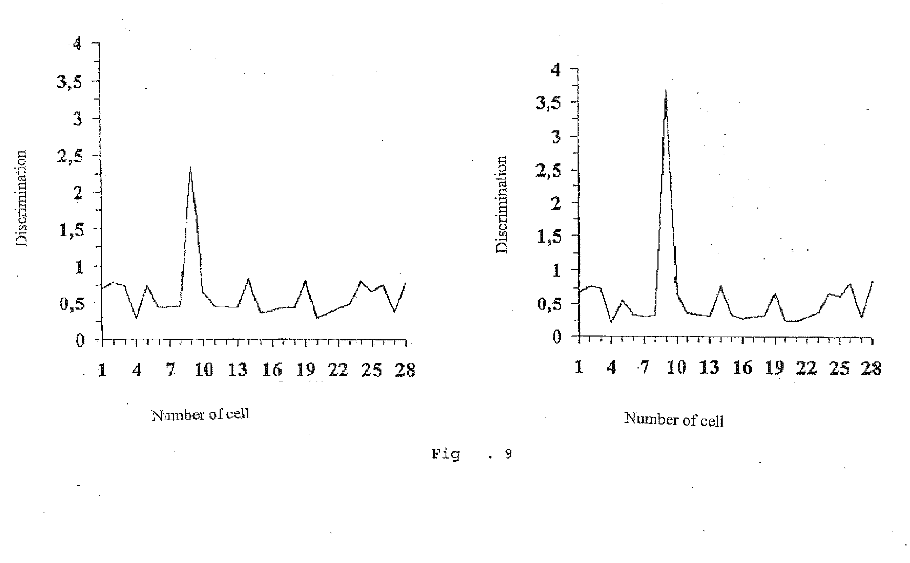

Fig. 9 illustrates the results of detection of tuberculosis mycobacterium mutation by means of hybridization over microchips using dye of formula (**I**) (R₁= -CH₃, R₃= -(CH₂)₅COOH, = -SO₃⁻; R₄= -C₂H₅; R₂= R₅= R₆= -H) and dye (**A10**). Second chart (**b**) shows two times higher discrimination effect, it means that dye (**A10**) has higher sensitivity in detection of mutation over biochips.

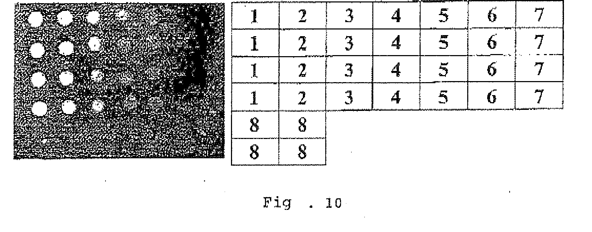

A use of claimed dyes of formula (**A**) and (**B**) as fluorescent labels in technology of protein microchip is another example of application thereof. To solutions IgG having initial concentrations of 6.5 mg/ml, there are respectively added succinimide esters of dye of formula I (R₁= -CH₃, R₂=R₅=R₆= -H; R₃= -(CH₂)₅COOH, R₄= -C₂H₅) and dye (**A10**). Staining is performed with mixing in bicarbonate buffer for 4 h at 4°C. The excess of dye is separated using spin-column with Sephadex G-25. Antibodies stained are analyzed using microchip having different concentrations of immobilized protein A (Fig. 10).

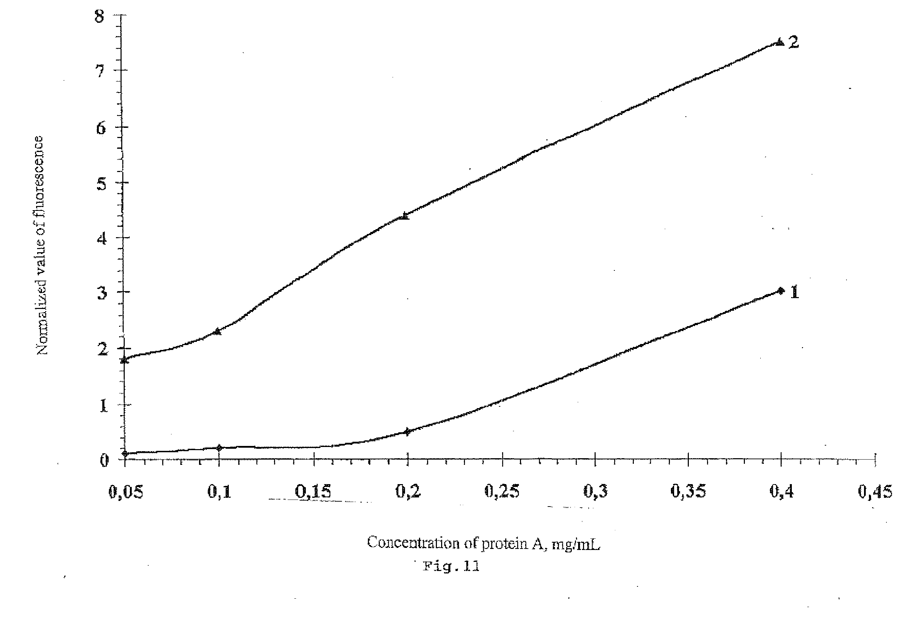

Cell signals are recorded by means of research fluorescent microscope, and dependence of fluorescence versus concentration of immobilized protein being observed (Fig. 11),

Normalized signal of fluorescence is higher for dye (**A10**) as compared with dye of formula **I** (R₁= -CH₃, R₂= R₅= R₆= -H; R₃= -(CH₂)₅COOH, R₄= -C₂H₅), it means that dye (**A10**) has higher sensitivity of proteins record over biochips.

Hybridization assay with the use of claimed dyes of formulae (**A**) and (**B**) is characterized by peculiarities as follows:
- claimed dyes included in conjugates with oligonucleotides does not inhibit the polymerase chain reaction;
- claimed dyes are distinguished by the extremely high sensitivity of recording - up to 3*10⁻¹⁸ mole);
- the reproducibility of analysis results increases with rise in quantity of sulfo-group in molecule of claimed dyes;
- claimed dyes not containing substituents in phenyl ring of indolenyne moiety have the increased relative light resistance.

Having regard to the foregoing, it follows that indodicarbocyanine dyes claimed (**A1-A30**, **B1-B7**) may be successfully used as fluorescent markers in hybridization assay over biological microchips of both oligonucleotide and protein nature.

Another object of the invention is intermediate compounds to synthesize compounds according to the invention being selected from the group consisting of:
2,3-dimethyl-3-(4-carboxybutyl)-indolenyne (compound **6**);
2,3-dimethyl-3-(4-carboxybutyl)-5-sulfoindolenyne, potassium salt (compound **8**);
3-(4-carboxybutyl)-2,3,5-trimethylindolenyne (compound **10**),
5-carboxyethyl-2,3,3-trimethylindolenyne (compound **20**);
2,3-dimethyl-3-(4-carboxybutyl)-*N*-ethylindolenyneum iodide (compound **21**);
2,3-dimethyl-3-(4-carboxybutyl)-*N*-(4-sulfobutyl)-indolenyne (compound **22**);
3-(4-carboxybutyl)-*N*-(4-sulfobutyl)-2,3,5-trimethylindolenyne (compound **23**);
2,3-dimethyl-3-(4-carboxybutyl)-5-sulfo-*N*-ethylindolenyne (compound **24**);
2,3-dimethyl-3-(4-carboxybutyl)-*N*-(4-sulfobutyl)-5-sulfoindolenyne, potassium salt (compound **25**);
5-carboxyethyl-*N*-sulfobutyl-2,3,3-trimethylindolenyne (compound **34**);
5-carboxyethyl-2,3,3-trimethyl-*N*-ethylindolenyneum iodide (compound **35**).

### Detailed Description of the Invention

The preferred Examples of compounds, methods for producing and utilization thereof according to the invention

### Example 1. Preparation of 1-morpholinocyclohexene (compound 1)

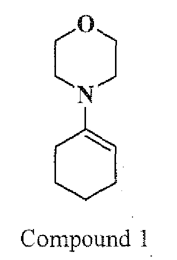

A mixture of morpholine (100.0 mL; 100.1 g; 1.15 mole), cyclohexanone (99,5 mL; 94.2 g; 0.96 mole), toluene (190 mL) and *p*-toluenesulfonic acid (1 g) was refluxed in a round-bottom flask equipped with backflow condenser and Dean-Stark trap, until water separation ceased (∼6 h).

Then, toluene was removed and the residue was fractionated under vacuum. Fraction having Bp= 114°C/8 mm Hg was drawn to obtain 1-morphoiinocyclohexene (compound 1) yield of 111.1 g (69.2%).

¹H-NMR (CDCl₃), δ (ppm): 1.55 (2H, m, 4-CH₂); 1.67 (2H, m, 3-CH₂); 2.05 (4H, m, 5-CH₂, 6-CH₂); 2.77 (4H, t, J=5, 2'-CH₂,6'-CH₂); 3.72 (4H, t, J=5, 3'-CH₂, 5'-CH₂); 4.66 (1H, t, J=4, 2-CH).

### Example 2. Preparation of 2-acetylcyclohexanone (compound 2)

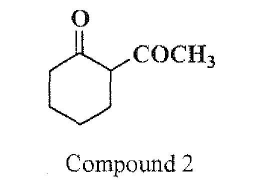

Method A. A three-necked flask equipped with backflow condenser, mechanical stirrer, thermometer, and dropping funnel was charged with anhydrous chloroform (475 mL), 1-morpholinocyclohexene (compound 1) (63.0 g; 0.38 mole), and triethylamine (56.7 mL; 41.28 g; 0.41 mole) rectified over sodium. To the reaction mixture chilled to 0°C, the solution of acetyl chloride (27.7 mL; 30.61 g; 0.39 mole) in anhydrous chloroform (140 mL) was added dropwise at such a rate to maintain the reaction temperature not higher than 0°C. The reaction mixture was further stirred on cooling with ice for 1 h, then, it was kept at room temperature for 12 h. Next, the reaction mixture was transferred to round-bottom flask equipped with backflow condenser and mechanical stirrer, the solution of concentrated hydrochloric acid (50.4 mL) in water (126 mL) was added thereto, and the reaction was refluxed under continuous stirring for 5 h. After chilling, the chloroform layer was separated, washed with water portions by 200 mL (up to pH 5-6), saturated solution of sodium chloride, and dried over sodium sulphate. Chloroform was removed and the residue was fractionated under vacuum. Fraction having Bp= 102-104°C/10 mm Hg was drawn to obtain 2-acetylcyclohexanone (compound 2) yield of 29.0 g (55%).

¹H- NMR (CDCl₃), δ (ppm): 1.67 (4H, m, 4-CH₂, 5-CH₂); 2.1 (3H, s, COCH₃); 2.31 (4H, m, 3-CH₂, 6-CH₂); 15.9 (1H, s, 2-CH)

Method B. A two-necked heart-shaped flask, equipped with backflow condenser and mechanical stirrer, was charged with cyclohexanone (5.2 mL; 4.9 g; 0.05 mole) and acetic anhydride (9.5 mL); 10.2 g; 0.1 mole). The reaction mixture was chilled and added BF₃*2CH₃COOH complex (13.9 mL; 18.8 g; 0.1 mole) at once. The reaction was stirred at room temperature for a period of day. Then, round-bottom flask equipped with backflow condenser and mechanical stirrer was charged with sodium acetate trihydrate (27.2 g; 0.2 mole) and water (150 mL). To the solution obtained, the reaction mixture from heart-shaped flask was transferred, and emulsion was refluxed under vigorous stirring for 3 h. The reaction mixture was further extracted with ether (3*80 mL). The combined ethereal extract was washed with saturated solution of sodium hydrocarbonate until gas evolution ceased, and dried over magnesium sulphate (MgSO₄). Ether was removed and the residue was fractionated under vacuum and nitrogen flow. Fraction having Bp= 105-108°C/17 mm Hg was drawn to obtain 2-acetylcyclohexanone (compound 2) yield of 3.8 g (54.4%).

¹H- NMR (CDCl₃), δ (ppm): 1.69 (4H, m, 4-CH₂, 5-CH₂); 2.13 (3H, s, 2-COCH₃); 2.33 (4H, m, 3-CH₂, 6-CH₂); 15.8 (1H, s, 2-CH)

### Example 3. Preparation of 2-acetyl-2-methylcyclohexanone (compound 3)

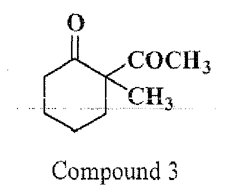

A three-necked round-bottom flask equipped with backflow condenser, mechanical stirrer, and thermometer, on cooling with ice, was charged with methyl alcohol (83 mL), 2-acetylcyclohexanone (compound 2) (29.0 g; 26.9 mL; 0.21 mole), and methyl iodide (44.0 g; 19.3 mL; 0.31 mole). Under stirring, the solution of sodium hydroxide (8.27 g, 0.21 mole) in water (41.4 mL) was added dropwise to the reaction mixture. The reaction was stirred at room temperature for 20 h and, then, refluxed for 2 h. After chilling, the reaction mixture was transferred to a separating funnel, water (85 mL) was added, and the mixture was extracted with chloroform (2*85 mL). Combined chloroform extracts were washed with 2M sodium hydroxide (200 mL), saturated solution of sodium chloride (100 mL), and dried over MgSO₄. The solvent was removed and the residue was fractionated under vacuum and nitrogen flow. Fraction having Bp= 123-128°C/32 mm Hg was drawn to obtain 2-acetyl-2-methylcyclohexanone (compound 3) yield of 23.8 g (74.7%).

¹H- NMR (CDCl₃), δ (ppm): 1.23 (3H, s, 2-CH₃); 1.41-1.48 (1H, m, 4-CH₂); 1.61-1.69 (3H, m, 4-CH₂, 5-CH₂); 1.93-1.99 (1H, m, 3-CH₂); 2.09 (3H, s, 2-COCH₃); 2.25-2.34 (1H, m, 3-CH₂); 2.42-2.48 (2H, m, 6-CH₂)

### Example 4. Preparation of 6-methyl-7-oxooctane acid, methyl ester (compound 4)

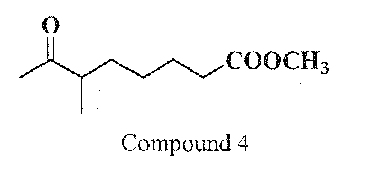

A two-necked flask equipped with backflow condenser and mechanical stirrer was charged with anhydrous methyl alcohol (52.4 mL) and lithium metal (0.5 g; 0.071 mole) was added as small bits. 2-Acetyl-2-methylcyclohexanone (compound 3) (10.0 g; 0.0648 mole) was added to the solution obtained and the mixture was refluxed for 1 h and allowed to hold at room temperature for 12 h. Next, the reaction mixture was acidified with concentrated sulphuric acid to pH 5, slurry was transferred to a separating funnel, water (55 mL) was added, and the mixture was extracted with ether to decolourize a water phase. Combined ethereal extracts were washed with saturated solution of sodium chloride and dried over MgSO₄. The solvent was removed and the residue was fractionated under vacuum. Fraction having Bp= 139-145°C/20 mm Hg was drawn to obtain 6-methyl-7-oxooctane acid, methyl ester (compound 4) yield of 6.2 g (52%).

¹H- NMR (CDCl₃), δ (ppm): 1.06 (3H, d, 6-CH₃); 1.23-1,35 (3H, m, 5-CH₂, 4-CH₂); 1.56-1.69 (3H, m, 3-CH₂, 4-CH₂); 2.11 (3H, s, 8-CH₃); 2.28 (2H, t, J=7.5, 2-CH₂); 2.49 (1H, m, 6-CH)

### Example 5. Preparation of 6-methyl-7-oxooctane acid (compound 5)

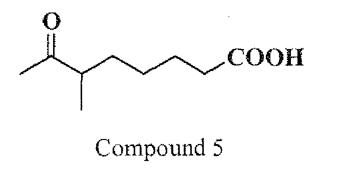

A mixture of diluted (2:1) hydrochloric acid (37 mL) and 6-methyl-7-oxooctane acid, methyl ester (compound 4) (9.11 g; 0.049 mole) was refluxed under stirring for 6 h. The reaction produced two immiscible layers. The reaction mixture was extracted with ether. Ethereal extracts were combined. The solvent was removed and the residue was fractionated under vacuum. Fraction having Bp= 100-143°C/0.1-0.01 mm Hg was drawn to obtain 6-methyl-7-oxooctane acid (compound 5) yield of 5.9 g (75%).

¹H-NMR (CDCl₃), δ (ppm): 1.06 (3H, d, 6-CH₃); 1.25-1.39 (3H, m, 5-CH₂, 4-CH₂); 1.58-1.69 (3H, m, 3-CH₂, 4-CH₂); 2.12 (3H, s, 8-CH₃); 2.33 (2H, t, J=7.5, 2-CH₂); 2.5 (1H, m, 6-CH).

### Example 6. Preparation of 2,3-dimethyl-3-(4-carboxybutyl)indolenyne (compound 6)

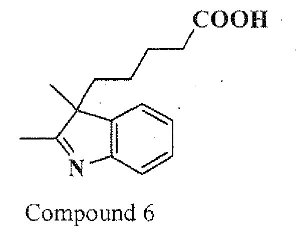

A glass vial was charged with 6-methyl-7-oxooctane acid (compound 5) (723.3 mg; 4.2 mmole), phenyl hydrazines (454.2 mg; 413.7 µL; 4.2 mmole), and glacial acetic acid (4.2 mL). The mixture was heated at 118°C for 3.5 h. The solvent was removed and the oily residue was dissolved in chloroform. The chloroform solution was washed with water, saturated solution of sodium chloride, and dried over MgSO₄. The solvent was removed and the residue subjected to recrystallization from ethyl acetate-hexane (0.6:2) mixture. 2,3-Dimethyl-3-(4-carboxybutyl)-indolenyne (compound 6) was obtained as a yellow-orange powder, yield was 0.8 g (78%).

λₘₐₓ 256 nm. Mass-spectrum (MALDI) (C₁₅H₁₉NO₂): found m/z 246.3, calculated m/z 245.32. ¹H- NMR (CDCl₃), δ (ppm): 0.56-0.81 (2H, m, CH₂CH₂CH₂CH₂COOH); 1.28 (3H, s, 3-CH₃); 1.43-1.51 (2H, m, CH₂CH₂CH₂CHCOOH); 1.76-1.92 (2H, m, CH₂CH₂CH₂CH₂COOH); 2.13-2.21 (2H, m, CH₂CH₂CH₂CH₂COOH); 2.24 (3H, s, 2-CH₃); 7.18-7.54 (4H, m, ArH)

### Example 7. Preparation of phenylhydrazine-p-sulfuric acid (compound 7)

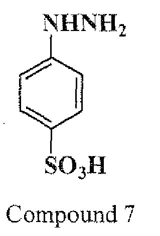

Sulfanilic acid (52.0 g; 0.3 mole) and sodium carbonate (16.5 g; 0.06 mole) were dissolved in water (200 mL) on heating. The solution obtained was filtered off and concentrated sulphuric acid (35.0 g; 19.1 mL; 0.36 mole) was added thereto, with white precipitate being formed. To this slurry, on cooling with ice and stirring, the solution of sodium nitrite (21.0 g; 0.3 mole) in water (50 mL) was added dropwise at such a rate to maintain reaction temperature not higher than 12°C. The additional stirring was continued for 15 min. The obtained precipitate of diazo compound was collected by filtration and washed with small amount of ice water.

To solution of sodium sulphite (84.8 g; 0.67 mole) in water (250 mL), under stirring and cooling with ice and salt, the crude diazo compound was added with portions at such a rate to maintain reaction temperature lower than 5°C. The mixture was additionally stirred for 1 h, then, heated to boiling and added dropwise the concentrated hydrochloric acid (200 mL), with precipitate being formed. A zinc powder (2 g) was added to discolour the mixture completely. The reaction mixture was allowed to hold at room temperature for 12 h. The precipitate was collected by filtration, washed with cold water, and air dried. Phenylhydrazine-*p*-sulfonic acid (compound 7) was obtained with yield of 41.7 g (74%).

### Example 8. Preparation of 2,3-dimethyl-3-(4-carboxybutyl)-5-sulfoindolenyne, potassium salt (compound 8)

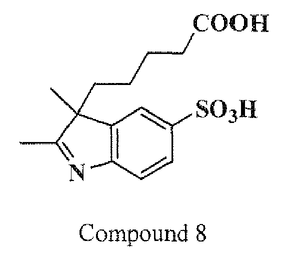

A mixture of phenylhydrazine-p-sulphonic acid (compound 7) (1.8 g; 9.7 mmole), 6-methyl-7-oxooctane acid (compound 5) (2.0 g; 11.6 mmole), and glacial acetic acid (15 mL) was refluxed under stirring for 8 h, then an additional amount of acetic acid was added and treated with activated carbon. The solvent was removed and the residue was dissolved in methanol (10 mL). To solution obtained, there are added dropwise the solution of potassium hydroxide (600 mg; 10,56 mmole) in methanol (7 mL), isopropyl alcohol (15 mL), and diethyl ether (2 mL). The precipitate formed was collected by filtration and dried in vacuum desiccator over P₂O₅. 2,3-Dimethyl-3-(4-carboxybutyl)-5-sulfoindolenyne, potassium salt (compound 8) was obtained with yield of 3.47 g (83%), λₘₐₓ 258 nm. Mass-spectrum (MALDI) (C₁₅H₁₉NO₅S): found m/z 327.2, calculated m/z 325.38.

¹H-NMR (D₂O), δ (ppm): 0.45-0.7 (2H, m, CH₂CH₂CH₂CH₂COOH); 1.31 (3H, s, 3-CH₃); 1.36 (2H, m, CH₂CH₂CH₂CH₂COOH); 1.88-2.14 (4H, m, CH₂CH₂CH₂CH₂COOH); 7.53 (1H, d, ArH), 7.8 (2H, m, ArH)

### Example 9. Preparation of 4-tolyl hydrazine, hydrochloride (compound 9)

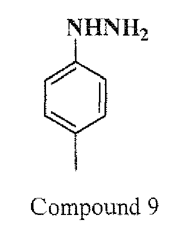

To a mixture of *p*-toluidine (32.2 g; 0.3 mole) and concentrated hydrochloric acid (220 mL) chilled to 0°C, the solution of sodium nitrite (20.6 g; 0.3 mole) in water (60 mL) was added dropwise at such a rate to maintain reaction temperature not higher than 5°C. Then, the solution prepared was filtered off and was added to a solution of stannous chloride (124 g; 0.65 mole) in concentrated hydrochloric acid (124 mL) chilled to 5°C, while vigorous stirring. The precipitate formed was collected by filtration, slurried in water (500 mL), and alkalized to pH 12 with solution of sodium hydroxide (40%). The reaction mixture was extracted with diethyl ether (3*200 mL). Ethereal extracts were dried over MgSO₄. Then, a stream of dried hydrogen chloride was bubbled via the ethereal solution of *p*-tolyl hydrazine. The precipitate formed was collected by filtration, washed with ether, and dried in vacuum desiccator over P₂O₅. 4-Tolylhydrazine hydrochloride (compound 9) was obtained with yield of 30.2 g (63%). λₘₐₓ 283 nm. Mass-spectrum (MALDI) (C₇H₁₀N₂): found m/z 122.1, calculated m/z 122.17.

¹H- NMR (D₂O), δ (ppm): 2.11 (3H, s, CH₃), 6.79 (2H, d, ArH), 7.06 (2H, d, ArH)

### Example 10. Preparation of 3-(4-carboxybutyl)-2,3,5-trimethylindolenyne (compound 10)

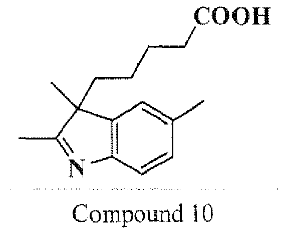

A mixture of 4-tolylhydrazine hydrochloride (compound 9) (825 mg; 5.2 mmole), glacial acetic acid (8 mL), 6-methyl-7-oxooctane acid (compound 5) (913 mg; 5.3 mmole), and anhydrous potassium acetate (980 mg; 10 mmole) was refluxed under stirring for 6 h. The solvent was removed, and the residue was dissolved in chloroform. The chloroform solution was washed with water, saturated solution of sodium chloride, and then dried over MgSO₄. The solvent was removed and the residue was subjected to recrystallization from a mixture of diethyl ether - hexane, 1:10. 3-(4-Carboxybutyl)-2,3,5-trimethylindolenyne (compound 10) was obtained as a dark orange powder with yield of 1.13 g (83.7%). λₘₐₓ 254 nm. Mass-spectrum (MALDI) (C₁₆H₂₁NO₂): found m/z 260.7, calculated m/z 259.34.

¹H-NMR (CDCl₃), δ (ppm): 0.6-0.83 (2H, m, CH₂CH₂CH₂CH₂COOH); 1.25 (3H, s, 3-CH₃); 1.46 (2H, m, CH₂CH₂CH₂CH₂COOH); 1.72-1.88 (2H, m, CH₂CH₂CH₂CH₂COOH); 2.09-2.18 (2H, m, CH₂CH₂CH₂CH₂COOH); 2.28 (3H, s, 2-CH₃); 2.37 (3H, s, 5-CH₃), 7,01-7.4 (3H, m, ArH), 10.11 (1H, s, COOH)

### Example 11. Preparation of 5-sulfo-2,3,3-trimethylindolenyne, potassium salt (compound 11)

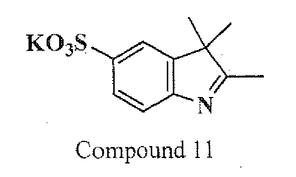

A mixture of phenylhydrazine-*p*-sulphonic acid (compound 7) (11.3 g; 0.06 mole), 3-methyl-2-butanone (6.2 g; 7.72 mL; 0.072 mole), and glacial acetic acid (34 mL) was refluxed for 3 h, then, allowed to hold for 12 h at 0°C. The precipitate formed was collected by filtration, washed with acetone, and dried in a vacuum desiccator over P₂O₅. 5-Sulfo-2,3,3-trimethylindolenyne was obtained with yield 5.1 g (71.3%).

¹H- NMR (D₂O), δ (ppm): 1.28 (6H, s, C(CH₃)₂); 7.47-7.79 (3H, m, ArH)

While heating, 5-sulfo-2,3,3-trimethylindolenyne (4.77 g; 0.02 mole) was dissolved in methanol (25 mL). To the prepared solution there was added dropwise a solution of potassium hydroxide (1.35 g; 0.024 mole) in methanol (16 mL). Then, isopropyl alcohol (50 mL) was added. The residue was filtered off, dried in vacuum desiccator over P₂O₅. The potassium salt of 5-sulfo-2,3,3-trimethylindolenyne (compound 11) was prepared as lustrous plates of light beige colour with yield of 4.7 g (85%). λₘₐₓ 260 nm. Mass-spectrum (MALDI) (C₁₁H₁₂NO₃S⁻): found m/z 239.0, calculated m/z 238.28.

¹H- NMR (D₂O), δ (ppm): 1.19 (6H, s, C(CH₃)₂); 2.19 (6H; s, 2-CH₃); 7:41-7.7 (3H, m, ArH)

### Example 12. Preparation of 2,3,3,5-tetramethylindolenyne (compound 12)

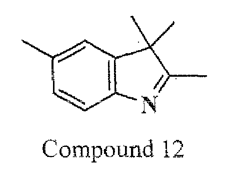

A mixture of 3-methyl-2-butanone (7 mL; 5.6 g; 0.065 mole), 4-tolylhydrazine hydrochloride (compound 9) (11.0 g; 0.069 mole), anhydrous potassium acetate (13.9 g; 0.14 mole), and glacial acetic acid (70 mL) was stirred at room temperature for 30 min, and then refluxed for 1 h. The solvent was removed, and the oily residue was dissolved in chloroform. The chloroform solution was washed with water, saturated solution of sodium chloride, and then dried over MgSO₄. The solvent was removed and the residue was fractionated under vacuum and a nitrogen flow with the use of water-jet pump (Bp= 115°C/10 mm Hg). 2,3,3,5-Tetramethylindolenyne (compound 12) was obtained with yield of 9.53 g (79.5%). λₘₐₓ 263 nm. Mass-spectrum (MALDI) (C₁₂H₁₅N): found m/z 173.9, calculated m/z 173.25.

¹H-NMR (DMSO-d6), δ (ppm): 1.21 (6H, s, C(CH₃)₂), 2.17 (3H, s, 2-CH₃), 2.32 (3H, s, 5-CH₃), 7.05 (1H, d, 6-ArH), 7.19 (1H, s, 4-ArH), 7.28 (1H, d, 7-ArH)

### Example 13, Preparation of 2.4-xylylhydrazine hydrochloride (compound 13)

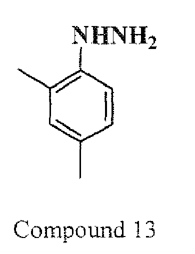

To a mixture of 2.4-dimethylaniline (35.4 mL; 36.3 g; 0.3 mole) and concentrated hydrochloric acid (220 mL) chilled to 0°C, the solution of sodium nitrite (20.6 g; 0.3 mole) in water (60 mL) was added dropwise at such a rate to maintain reaction temperature not higher than 5°C. Then, the solution prepared was filtered off and added to a solution of stannous chloride (124 g; 0.65 mole) in concentrated hydrochloric acid (124 mL) chilled to 5°C, while vigorous stirring. The precipitate formed was separated by filtration, slurried in water (500 mL), and alkalized to pH 12 with solution of sodium hydroxide (440%). The reaction mixture was extracted with diethyl ether (3*200 mL). Ethereal extracts were dried over MgSO₄. Then, a stream of dried hydrogen chloride was bubbled via the ethereal solution of *p*-tolyl hydrazine. The precipitate formed was filtered, washed with ether, and dried in vacuum desiccator over P₂O₅. 2.4-Xylylhydrazine hydrochloride (compound 13) was obtained with yield of 18.5 g (35.8%). λₘₐₓ 281 nm. Mass-spectrum (MALDI) (C₈H₁₂N₂): found m/z 137.2, calculated m/z 136.19.

¹H- NMR (D₂O), δ (ppm): 2.03 (3H, s, *p*-CH₃), 2.08 (3H, s, 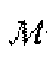 -CH₃), 6.72 (1H, s, ArH), 6.93 (2H, d, ArH)

### Example 14. Preparation of 2,3,3,5.7-pentamethylindolenyne (compound 14).

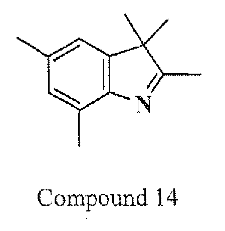

A mixture of 3-methyl-2-butanone (7 mL; 5.6 g; 0.065 mole), 2.4-xylyl hydrazine hydrochloride (compound 13) (11.9 g; 0.069 mole), anhydrous potassium acetate (13.9 g; 0.14 mole), and glacial acetic acid (70 mL) was stirred at room temperature 30 min, and then, refluxed for 1 h. The solvent was removed and oily residue was dissolved in ether. The ethereal solution was washed with water, saturated solution of sodium chloride, and then dried over MgSO₄. The solvent was removed and the residue was fractionated under vacuum water-jet pump in a nitrogen flow (Bp= 132°C/20 mm Hg). 2,3,3,5.7-Pentamethylindolenyne (compound 14) was obtained with yield of 8.3 g (63.7%). λₘₐₓ 265 nm. Mass-spectrum (MALDI) (C₁₃H₁₇N): found m/z 188.5, calculated m/z 187.28.

¹H-NMR (DMSO-d6), δ (ppm): 1.43 (6H, s, C(CH₃)₂), 2.35 (3H, s, 2-CH₃), 2.48 (3H, s, 5-CH₃), 2.66 (3H, s, 7-CH₃), 7.12 (1H, s, ArH), 7.33 (1H, s, ArH)

### Example 15. Preparation of α-chloro-β-(p-nitrophenyl)propionic nitrile (compound 15)

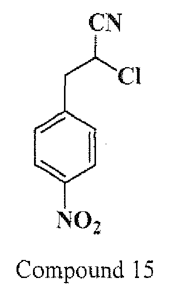

*p*-Nitroaniline (11.5 g; 0.083 mole), water (60 mL), and concentrated hydrochloric acid (62 mL) were charged in a flask and heated until complete dissolution of p-nitroaniline hydrochloride produced. Then the reaction mixture was chilled to start crystallization, finely chopped ice (50 g) was added and the solution of sodium nitrite (5.9 g; 0.083 mole) in water (13.8 mL) was added dropwise to the slurry obtained, while intensive stirring. The reaction progress was monitored using a starch iodide paper. To the solution obtained, there were added under stirring a solution of acrylonitrile (4.43 g; 0.083 mole) in acetone (37.5 mL) and crystalline CuSO₄*5H₂O (2.5 g; 0.01 mole), and the reaction allowed to hold for a period of day at room temperature. Then, the precipitate formed was filtered off and washed with water. The residue was transferred in a flask with water (250 mL) and impurities were evaporated by steam distillation. After collection of condensate (500 mL), the distillation was ceased. The distillation residue was cooled with ice and aqueous layer was removed by inverse filtering. The solid residue was dissolved in methanol (100 mL), treated with activated carbon (1 g), the hot solution was filtered and added water (300 mL). The mixture was allowed to hold overnight at +5°C. The precipitate formed was filtered off and dried at 80°C. α-Chloro-β-(*p*-nitrophenyl)propionic nitrile (compound 15) was obtained with yield of 14.3 g (82%). Mp 110-111°C (from methanol).

¹H-NMR (DMSO-d6), δ (ppm): 3.55 (2H, m, CH₂CH(Cl)CN), 5.64 (1H, t, J=7.0, CH₂CH(Cl)CN), 7.65-8.26 (4H, d, J=9.0. ArH)

### Example 16. Preparation of p-nitrocinnamic nitrile (compound 16)

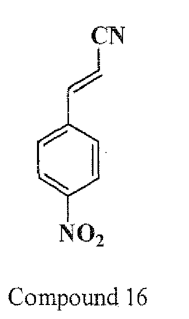

A mixture of α-chloro-β-(*p*-nitrophenyl)propionic nitrile (compound 15) (10.5 g; 0.05 mole), sodium acetate (15.0 g; 0.18 mole), water (40 mL), and ethyl alcohol (90 mL) was refluxed for a 4 h. Then, water (150 mL) was added to the reaction mixture that allowed to hold at +5°C to complete crystallization. The precipitate formed was collected by filtration, dissolved in ethanol (200 mL), treated with activated carbon (1.5 g), and the hot solution was filtered. Water (400 mL) was added to the filtrate that allowed to hold overnight at +5°C. The precipitate formed was collected by filtration and dried. The yield of crude compound 16 was 8.2 g. After additional recrystallization from alcohol (200 mL), *p*-nitrocinnamic nitrile (compound 16) was obtained as yellow needles with yield of 4.1 g (47%). Mp 201°C (from ethanol).

¹H-NMR (DMSO-d6), δ (ppm): 6.72 (1H, d, J=16.5, CHCHCN), 7.81 (1H, d, J=16.5, CHCHCN), 7.79-8.29 (4H, d, J=9.0, ArH)

### Example 17. Preparation of p-nitrocinnamic acid (compound 17)

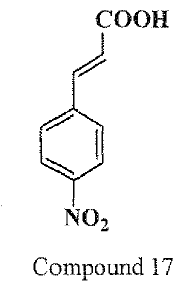

Method A. A mixture of *p*-nitrocinnamic nitrile (compound 16) (17.4 g; 0.1 mole) and concentrated phosphoric acid (350 mL) was heated at 150°C for 2 h. After cooling to room temperature, the mixture was transferred into ice water (4 L). The precipitate formed was filtered off and dried. The yield of crude product 17 was 18.3 g. The crude product was dissolved in 0.12M sodium hydroxide (1 L) and treated with activated carbon (3.5 g). The solution formed was filtered off and concentrated hydrochloric acid was added thereto dropwise to pH∼2. The precipitate formed was collected by filtration, washed with water, and dried at 90°C. *p-*Nitrocinnamic acid (compound 17) was obtained as yellow needles with yield of 17.7 g (92%).

Mp 283.5°C.

Method B. On performing reaction using crude *p*-nitrocinnamic nitrile (compound 16) by the same procedure, *p*-nitrocinnamic acid (compound 17) was obtained with yield of 11.2 g (58%).

¹H-NMR (DMSO-d6), δ (ppm): 6.74 (1H, d, J=16.5, CHCHCOOH), 7.69 (1H, d, J=16.5, CHCHCOOH), 7.96-8.25 (4H, d, J=9.0, ArH)

### Example 18. Preparation of 3-(p-aminophenyl)propionic acid, hydrochloride (compound 18)

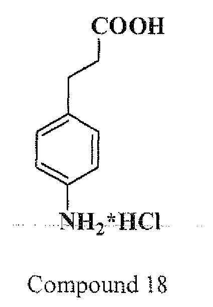

A flask was charged with catalyst - 5% Pd/C (400 mg), acetic acid (80 mL), and *p*-nitrocinnamic acid (compound 17) (3.86 g; 20 mmole). While vigorous stirring, the mixture was hydrogenated with hydrogen flow under atmospheric pressure, at room temperature, for 2.5 h. The reaction mixture was filtered off and then evaporated. Oily residue dissolved in mixture of 5M HCl (7 mL) and water (30 mL) was treated with activated carbon on heating. Being filtered off carbon, the solution was evaporated, and the residue was dried in vacuum desiccator over P₂O₅ and KOH to yield 3-(*p*-aminophenyl)propionic acid hydrochloride (compound 18), 3.87 g (96%).

¹H-NMR (DMSO-d6), δ (ppm): 2.53 (2H, t, J=7.5, CH₂CH₂COOH), 2.83 (2H, t, J=7.5, CH₂CH₂COOH), 7.28-7.35 (4H, d, J=8.5, ArH), 10.31 (s, 2H, NH₂)

### Example 19. Preparation of p-carboxyethylphenyl hydrazine (compound 19)

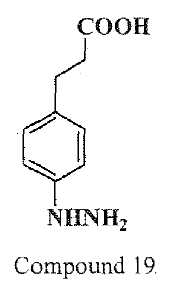

A three-necked flask equipped with mechanical stirrer and thermometer was charged with 3-(*p-*aminophenyl)propionic acid hydrochloride (compound 18) (2.01 g; 10 mmole), concentrated hydrochloric acid (35 mL), and while cooling, the sodium nitrite (0.69 g, 10 mmole) solution in water (0.9 mL) was added dropwise, under layer of liquid, at such a rate to maintain reaction temperature not higher than -5°C. Then, temperature increased to 0°C in 30 min. Again, the mixture was cooled down to -5°C and the solution of SnCl₂*2H₂O (10 g; 44 mmole) in concentrated hydrochloric acid (15 mL) chilled to -18°C was added at such a rate to maintain reaction temperature not higher than -5°C. The mixture was additionally stirred at -5°C for 30 and allowed to hold at +5°C, for a period of day. The precipitated crystals were collected by filtration and washed with concentrated hydrochloric acid (3*2 mL), chilled to -18°C. p-Carboxyethylphenyl hydrazine hydrochloride (compound 19) was obtained with yield of 1.62 g (75%).

¹H-NMR (DMSO-d6), δ (ppm): 2.47 (2H, t, J= 7.5, CH₂CH₂COOH), 2.73 (2H, t, J= 7.5, CH₂CH₂COOH), 6.91-7.13 (4H, d, J= 8.5, ArH), 10.27 (3H, s, NHNH₂)

### Example 20. Preparation of 5-carboxyethyl-2,3,3-trimethylindolenyne (compound 20)

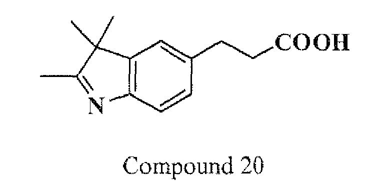

A glass vial was charged with p-carboxyethylphenyl hydrazine hydrochloride (compound 19) (216 mg; 1 mmole), anhydrous potassium acetate (196 mg; 2.0 mmole), 3-methyl-2-butanone (147 µL; 118.1 mg; 1.37 mmole), and acetic acid (1 mL) and heated at 118°C for 1 h. Then, solvents were removed; the residue was dissolved in chloroform, washed with saturated solution of sodium chloride and dried over sodium sulphate. After chloroform removal, the oily residue was subjected to recrystallization from ethyl acetate: hexane mixture, 1:10. The precipitate was collected by filtration and dried in vacuum desiccator over P₂O₅ and KOH. 5-Carboxyethyl-2,3,3-trimethylindolenyne (compound 20) was obtained with yield of 118 mg (55%). λₘₐₓ 262 nm. Mass-spectrum (MALDI) (C₁₄H₁₇NO₂): found m/z 232.3, calculated m/z 231.29.

¹H- NMR (CDCl₃), δ (ppm): 1.27 (6H, s, C(CH₃)₂), 2.27 (3H, s, CCH₃), 2.68 (2H, t, J=8, CH₂CH₂COOH), 3.00 (2H, t, J=8, CH₂CH₂COOH), 7.12-7.46 (3H, d, J=8.5, ArH)

### Example 21. Preparation of 2,3-dimethyl-3-(4-carboxybutyl)-N-ethylindolenyneum iodide (compound 21)

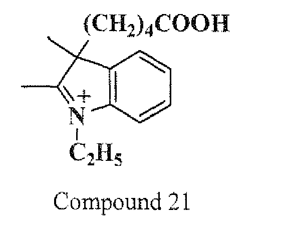

A glass vial was charged with 2,3-dimethyl-3-(4-carboxybutyl)indolenyne (compound 6) (49.1 mg; 0.2 mmole), ethyl iodide (1 mL), and acetonitrile (0.4 mL) and heated at 70°C for 20 h. After that heating, the solvent was removed under vacuum; the residue was washed with diethyl ether and dried in vacuum desiccator over P₂O₅. 2,3-Dimethyl-3-(4-carboxybutyl)-*N-*ethylindolenyneum iodide (compound 21) was obtained with yield of 76.2 mg (95%). λₘₐₓ 279 nm. Mass-spectrum (MALDI) (C₁₇H₂₄NO₂⁺): found m/z 274.4, calculated m/z 274.38.

¹H- NMR (CDCl₃), δ (ppm): 0.52-0.78 (2H, m, CH₂CH₂CH₂CH₂COOH); 1.25 (3H, s, 3-CH₃); 1.38-1.49 (2H, m, CH₂CH₂CH₂CH₂COOH); 1.62 (3H, t, CH₂CH₃), 1.70-1.81 (2H, m, CH₂CH₂CH₂CH₂COOH); 2.18 (2H, m, CH₂CH₂CH₂CH₂COOH); 2.28 (3H, s, 2-CH₃); 4.73 (2H, m, CH₂CH₃), 7.5-7.68 (4H, m, ArH)

### Example 22. Preparation of 2,3-dimethyl-3-(4-carboxybutyl)-N-(4-sulfobutyl)indolenyne (compound 22)

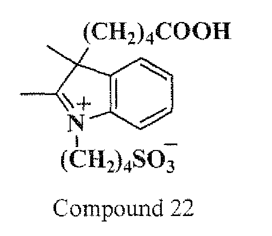

A glass vial was charged with 2,3-dimethyl-3-(4-carboxybutyl)indolenyne (compound 6) (637.8 mg; 2.6 mmole), 1,4-butansultone (708.1 mg; 526.6 µL; 5.2 mmole), and 1,2-dichlorobenzene (4.2 mL) and heated at 118°C for 25 h. After solvent decanting, the oily residue was triturated with diethyl ether. The precipitate formed was collected by filtration, washed with diethyl ether and dried in vacuum desiccator over P₂O₅. 2,3-Dimethyl-3-(4-carboxybutyl)-*N*-(4-sulfobutyl)-indolenyne (compound 22) was obtained as a maroon powder with yield of 0.98 g (98,9%). λₘₐₓ 280 nm. Mass-spectrum (MALDI) (C₁₉H₂₇NO₅S): found m/z 382.0, calculated m/z 381.49.

¹H-NMR (D₂O), δ (ppm): 0.38-0.68 (2H, m, CH₂CH₂CH₂CH₂COOH); 1.23-1.35 (2H, m, CH₂CH₂CH₂CH₂COOH); 1.43 (3H, s, 3-CH₃); 1.71-1.81 (2H, m, CH₂CH₂CH₂CH₂SO₃); 1.92-2.2 (6H, m, CH₂CH₂CH₂CH₂COOH, CH₂CH₂CH₂CH₂SO₃); 2.84 (2H, t, J=7.5, CH₂CH₂CH₂CH₂SO₃); 4.41 (2H, t, J=7.5, CH₂CH₂CH₂CH₂SO₃); 7.18-7.7 (4H, m, ArH)

### Example 23. Preparation of 3-(4-carboxybutyl)-N-(4-sulfobutyl)-2,3,5-trimethylindolenyne (compound 23)

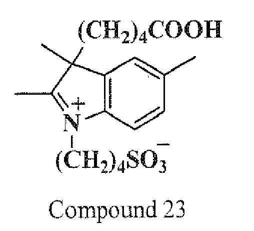

A glass vial was charged with 3-(4-carboxybutyl)-2,3,5-trimethylindolenyne (compound 10) (400 mg; 1.55 mmole), 1,4-butansultone (422 mg; 315 µL; 3.1 mole), and 1,2-dichlorobenzene (2.6 mL) and heated at 118°C for 50 hours. After solvent decanting, the oily residue was triturated with diethyl ether. The precipitate formed was collected by filtration, washed with diethyl ether and dried in vacuum desiccator over P₂O₅. 3-(4-Carboxybutyl)-*N*-(4-sulfobutyl)-2,3,5-trimethylindolenyne (compound 23) was obtained as a maroon powder with yield of 0.58 g (95%). λₘₐₓ 286 nm. Mass-spectrum (MALDI) (C₂₀H₂₉NO₅S): found m/z 396.7, calculated m/z 395.51.

¹H-NMR (D₂O), δ (ppm): 0.41-0.67 (2H, m, CH₂CH₂CH₂CH₂COOH); 1.25 (2H, m, CH₂CH₂CH₂CH₂COOH); 1.39 (3H, s, 3-CH₃); 1.75 (4H, m, CH₂CH₂CH₂CH₂SO₃); 1.94 (2H, m, CH₂CH₂CH₂CH₂COOH); 2.05 (6H, m, CH₂CH₂CH₂CH₂COOH, CH₂CH₂CH₂CH₂SO₃); 2.32 (3H, s, 5-CH₃); 4.37 (2H, m, CH₂CH₂CH₂CH₂SO₃); 7.31-7.56 (3H, m, ArH)

### Example 24. Preparation of 2,3-dimethyl-3-(4-carboxybutyl)-5-sulfo-N-ethylindolenyne (compound 24)

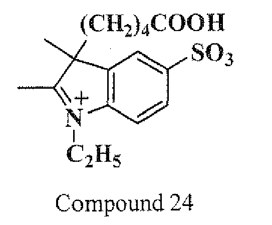

A glass vial was charged with 2,3-dimethyl-3-(4-carboxybutyl)-5-sulfoindolenyne, potassium salt (compound 8) (0.5 g; 1.38 mmole), ethyl iodide (1.08 g; 550 µL; 6.9 mmole), and 1,2-dichlorobenzene (25 µL). This slurry was heated at 72°C for 50 h. After heating ceased, the solvent was decanted; the precipitate was washed with acetone, collected by filtration, and dried in vacuum desiccator over P₂O₅. 2,3-Dimethyl-3-(4-carboxybutyl)-5-sulfo-*N*-ethylindolenyne (compound 24) was obtained with yield of 0.47 g (96%). λₘₐₓ 266 nm. Mass-spectrum (MALDI) (C₁₇H₂₃NO₅S): found m/z 354.2 , calculated m/z 353.43.

¹H-NMR (D₂O), δ (ppm): 0.58-0.72 (2H, m, CH₂CH₂CH₂CH₂COOH); 1.49 (t, 3H, CH₂CH₃), 1.56 (3H, s, 3-CH₃); 1.61 (2H, m, CH₂CH₂CH₂CH₂COOH); 2.14 (4H, m, CH₂CH₂CH₂CH₂COOH); 4.5 ( 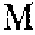 , 2H, CH₂CH₃); 7.9 (1H, d, ArH), 8.06 (2H, m, ArH)

### Example 25. Preparation of 2,3-dimethyl-3-(4-carboxybutyl)-N-(4-sulfobutyl)-5-sulfoindolenyne, potassium salt (compound 25)

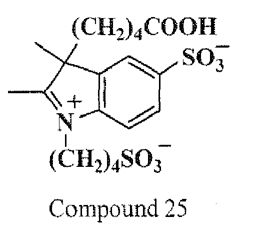

A glass vial was charged with 2,3-dimethyl-3-(4-carboxybutyl)-5-sulfoindolenyne, potassium salt (compound 8) (0.6 g; 1.65 mmole) and 1,4-butansultone (1.0 mL), The slurry was heated at 118°C for 50 h. After cooling down to room temperature, the reaction mixture was triturated with diethyl ether. The precipitate formed was collected by filtration, washed with diethyl ether, and dried in vacuum desiccator over P₂O₅. 2,3-Dimethyl-3-(4-carboxybutyl)-*N*-(4-sulfobutyl)-5-sulfoindolenyne, potassium salt (compound 25) was obtained with yield of 0.8 g (97%). λₘₐₓ 268 nm. Mass-spectrum (MALDI) (C₁₉H₂₆NO₈S₂⁻): found m/z 461.1, calculated m/z 460.54.

¹H-NMR (D₂O), δ (ppm): 0.64-0.77 (2H, m, CH₂CH₂CH₂CH₂COOH); 1.39 (2H, m, CH₂CH₂CH₂CH₂COOH); 1.58 (3H, s, 3-CH₃); 1.68-1.75 (2H, m, CH₂CH₂CH₂CH₂SO₃); 1.95-2.06-2.31 (6H, m, CH₂CH₂CH₂CH₂COOH, CH₂CH₂CH₂CH₂SO₃); 2.89 (2H, t, J=7.5, CH₂CH₂CH₂CH₂SO₃); 4.51 (2H, t, J=7.5, CH₂CH₂CH₂CH₂SO₃); 7.7 (1H, d, ArH), 8.04 (1H, s, ArH).

### Example 26. Preparation of N-(4-sulfobutyl)-2,3,3-trimethylindolenyne (compound 26)

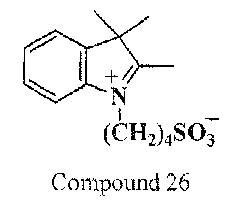

A glass vial charged with 2,3,3-trimethylindolenyne (414 mg; 417.3 µL; 2.6 mmole), 1,4-butansultone (708.1 mg; 526.6 µL; 5.2 mmole), and 1,2-dichlorobenzene (4.2 mL) was heated at 118°C for 25 h. After solvent decanting, the residue was triturated with diethyl ether. The precipitate formed was collected by filtration, washed with diethyl ether, and dried in vacuum desiccator over P₂O₅, *N*-(4-Sulfobutyl)-2,3,3-trimethylindolenyne (compound 26) was obtained as a maroon powder with yield of 0.75 g (97.4%). λₘₐₓ 278 nm. Mass-spectrum (MALDI) (C₁₅H₂₁NO₃S): found m/z 296.0, calculated m/z 295.40.

¹H-NMR (D₂O), δ (ppm): 1.44 (6H, s, C(CH₃)₂); 1.77 (2H, m, CH₂CH₂CH₂CH₂SO₃); 2.02 (2H, m, CH₂CH₂CH₂CH₂SO₃); 2.84 (2H, t, J=7.5, CH₂CH₂CH₂CH₂SO₃); 4.39 (2H, t, J=7.5, CH₂CH₂CH₂CH₂SO₃); 7.49-7.68 (4H, m, ArH)

### Example 27. Preparation of 2,3,3-trimethyl-N-ethylindolenyneum iodide (compound 27)

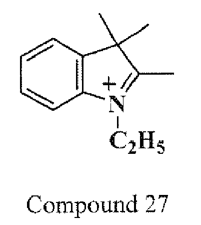

A mixture of 2,3,3-trimethylindolenynea (1.35 g; 1.36 mL; 8.48 mmole), ethyl iodide (5.5 mL), and acetonitrile (5 mL) was refluxed under stirring in a nitrogen flow for 18 h. After cooling down the reaction mixture to room temperature anhydrous diethyl ether (30 mL). The crystals formed were collected by filtration and subjected to recrystallization from isopropanol (5 mL). 2,3,3-Trimethyl-*N*-ethylindolenyneum iodide (compound 27) was obtained with yield of 2.46 g (91.9%). λₘₐₓ 278 nm. Mass-spectrum (MALDI) (C₁₃H₁₈N⁺): found m/z 189.3, calculated m/z 188.29.

¹H-NMR (CDCl₃), δ (ppm): 1.64 (9H, m, CH₂CH₃, C(CH₃)₂), 3.14 (3H, s, 2-CH₃), 4.75 (2H, m, CH₂CH₃), 7.56-7.72 (4H, m, ArH)

### Example 28. Preparation of 5-sulfo-N-(4-sulfobutyl)-2,3,3-trimethylindolenyne, potassium salt (compound 28)

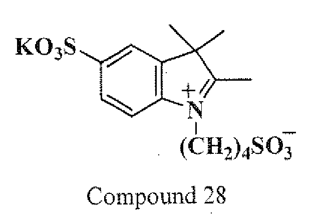

A glass vial was charged with 5-sulfo-2,3,3-trimethylindolenyne, potassium salt (compound 11) (0.6 g; 2.16 mmole), 1,4-butansultone (882.4 mg; 660 µL; 6.48 mmole), and 1,2-dichlorobenzene (3.5 mL). The slurry was heated at 118°C for 30 h. After cooling down the reaction mixture to room temperature, acetone (10 mL) was added thereto. The precipitate was collected by filtration, washed with diethyl ether, and dried in vacuum desiccator over P₂O₅. 5-Sulfo-*N-*(4-sulfobutyl)-2,3,3-trimethylindolenyne, potassium salt (compound 28) was obtained as a bright pink powder with yield of 0.78 g (87.6%). λₘₐₓ 274 nm. Mass-spectrum (MALDI) (C₁₅H₂₀NO₆S₂⁻): found m/z 375.6, calculated m/z 374.45.

¹H-NMR (D₂O), δ (ppm): 1.43 (6H, s, C(CH₃)₂); 1.74 (2H, m, CH₂CH₂CH₂CH₂SO₃); 1.95 (2H, m, CH₂CH₂CH₂CH₂SO₃); 2.81 (2H, t, J=7.5, CH₂CH₂CH₂CH₂SO₃); 4.38 (2H, t, J=7.5, CH₂CH₂CH₂CH₂SO₃); 7.49-7.68 (3H, m, ArH).

### Example 29. Preparation of 2,3,3-trimethyl-5-sulfo-N-ethylindolenyne (compound 29)

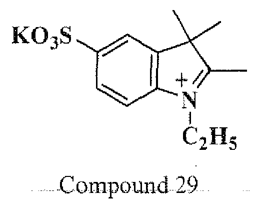

A slurry of 5-sulfo-2,3,3-trimethylindolenyne, potassium salt (compound 11) (5.54 g; 20 mmole), ethyl iodide (20 mL), and 1,2-dichlorobenzenea (5 mL) was heated for 50 h, under stirring. After heating ceased, the solvent was decanted and superabundant acetone was added. The crystals formed were collected by filtration, washed with acetone, to remove potassium iodide, and dried in vacuum desiccator over P₂O₅. 2,3,3-Trimethyl-5-sulfo-*N*-ethylindolenyne (compound 29) was obtained with yield of 5.31 g (99%). λₘₐₓ 228 nm. Mass-spectrum (MALDI) (C₁₃H₁₇NO₃S): found m/z 268.4, calculated m/z 267.35.

¹H-NMR (D₂O), δ (ppm): 1.43 (9H, m, CH₂CH₃, C(CH₃)₂), 4.36 ( 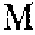 , 2H, CH₂CH₃), 7.63 ( 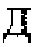 , 1H, 7-ArH), 7.75 ( 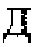 , 1H, 6-ArH), 7.97 (s, 1H, 4-ArH)

### Example 30. Preparation of N-(4-sulfobutyl)-2,3,3,5-tetramethylindolenyne (compound 30)

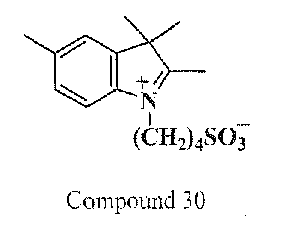

A mixture of 2,3,3,5-tetramethylindolenyne (compound 12) (1.3 g; 7.5 mmole), 1,4-butansultonea (3.4 g; 4.5 mL; 25 mmole), and 1,2-dichlorobenzenea (10.0 mL) was heated at 120°C for 8 h, under stirring. After heating ceased, the reaction mixture was cooled to room temperature and acetone (40 mL) was added thereto. The crystals formed were collected by filtration and dried in vacuum desiccator over P₂O₅. *N*-Sulfobutyl-2,3,3,5-tetramethylindolenyne (compound 30) was obtained with yield of 2.26 g (89.9%). λₘₐₓ 288 nm. Mass-spectrum (MALDI) (C₁₄H₂₃NO₃S): found m/z 310.8, calculated m/z 309.42.

¹H-NMR (D₂O), δ (ppm): 1.39 (6H, s, C(CH₃)₂), 1.73 (2H, m, CH₂CH₂CH₂CH₂SO₃), 2.07 (2H, m, CH₂CH₂CH₂CH₂SO₃), 2.3 (s, 3H, 5-CH₃), 2.81 (2H, t, J=7.5, CH₂CH₂CH₂CH₂SO₃), 4.46 (2H, t, J=7.5, CH₂CH₂CH₂CH₂SO₃), 7.28-7.52 (3H, m, ArH)

### Example 31. Preparation of 2,3,3,5-tetramethyl-N-ethylindolenyneum iodide (compound 31)

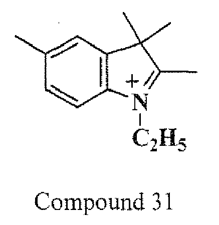

A mixture of 2,3,3,5-tetramethylindolenyne (compound 12) (1.47 g; 8.48 mmole) and ethyl iodide (5.5 mL) was refluxed in a nitrogen flow for 15 h, under stirring. After heating ceased, the reaction mixture was cooled to room temperature and anhydrous diethyl ether (30 mL) was added thereto. The crystals formed were collected by filtration and subjected to recrystallization from boiling isopropanol (3 mL). 2,3,3,5-Tetramethyl-*N*-ethylindolenyneum iodide (compound 31) was obtained with yield of 1.14 g (40.7%). λₘₐₓ 283 nm. Mass-spectrum (MALDI) (C₁₄H₂₀N⁺): found m/z 201.3, calculated m/z 202.32.

¹H-NMR (CDCl₃), δ (ppm): 1.59 (3H, t, CH₂CH₃), 1.61 (6H, s, C(CH₃)₂), 2.45 (3H, s, 2-CH₃), 3.09 (3H, s, 5-CH₃), 4.70 (2H, m, CH₂CH₃), 7.33 (2H, d, 4.6-ArH), 7.55 (1H, d, 7-ArH)

### Example 32. Preparation of 2,3,3,5,7-pentamethyl-N-(4-sulfobutyl)indolenyne (compound 32)

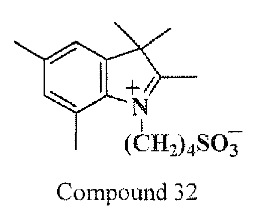

A glass vial was charged with 2,3,3,5,7-pentamethylindolenyne (compound 14) (187 mg; 1 mmole), 1,4-butansultone (272 mg; 200 µL, 2 mmole), and 1,2-dichlorobenzene (1.6 mL) and heated at 118°C for 30 h. Diethyl ether was added into the reaction mixture. The precipitate formed was collected by filtration and dried in vacuum desiccator over P₂O₅. 2,3,3,5,7-Pentamethyl-*N*-(4-sulfobutyl)indolenyne (compound 32) was obtained as a pale brown powder with yield of 0.25 g (77%). λₘₐₓ 282 nm. Mass-spectrum (MALDI) (C₁₇H₂₅NO₃S): found m/z 323.9, calculated m/z 323.45.

¹H-NMR (D₂O), δ (ppm): 1.41 (6H, s, C(CH₃)₂); 1.74 (2H, m, CH₂CH₂CH₂CH₂SO₃); 2.03 (2H, m, CH₂CH₂CH₂CH₂SO₃); 2.38 (3H, s, 5-CH₃), 2.63 (3H, s, 7-CH₃), 2.81 (2H, t, J=7.5, CH₂CH₂CH₂CH₂SO₃); 4.38 (2H, t, J=7.5, CH₂CH₂CH₂CH₂SO₃); 7.43-7.61 (2H, m, ArH)

### Example 33. Preparation of 2,3,3,5,7-pentamethyl-N-ethylindolenyneum iodide (compound 33)

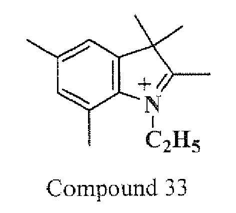

A mixture of 2,3,3,5,7-pentamethylindolenyne (compound 14) (1.59 g; 8.48 mmole), ethyl iodide (5.5 mL), and acetonitrile (5 mL) was refluxed in a nitrogen flow, for 15 h, under stirring. After heating ceased, the reaction mixture was cooled to room temperature and anhydrous diethyl ether (30 mL) was added thereto. The crystals formed were collected by filtration and subjected to recrystallization from isopropanol (4 mL). 2,3,3,5,7-Pentamethyl-*N-*ethylindolenyneum iodide (compound 33) was obtained with yield of 0.8 g (27.6%). λₘₐₓ 291 nm. Mass-spectrum (MALDI) (C₁₅H₂₂N⁺): found m/z 216.7, calculated m/z 216.34.

¹H- NMR (CDCl₃), δ (ppm): 1.57 (9H, m, C(CH₃)₂, CH₂CH₃), 2.39 (3H, s, 2-CH₃), 2.69 (3H, s, 5-CH₃), 3.06 (3H, s, 7-CH₃), 4.83 (2H, m, CH₂CH₃), 7.07 (s, 2H, ArH)

### Example 34. Preparation of 5-carboxyethyl-N-sulfobutyl-2,3,3-trimethylindolenyne (compound 34)

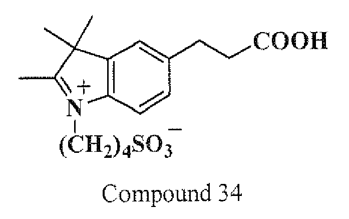

A glass vial charged with 5-carboxyethyl-2,3,3-trimethylindolenyne (compound 20) (231 mg; 1 mmole), 1,4-butansultone (162 µL; 216 mg; 1.6 mmole), and butyronitrile (2.2 mL) was heated at 118°C for 22 h. After cooling down to room temperature, the reaction mixture was triturated with anhydrous diethyl ether. The precipitate formed was collected by filtration and dried in vacuum desiccator over P₂O₅ and KOH. 5-Carboxyethyl-*N*-sulfobutyl-2,3,3-trimethylindolenyne (compound 34) was obtained with yield of 300 mg (86%). λₘₐₓ 284 nm. Mass-spectrum (MALDI) (C₁₈H₂₅NO₅S): found m/z 368.2, calculated m/z 367.46.

¹H-NMR (DMSO-d6), δ (ppm): 1.51 (s, 6H, C(CH₃)₂), 1.73 (t, 2H, CH₂CH₂CH₂CH₂SO₃), 1.96 (t, 2H, CH₂CH₂CH₂CH₂SO₃), 2.61 (t, J=7.5, 2H, CH₂CH₂COOH), 2.81 (s, 3H, CH₃), 2.95 (t, J=7.5, 2H, CH₂CH₂COOH), 4.46 (M, 2H, NCH₂), 7.47-7.93 (M, 3H, ArH).

### Example 35. Preparation of 5-carboxyethyl-2,3,3-trimethyl-N-ethylindolenyneum iodide (compound 35)

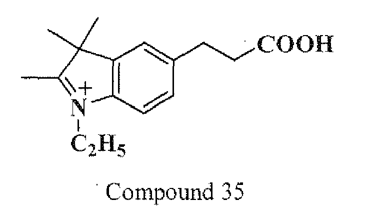

A glass vial charged with 5-carboxyethyl-2,3,3-trimethylindolenyne (compound 20) (231 mg; 1 mmole), ethyl iodide (500 µL; 975 mg; 5.5 mmole), and acetonitrile (2.2 mL) was heated at 118°Cfor 15 h. Then mixture was cooled to room temperature, added anhydrous diethyl ether (2 mL), and allowed to hold at -18°C for a period of day. The crystals formed were collected by filtration and dried in vacuum desiccator over P₂O₅ and KOH. 5-Carboxyethyl-2,3,3-trimethyl*-N-*ethylindolenyneum iodide (compound 35) was obtained with yield of 377 mg (97%). λₘₐₓ 284 nm. Mass-spectrum (MALDI) (C₁₆H₂₁NO₂): found m/z 259.8, calculated m/z 259.34.

¹H-NMR (DMSO-d6), δ (ppm): 1.44 (t, J=7.0, 3H, CH₂CH₃), 1.52 (s, 6H, C(CH₃)₂), 2.62 (t, J=7.5, 2H, CH₂CH₂COOH), 2.80 (s, 3H, CH₃), 2.96 (t, J=7.5, 2H, CH₂CH₂COOH), 4.47 (q, J=7.0, 2H, CH₂CH₃), 7.47-7.86 ( 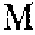 , 3H, ArH)

### Example 36. Preparation of malonic dialdehyde bis-(diethylacetal) (compound 36)

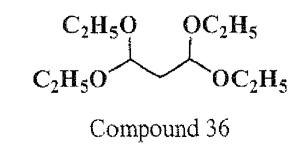

A three-necked flask equipped with backflow condenser, mechanical stirrer, thermometer, and dropping funnel was charged with ethyl orthoformiate (50.0 mL; 44.55 g; 0.3 mole) and boron trifluoride-etherate (0.5 mL). The reaction mixture was heated to 30°C and vinylethyl ether (27 mL; 20.3 g; 0.28 mole) was added dropwise at such a rate to maintain the reaction temperature not higher than 36-38°C. The mixture was additionally stirred at room temperature for 1 h. Then, anhydrous potassium carbonate (3 g; 0.02 mole) was added to the reaction mixture and stirred at room temperature for 2 hours more. The precipitate formed was filtered off and the filtrate was fractionated under vacuum in a nitrogen flow. Fraction having Bp= 101-102°C/12 mm Hg was drawn to obtain malonic dialdehyde bis-(diethylacetal) (compound 36) with yield of 43 g (69.2%). Mass-spectrum (MALDI) (C₁₁H₂₄O₄): found m/z 221.5, calculated m/z 220.31.

¹H- NMR (CDCl₃), δ (ppm): 1.20 (12H, t, OCH₂CH₃), 1.96 (2H, t, CH₂), 3.46-3.71 (8H, m, OCH₂CH₃), 4.62 (2H, t, CH)

### Example 37. Preparation of malonic dialdehyde dianil, hydrochloride (compound 37)

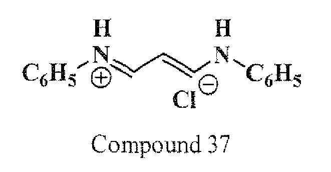

A mixture of aniline hydrochloride (4.72 g; 36.4 mmole) and methanol (6 mL) was heated to obtain a homogeneous solution and malonic dialdehyde bis-(diethylacetal) (compound 36) (4.0 g, 18.2 mmole) was added dropwise. This slurry was then refluxed for 10 min, cooled down to room temperature, water (5500 mL) was added and allowed to hold at +5°C for 24 h. The precipitate formed was collected by filtration, washed with ice water, and dried in vacuum desiccator over P₂O₅ and KOH. Malonic dialdehyde dianil, hydrochloride (compound 37)was obtained as yellow-orange fine crystals with yield of 4.2 g (89%). Mass-spectrum (MALDI) (C₁₅H₁₅N₂⁺): found m/z 224.5, calculated m/z 223.29

¹H-NMR (DMSO-d6), δ (ppm): 6.48 (2H, t, β-CH), 7.4 (10H, s, ArH), 8.9 (2H, t, α,α'-CH), 12.49 (²H, d, NH)

### Example 38. Preparation of 3-(4-carboxybutyl)-N-(4-sulfobutyl)-3,3',3'-trimethyl-N'-ethylindodicarbo cyanine (compound A1)

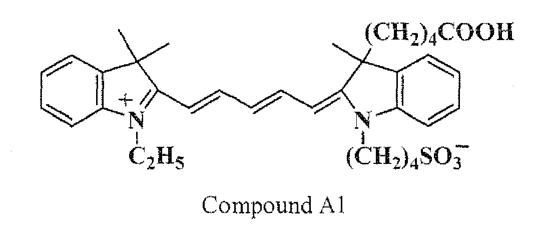

Step 1. A glass vial was charged with 2,3,3-trimethyl-*N-*cthylindolenyneum iodide (compound 27) (63.2 mg; 0.2 mmole), malonic dialdehyde dianil, hydrochloride (compound 37) (51.8 mg; 0.2 mmole), acetic anhydride (700 µL), and acetic acid (300 µL) and heated at 118°C for 2 h. The solvent was removed under vacuum, after heating was completed.

Step 2. The residue was dissolved in acetic anhydride (1.7 mL) and added the solution of 2,3-dimethyl-3-(4-carboxybutyl)-*N*-(4-sulfobutyl)-indolenyne (compound 22) (80.1 mg; 0.21 mmole) in a mixture of diisopropylethyl amine (200 µL; 269.5 mg; 2.1 mmole) and acetic anhydride (800 µL; 739.4 mg; 7.2 mmole). The reaction mixture was allowed to hold at room temperature for a period of day.

Isolation. Reaction products were preliminary purified by flash-chromatography using column RP-18. Then, the desired compound was isolated by reversed phase chromatography using column RP-18 with gradient elution in a system acetonitrile-0.05 M triethylammonium acetate (TEAA) from 0 to 50% acetonitrile. Solvents were removed and the residue was diluted with water, again applied to column RP-18, washed with 0.1M solution NaCl, and water; the product was then isolated by reversed phase chromatography using gradient elution in a system acetonitrile-water from 0 to 50% acetonitrile. The solvent was removed under vacuum; the residue was dried in vacuum desiccator over P₂O₅. 3-(4-Carboxybutyl)-*N*-(4-sulfobutyl)-3,3',3'-trimethyl-*N*'-ethylindodicarbo cyanine (compound A1) was obtained with yield of 45.7 mg (37.5%). For spectral characteristics see Table. Mass-spectrum (MALDI) (C₃₅H₄₄N₂O₅S): found m/z 606.6, calculated m/z 604.80.

¹H-NMR (DMSO-d6), δ (ppm): 0.43 (1H, m, CH₂CH₂CH₂CH₂COOH); 0.78 (1H, m, CH₂CH₂CH₂CH₂COOH); 1.24-1.31 (5H, m, CH₂CH₃, CH₂CH₂CH₂CH₂COOH); 1.64 (9H, s, 3,3',3'-CH₃); 1.73 (4H, m, CH₂CH₂CH₂CH₂SO₃); 1.89 (2H, m, CH₂CH₂CH₂CH₂COOH); 2.18 (1H, m, CH₂CH₂CH₂CH₂COOH); 2.41 (3H, m, CH₂CH₂CH₂CH₂COOH, CH₂CH₂CH₂SO₃); 4.12 (4H, m. CH₂CH₃, CH₂CH₂CH₂CH₂SO₃); 6.26 (1H, d, J=13.5, α'-CH); 6.41 (1H, d, J=13.5, α-CH); 6.57 (1H, t, J=12.0, γ-CH); 7.21-7.61 (8H, m, ArH); 8.32 (2H, m, β,β'-CH)

### Example 39. Preparation of 3-(4-carboxybutyl)-N-(4-sulfobutyl)-3,3',3'5'-tetramethyl-N'-ethylindodicarbo cyanine (compound A2)

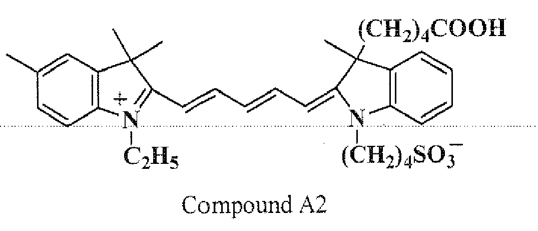

3-(4-Caxbgxybutyl)-*N*-(4-sulfobutyl)-3.3',3',5'-tetramethy-*N*'-ethylindodicarbpcyanine (compound A2) was prepared following the synthesis of 3-(4-carboxybutyl)-*N*-(4-sulfobutyl)-3,3',3'-trimethyl-*N*'-ethylindodicarbo cyanine (compound A1) using the reagents ratio as follows: (1^{st} step) 2,3,3.5-tetramethyl-*N*-ethytindolenyneum iodide (compound 31) (67 mg; 0.2 mole), malonic dialdehyde dianil, hydrochloride (compound 37) (51.8 mg; 0.2 mmole), acetic anhydride (700 µL), and acetic acid (350 µL); (2^{nd} step) acetic anhydride (1.7 mL), 2,3-dimethyl-3-(4-earboxybutyl)-*N*-(4-sulfobtyl)-indolenyne (compound 22) (80.1 mg; 0.21 mmole), a mixture of diisopropylethyl amine (200 µL; 269.5 mg; 2.1 mmole), and acetic anhydride (800 µL; 739.4 mg; 7.2 mmole). 3-(4-Carboxybutyl)-*N*-(4-sulfobutyl)-3,3',3',5'-tetramethyl-*N*'-ethylindodicarbocyanine (compound A2) was obtained with yield of 40.2 mg (31.9%). For spectral characteristics see Table. Mass-spectrum (MALDI) (C₃₆H₄₆N₂O₅S): found m/z 619.3, calculated m/z 618.83.

¹H-NMR (DMSO-d6), δ (ppm): 0.48 (1H, m, CH₂CH₂CH₂CH₂COOH); 0.78 (1H, m, CH₂CH₂CH₂CH₂COOH); 1.22-1,35 (5H, m, CH₂CH₃, CH₂CH₂CH₂CH₂COOH); 1.64 (9H, s, 3,3',3'-CH₃); 1.73 (4H, m, CH₂CH₂CH₂CH₂SO₃); 2.01 (2H, m, CH₂CH₂CH₂CH₂COOH); 2.18 (1H. m, CH₂CH₂CH₂CH₂COOH); 2.37 (3H, s, 5'-CH₃); 2.45 (3H, m, CH₂CH₂CH₂CH₂COOH, CH₂CH₂CH₂CH₂SO₃); 4.12 (4H, m, CH₂CH₃, CH₂CH₂CH₂CH₂SO₃); 6.32 (2H, m, α,α'-CH); 6.55 (1H, t, J=12.0, γ-CH); 7.21-7.55 (7H, m, ArH); 8.27 (2H, m, β,β'-CH)

### Example 40. Preparation of 3-(4-carbcxybutyl)-3,3',3',5',7'-pentamethyl-N-(4-sulfobutyl)-N'-ethylindodicarbocyanine (compound A3)

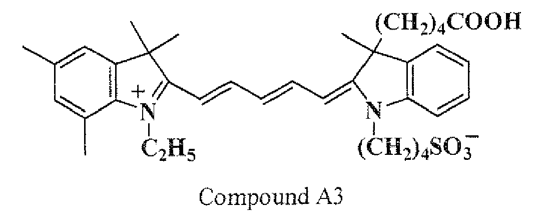

3-(4-Carboxybutyl)-3,3',3',5',7'-pentamethyl-*N*-(4-sulfobutyl)-*N*'-ethylindodiearbocyanine (compound A3) was prepared following the synthesis of 3-(4-carboxybutyl)-*N*-(4-sulfobutyl)-3,3',3'-trimethyl-N'-ethylindodicarbocyanine (compound A1) using the reagents ratio as follows: (1^{st} step) 2,3,3,5,7-pentamethyl-*N*-ethylindolenyneurn iodide (compound 33) (68.7 mg; 0.2 mmole), malonic dialdehyde dianil, hydrochloride (compound 37) (51.8 mg; 0.2 mmole), acetic anhydride (700 µL), and acetic acid (350 µL); (2^{nd} step) acetic anhydride (1.7 mL), 2,3-dimethyl-3-(4-carboxybutyl)-*N*-(4-sulfobutyl)-indolenyne (compound 22) (80. mg; 0.21 mmole), a mixture of diisopropylethyl amine (200 µL; 269.5 mg; 2.1 mmole), and acetic anhydride (800 µL; 739.4 mg; 7.2 mmole). 3-(4-Carboxybutyl)-3,3',3',5',7'-pentamethyl-*N*-(4-sulfobutyl)-*N*'-ethylindodicarbocyanine (compound A3) was obtained with yield of 19.3 mg (20.2%). For spectral characteristics see Table. Mass-spectrum (MALDI) (C₃₇H₄₈N₂O₅S): found m/z 633.6, calculated m/z 632.85.

¹H-NMR (DMSO-d6), δ (ppm): 0.49 (1H, m. CH₂CH₂CH₂CHCOOH); 0.78 (1H, m, CH2CH₂CH₂CH₂COOH); 1.33 (5H, m, CH₂CH₃, CH₂CH₂CH₂CH₂COOH); 1.64 (9H, s, 3,3',3'-CH₃); 1.72 (4H, m, CH₂CH₂CH₂CH₂SO₃); 2.01 (2H, m, CH₂CH₂CH₂CH₂COOH); 2.14 (1H, m, CH₂CH₂CH₂CH₂COOH); 2.31 (3H. s, 5'-CH₃); 2.4 (3H, m, CH₂CH₂CH₂CH₂COOH, CH₂CH₂CH₂CH₂SO₃); 2.59 (3H, s, 7'-CH₃); 4.06 (2H, m, CH₂CH₃); 4.27 (2H, m, CH₂CH₂CH₂CH₂SO₃); 6.35 (2H. m, α,α'-CH); 6.56 (1H, t, J=12.0, γ-CH); 6.99-7.54 (6H, m, ArH); 8.29 (2H, m, β,β'-CH)

### Example 41. Preparation of 3-(4-carboxybutyl)-N-(4-sulfobutyl)-3,3',3',5-tetramethyl-N'-ethylindodicarbocyanine (compound A4)

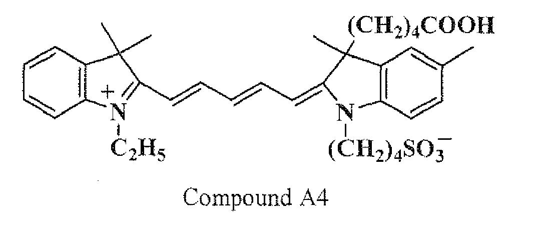

3-(4-Carboxybutyl)-*N-*(4-sulfobutyl)-3,3',3',5-tetramethyl-*N*'-ethylin:dodicarbpcyanine (compound A4) was prepared following the synthesis of 3-(4-earboxybutyl)-*N-*(4-sulfobutyl)-3,3',3'-trimethyl-*N*'-ethylindodicarbocyaninea (compound A1) using the reagents ratio as follows: (1^{st} step) 2,3,3-trimethyl-*N-*ethylindolenyneum iodide (compound 27) (63.2 mg; 0.2 mmole), malonic dialdehyde dianil, hydrochloride (compound 37) (51.8 mg; 0.2 mmole), acetic anhydride (700 µL), and acetic acid (350 µL); (2^{nd} step) acetic anhydride (1.7 mL), 3-(4-carboxybutyl)-*N*-(4-sulfobutyl)-2,3,5-trimethylindolenyne (compound 23) (83.1 mg; 0.21 mmole), a mixture of diisopropylethyl amine (200 µL; 269.5 mg; 2.1 mmole), and acetic anhydride (800 µL; 739.4 mg; 7.2 mmole). 3-(4-Carboxybutyl)-*N*-(4-sulfobutyl)-3,3',3',5. tetramethyl-*N*'-ethylindodicarbocyanine (compound A4) was obtained with yield of 23.1 mg (18.3%). For spectral characteristics see Table. Mass-spectrum (MALDI) (C₃₆H₄₆N₂O₅S); found m/z 619.1, calculated m/z 618.83.

¹H-NMR (DMSO-d6), δ (ppm): 0.48 (1H, m, CH₂CH₂CH₂CH₂COOH); 0.76 (1H, m, CH₂CH₂CH₂CH₂COOH); 1.21-1.35 (5H, m, CH₂CH₃, CH₂CH₂CH₂CH₂COOH); 1.64 (9H, s, 3,3',3'-CH₃); 1.73 (4H, m, CH₂CH₂CH₂CH₂SO₃); 1.99 (2H, m, CH₂CH₂CH₂CH₂COOH); 2.16 (1H, m. CH₂CH₂CH₂CH₂COOH); 2.36 (3H, s, 5-CH₃); 2.44 (3H, m, CH₂CH₂CH₂CH₂COOH, CH₂CH₂CH₂CH₂SO₃); 4.1 (4H, m, CH₂CH₃, CH₂CH₂CH₂CH₂SO₃); 6.3 (2H, m, α,α'-CH); 6.54 (1H, t, J=12.0, γ-CH); 7.22-7.53 (7H, m, ArH); 8.26 (2H, m, β.β'-CH)

### Example 42. Preparation of 3-(4-carboxybutyl)-3,3',3',5,5'pentamethyl-N-(4-sulfobutyl)-N'-ethylindodicarbocyanine (compound A5)

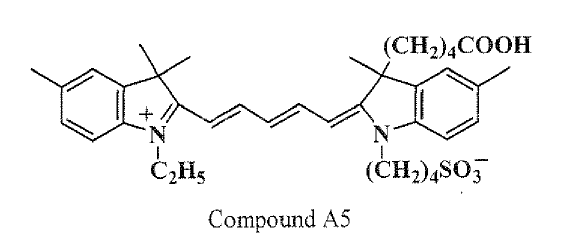

3-(4-Carboxybutyl)-3,3',3',5,5'-pentamethyl-*N*-(4-sulfobutyl)-*N*'-ethylindodicarbocyanine (compound A5) was prepared following the synthesis of 3-(4-carboxybutyl)-*N*-(4-sulfobutyl)-3,3',3'-trimethyl-*N'*-ethylindodicarbocyaninea (compound A1) using the reagents ratio as follows: (1^{st} step) 2,3,3.5-tetramethyl-*N-*ethylindolenyneum iodide (compound 31) (67 mg; 0.2 mmole), malonic dialdehyde dianil, hydrochloride (compound 37) (51.8 mg; 0.2 mmole), acetic anhydride (700 µL), and acetic acid (350 µL); (2^{nd} step) acetic anhydride (1 mL), 3-(4-carboxybutyl)-*N*'-(4-sulfobutyl)-2,3,5-trimethylindolenyne (compound 23) (84 mg; D.21 mmole), a mixture of diisopropylethyl amine (200 µL, 269.5 mg; 2.1 mmole), and acetic anhydride (800 µL; 739.4 mg; 7.2 mmole). 3-(4-Carboxybutyl)-3,3',3',5,5'-pentamethyl-*N*-(4-sulfobutyl)-*N*'-ethylindodicarbocyanine (compound A5) was obtained with yield of 35 mg (27.2%). For spectral characteristics see Table. Mass-spectrum (MALDI) (C₃₇H₄₈N₂O₅S): found m/z 632.2, calculated m/z 632.85.

¹H-NMR (DMSO-d6), δ (ppm): 0.47 (1H, m, CH₂CH₂CH₂CH₂COOH); 0.75 (1H, m, CH₂CH₂CH₂CH₂COOH); 1.23-1.35 (5H, m, CH₂CH₃, CH₂CH₂CH₂CH₂COOH); 1.63 (9H, s, 3,3',3'-CH₃); 1.74 (4H, m, CH₂CH₂CH₂CH₂SO₃); 1.93 (2H, m, CH₂CH₂CH₂CH₂COOH); 2.13 (1H, m, CH₂CH₂CH₂CH₂COOH); 2.36 (6H, s, 5.5'-CH₃); 2.43 (3H, m, CH₂CH₂CH₂CH₂COOH, CH₂CH₂CH₂CH₂SO₃); 4.09 (4H, m, CH₂CH₃, CH₂CH₂CH₂CH₂SO₃); 6.22 (1H, d, J=13.5, α'-CH); 6.36 (1H, d, J=13.5, α-CH): 6.52 (1H, t, J=12.0, γ-CH); 7.18-7.42 (6H. m, ArH); 8.25 (2H, t, J=13, β,β'-CH)

### Example 43. Preparation of 3,3',3',5,5'.7'-hexamethyl-3-(4-carboxybutyl)-N-(4-sulfobutyl)-N'-ethylindodicarbocyanine (compound A6)

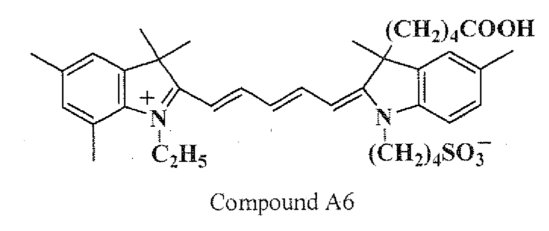

3,3',3',5,5'.7'-Hexamethyl-3-(4-carboxybutyl)-*N-*(4-sulfobutyl)-*N*'-ethylindodicarbocyanine (compound A6) was prepared following the synthesis of 3-(4-carboxybutyl)-*N*-(4-sulfobutyl)-3,3',3'-trirnethyl-*N*'-ethylindodiearbocyanine (compound A1) using the reagents ratio as follows: (1^{st} step) 2,3,3,5,7-pentamethyl-*N-*ethylindolenyneum iodide (compound 33) (68.7 mg; 0.2 mmole), malonic dialdehyde dianil, hydrochloride (compound 37) (51.8 mg; 0.2 mmole), acetic anhydride (700 µL), and acetic acid (350 µL); (2^{nd} step) acetic anhydride (1.7 mL), 2,3-dimethyl-3-(4-carboxybutyl)-*N-*(4-sulfobutyl)-indolenyne (compound 23) (80.1 mg; 0.21 mmole), a mixture of diisopropylethyl amine (200 µL; 269.5 mg; 2.1 mmole) and acetic anhydride (800 µL; 739.4 mg; 7.2 mmole). 3,3',3',5,5'.7'-Hexamethyl-3-(4-carboxybutyl)-*N-*(4-sulfobutyl)-*N*'-ethylindodicarbocyanine (compound A6) was obtained with yield of 22.2 mg (17.1%). For spectral characteristics see Table. Mass-spectrum (MALDI) (c₃₈H₅₀N₂O₅S): found m/z 647.2, calculated m/z 646.88.

¹H-NMR (DMSO-d6), δ (ppm): 0.5 (1H, m, CH₂CH₂CH₂CH₂COOH); 0.78 (1H, m, CH₂CH₂CH₂CH₂COOH); 1.34 (5H, m, CH₂CH₃, CH₂CH₂CH₂CH₂COOH); 1.64 (9H, s, 3,3',3'-CH₃); 1.74 (4H, m, CH₂CH₂CH₂CH₂SO₃); 2.02 (2H, m, CH₂CH₂CH₂CH₂COOH); 2.17 (1H, m, CH₂CH₂CH₂CH₂COOH); 2.33 (6H, s, 5.5'-CH₃); 2.41 (3H, m, CH₂CH₂CH₂CH₂COOH, CH₂CH₂CH₂CH₂SO₃); 2.6 (3H, s, 7'-CH₃); 4.09 (2H, m, CH₂CH₃); 4.27 (2H, m, CH₂CH₂CH₂CH₂SO₃); 6.36 (2H, m, α,α'-CH); 6.55 (1H, t, J=12.0, γ-CH); 7.1-7.6 (5H, m, ArH); 8.3 (2H, m, β,β'-CH).

### Example 44. Preparation of N,N'-diethyl-3-(4-carboxybutyl)-5'-sulfo-3,3',3'-trimethylindodicarbocyanine (compound A7)

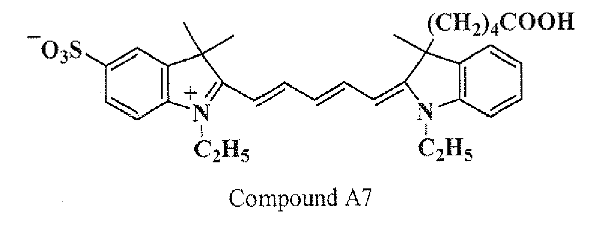

*N,N*'-Diethyl-3-(4-carboxybutyl)-5'-sulfo-3,3',3'-trimethylindodicarbocyanine (compound A7) was prepared following the synthesis of 3-(4-carboxybutyl)-*N*-(4-sulfobutyl)-3,3',3'-trimethyl-*N*'-ethylindodicarbocyanine (compound A1) using the reagents ratio as follows: (1^{st} step) 2,3-dimethyl-3-(4-carboxybutyl)-*N*-ethylindolenyneum iodide (compound 21) (80.2 mg; 0.2 mmole), malonic dialdehyde dianil, hydrochloride (compound 37) (72.5 mg; 0.28 mmole), acetic anhydride (3 mL), and acetyl chloride (65 µL); (2^{nd} step) 5-sulfo-2,3,3-trimethyl-*N-*ethylindolenyne (compound 29) (133.7 mg; 0.5 mmole), acetic anhydride (3 mL), and diisopropylethyl amine (120 µL; 161.7 mg; 1.25 mmole). *N,N*'-Diethyl-3-(4-earboxbutyl)-5'-sulfo-3.3',3'-trimethylindodicarbocyanine (compound A7) was obtained with yield of 31 mg (26.9%). For spectral characteristics see Table. Mass-spectrum (MALDI) (C₃₃H₄₀N₂O₅S): found m/z 577.0, calculated m/z 576.75.

¹H-NMR (DMSO-d6), δ (ppm): 0.46 (1H, m, CH₂CH₂CH₂CH₂COOH); 0.75 (1H, m, CH₂CH₂CH₂CH₂COOH); 1.23-1.33 (8H, m, CH₂CH₃, CH₂CH₂CH₂CH₂COOH); 1.68 (9H, s, 3,3',3'-CH₃); 1.89 (2H, m, CH₂CH₂CH₂CH₂COOH); 2.16 (1H, m, CH₂CH₂CH₂CH₂COOH); 2.4 (1H, m, CH₂CH₂CH₂CH₂COOH); 4.13 (4H, m, CH₂CH₃); 6.26 (1H, d, J=13.5, α-CH); 6.34 (1H, d, J=13.5, α-CH); 6.56 (1H, t, J=12,0, γ-CH); 7.2-7.8 (7H, m, ArH); 8.35 (2H, t, J=13, β,β'-CH)

### Example 45. Preparation of 3-(4-carboxybutyl)-N'-(4-sulfobutyl)-3,3',3'-trimethyl-N-ethylindodicarbocyanine (compound A8)

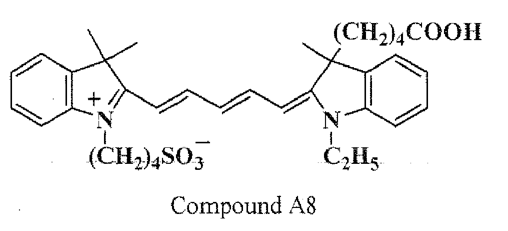

3-(4-Carboxybutyl)-*N'*-(4-sulfobutyl)-3,3',3'-trimethyl-*N*-ethylindodicarbocyanine (compound A8) was prepared following the synthesis of 3-(4-carboxybutyt)-*N*-(4-sutfobutyl)-3,3',3'-trimethyl-*N*'-ethylindodicarbocyanine (compound A1) using the reagents ratio as follows: (1^{st} step) 2,3-dimethyl-3-(4-carboxybutyl)-*N*-ethylindolenyneum iodide (compound 21) (80.2 mg; 0.2 mmole), malonic dialdehyde dianil, hydrochloride (compound 37) (72.5 mg; 0.28 mmole), acetic anhydride (3 mL), and acetyl chloride (65 µL); (2^{nd} step) *N*-(4-sulfobutyl)-2,3,3-trimethylindolenyne (compound 26) (148.7 mg; 0.5 mmole), acetic anhydride (3 mL), and diisopropylethyl amine (120 µL; 161.7 mg; 1.25 mmole). 3-(4-Carboxybutyl)-*N*'-(4-sulfobutyl)-3,3',3'-trimethyl-*N*-ethylindodicarbocyanine (compound A8) was obtained with yield of 34 mg (28%). For spectral characteristics see Table. Mass-spectrum (MALDI) (C₃₅H₄₄N₂O₅S): found *mlz* 605.7, calculated m/z 604.80.

¹H-NMR (DMSO-d6), δ (ppm): 0.48 (1H, m, CH₂CH₂CH₂CH₂COOH); 0.79 (1H, m, CH₂CH₂CH₂CH₂COOH); 1,2-1.35 (5H, m, CH₂CH₃, CH₂CH₂CH₂CH₂COOH); 1.65-1.81 (13H, m, 3,3',3'-CH₃, CH₂CH₂CH₂CH₂SO₃); 2.02 (2H, m, CH₂CH₂CH₂CH₂COOH); 2.17 (1H, m, CH₂CH₂CH₂CH₂COOH); 2.34 (3H, m, CH₂CH₂CH₂CH₂COOH, CH₂CH₂CH₂CH₂SO₃); 4.12 (4H, m, CH₂CH₃, CH₂CH₂CH₂CH₂SO₃); 6.35 (2H, m, α,α'-CH); 6.58 (1H, m, γ-CH); 7.22-7.61 (8H, m, ArH); 8.3 (2H. m, β,β'-CH)

### Example 46. Preparation of N,N'-diethyl-3-(4-carboxybutyl)-5-sulfo-3,3',3'-trimethylindodicarbocyanine (compound A9)

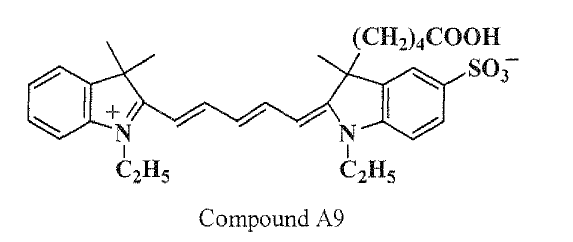

Step 1. A glass vial was charged with 2,3-dimethyl-3-(4-carboxybutyl)-5-sulfo-*N-*ethylindolenyne (compound 24) (70.7 mg; 0.2 mmole), malonic dialdehyde dianil, hydrochloride (compound 37) (51.8 mg; 0.2 mmole), acetic anhydride (700 µL), and acetic acid (350 µL). The mixture was heated at 118°C for 2 h.

Steep 2. 2,3.3-Trimethyl-*N*-ethylindolenyneum iodide (compound 27) (66.2 mg; 0.21 mmole), anhydrous potassium acetate (98.1 mg; 1 mmole), acetic anhydride (700 µL), and acetic acid (350 µL) were added to the reaction mixture and heated additionally for 2 h.

Isolation. Reaction products were preliminary purified by flash-chromatography using column RP-18. Then, the desired compound was isolated by reversed phase chromatography using column RP-18 with gradient elution in a system acetonitrile-0.05 M TEAA from 0 to 50% acetonitrile. Solvents were removed and the residue was diluted with water, again applied to column RP-18, washed with 0.1M solution NaCl, and water; the product was then isolated by reversed phase chromatography using gradient elution in a system acetonitrile-water from 0 to 50% acetonitrile. The solvents were removed under vacuum; the residue was dried in vacuum desiccator over P₂O₅. *N,N'*-diethyl-3-(4-carboxybutyl)-5-sulfo-3,3',3'-trimethylindodicarbo cyanine (compound A9) was obtained with yield of 20.2 mg (17.5%). For spectral characteristics see Table. Mass-spectrum (MALDI) (C₃₃H₄₀N₂O₅S): found m/z 577.3, calculated m/z 576.75.

¹H-NMR (DMSO-d6), δ (ppm): 0.47 (1H, m, CH₂CH₂CH₂CH₂COOH); 0.77 (1H, m, CH₂CH₂CH₂CH₂COOH); 1.24-1.36 (8H, m, CH₂CH₃, CH₂CH₂CH₂CH₂COOH); 1.7 (9H, s, 3,3',3'-CH₃); 1.9 (2H, m, CH₂CH₂CH₂CH₂COOH); 2.16 (1H, m, CH₂CH₂CH₂CH₂COOH); 2.42 (1H, is, CH₂CH₂CH₂CH₂COOH); 4.13 (4H, m, CH₂CH₃); 6.27 (1H, d, J=13.5, α'-CH); 6.35 (1H, d, J=13.5, α-CH); 6.57 (1H, t, J=12.0, γ-CH); 7,21-7.75 (7H, m, ArH); 8,34 (2H, t, J=13, β,β'-CH).

### Example 47. Preparation of N,N'-di(4-sulfabutyl)-3-(4-carboxybutyl)-3,3',3'-trimethylindodicarbocyanine, sodium salt (compound A10)

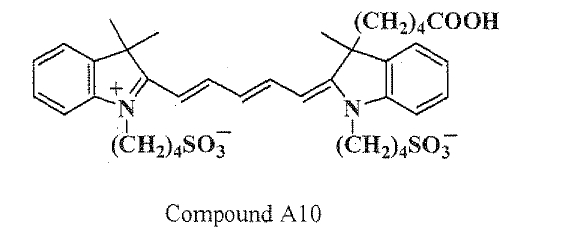

*N,N*'-di(4-sulfobutyl)-3-(4-carboxybutyl)-3,3',3'-trimethylindodicarbocyanine, sodium salt (compound A10) was prepared following the synthesis of *N,N*'-diethyl-3-(4-carbaxybutyl)-5-sulfo-3,3',3'-trimethylindodicarbocyanine (compound A9) using the reagents ratio as follows: (1^{st} step) *N*-(4-sulfabutyl)-2,3,3-trimethylindolenyne (compound 26) (59.1 mg; 0.2 mmole), malonic dialdehyde dianil, hydrochloride (compound 37) (51.8 mg; 0.2 mmole), acetic anhydride (700 µL), and acetic acid (160 µL); (2^{nd} step) 2,3-dimethyl-3-(4-carboxybutyl)-*N*-(4-sulfobutyl)indolenyne (compound 22) (80.1 mg; 0.21 mmole), anhydrous potassium acetate (117 mg; 1.2 mmole), acetic anhydride (700 µL), and acetic acid (350 µL). *N,N*'-di(4-Sulfobutyl)-3-(4-carboxybutyl)-3,3',3'-trimethylindodicarbocyanine, sodium salt (compound A10) was obtained with yield of 71 mg (48%). For spectral characteristics see Table. Mass-spectrum (MALDI) (C₃₇H₄₇N₂O₈S⁻): found m/z 712.1, calculated m/z 711.91.

¹H-NMR (DMSO-d6), δ (ppm): 0.45 (1H, m, CH₂CH₂CH₂CH₂COOH); 0.77 (1H, m, CH₂CH₂CH₂CH₂COOH); 1.32 (2H, m, CH₂CH₂CH₂CH₂COOH); 1.63-1.8 (17H, m, 3,3',3'-CH₃, CH2CH₂CH₂CH₂SO₃); 1.91 (2H, m, CH₂CH₂CH₂CH₂COOH); 2.17 (1H, m, CH2CH2CH2CH₂COOH); 2.42 (5H, m, CH₂CH₂CH₂CH₂COOH, CH₂CH₂CH₂CH₂SO₃); 4.09 (4H, m, CH₂CH₂CH₂CH₂SO₃); 6.37 (2H, m, α,α'-CH); 6.57 (1H, t, J=12.0, γ-CH); 7.21-7.6 (8H, m, ArH); 8.3 (2H, m, β,β'-CH)

### Example 48. Preparation of 3-(4-carboxybutyl)-N-(4-sulfobutyl)-5'-sulfo-3,3',3',-trimethyl-N'-ethylindodicarbocyanine, sodium salt (compound A11)

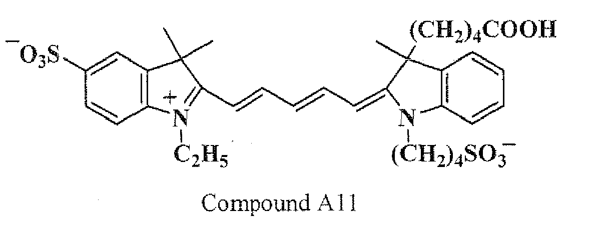

3-(4-Carboxybutyl)-*N*-(4-sulfobutyl)-5'-sulfo-3,3',3'-trimethyl-*N*'-ethylindodicarbocyanine, sodium salt (compound A11) was prepared following the synthesis of *N,N*'-diethyl-3-(4-carboxybutyl)-5-sulfo-3,3',3'-trimethylindodicarbocyanine, sodium salt (compound A9) using the reagents ratio as follows: (1^{st} step) 5-sulfo-2,3,3-trimethyl-*N*-ethylindolenyne (compound 29) (53.3 mg; 0.2 mmole), malonic dialdehyde dianil, hydrochloride (compound 37) (51.8 mg; 0.2 mmole), acetic anhydride (700 µL), and acetic acid (160 µL); (2^{nd} step) 2,3-dimethyl-3-(4-carboxybutyl)-*N*-(4-sulfobutyl)-indolenyne (compound 22) (80.1 mg; 0.21 mmole), anhydrous potassium acetate (117 mg; 1.2 mmole), acetic anhydride (700 µL), and acetic acid (350 µL). 3-(4-Carboxybutyl)-*N*-(4-sulfobutyl)-5'-sulfo-3,3',3',-trimethyl-*N*'-ethylindodicarboeyanine, sodium salt (compound A11) was obtained with yield of 31.5 mg (22.4%). For spectral characteristics see Table. Mass-spectrum (MALDI) (C₃₅H₄₃N₂O₈S₂⁻): found m/z 684.3, calculated m/z 683.86.

¹H-NMR (DMSO-d6), δ (ppm): 0.47 (1H, m, CH₂CH₂CH₂CH₂COOH); 0.77 (1H, m, CH₂CH₂CH₂CH₂COOH); 1.24-1.38 (5H, m. CH₂CH₃, CH₂CH₂CH₂CH₂COOH); 1.68 (13H, m, 3.3',3'-CH₃, CH₂CH₂CH₂CH₂SO₃); 2.0 (2H, m, CH₂CH₂CH₂CH₂COOH); 2.19 (1H, m, CH₂CH₂CH₂CH₂COOH); 2.4 (3H, m, CH₂CH₂CH₂CH₂COOH, CH₂CH₂CH₂CH₂SO₃); 4.13 (4H, m, CH₂CH₃. CH₂CH₂CH₂CH₂SO₃); 6.26 (1H, d, J=13.5, α'-CH); 6.43 (1H, d, J=13.5, α-CH); 6.57 (1H, t, J=12.0, γ-CH); 7.23-7.79 (7H, m, ArH); 8.33 (2H, t, J=13, β,β'-CH)

### Example 49. Preparation of N,N'-di(4-sulfobutyl)-3-(4-carboxybutyl)-3.3'.3',5'-tetramethylindodicarbocyanine, sodium salt (compound A12)

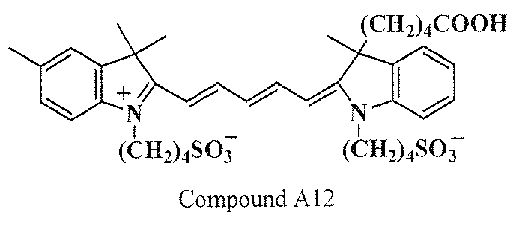

*N,N*'-Di(4-sulfobutyl)-3-(4-carboxybutyl)-3,3',3',5'-tetramethylindodicarbocyanine, sodium salt (compound A12) was prepared following the synthesis of 3-(4-carboxybutyl)-*N-*(4-sulfobutyl)-3,3',3'-trimethyl-*N*'-ethylindodicarbocyanine (compound A1) using the reagents ratio as follows: (1^{st} step) *N*-(4-sulfobutyl)-2,3,3.5-tetramethylindolenyne (61.9 mg; 0.2 mmole) (compound 30), malonic dialdehyde dianil, hydrochloride (compound 37) (51.8 mg; 0.2 mmole), acetic anhydride (700 µL), and acetic acid (160 µL); (2^{nd} step) acetic anhydride (1.7 mL), 2,3-dimethyl-3-(4-carboxybutyl)-*N*-(4-sulfobutyl)-mdolenyne (compound 22) (80.1 mg; 0.21 mmole), and a mixture of diisopropylethyl amine (200 µL; 269.5 mg; 2.1 mmole) and acetic anhydride (800 µL; 739.4 mg; 7.2 mmole). *N,N*'-Di(4-sulfobutyl)-3-(4-carboxybutyl)-3,3',3',5'-tetramethylindodicarbocyanine, sodium salt (compound A12) was obtained with yield of 53.5 mg (35.7%). For spectral characteristics see Table. Mass-spectrum (MALDI) (C₃₈H₄₉N₂O₈S₂ found m/z 726.9, calculated m/z 725.94.

¹H-NMR (DMSO-d6), δ (ppm): 0.47 (1H, m, CH₂CH₂CH₂CH₂COOH); 0.79 (1H, m, CH₂CH₂CH₂CH₂COOH); 1.32 (2H, m, CH₂CH₂CH₂CH₂COOH); 1.65-1.8 (17H, m, 3,3',3'-CH₃, CH₂CH₂CH₂CH₂SO₃); 2.03 (2H, m, CH₂CH₂CH₂CH₂COOH); 2.14 (1H, m, CH₂CH₂CH₂CH₂COOH); 2.36 (4H, s, 5'-CH₃, CH₂CH₂CH₂CH₂COOH); 2.48 (4H, m, CH₂CH₂CH₂CH₂SO₃); 4.08 (4H, m. CH₂CH₂CH₂CH₂SO₃); 6.35 (2H, t, J=14, α,α'-CH); 6.57 (1H, t, J=12.5, γ-CH); 7.19-7.54 (7H, m, ArH); 8.3 (2H, m, β,β'-CH)

### Example 50. Preparation of N,N'-di(4-sulfobutyl)-3-(4-carboxybutyl)-3,3',3',5',7'-pentamethylindodicarbocyanine, sodium salt (compound A13)

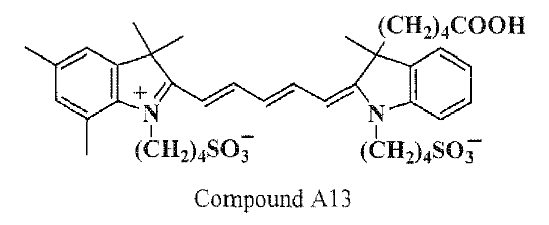

*N,N*'-Di(4-sulfobutyl)-3-(4-carbaxybutyl)-3,3',3',5',7'-pentamethylindadicarbocyanine, sodium salt (compound A13) was prepared following the synthesis of *N,N*'-diethyl-3-(4-carboxybutyl)-5-sulfo-3,3',3'-trimethylindodiearbocyanme, sodium salt (compound A9) using the reagents ratio as follows: (1^{st} step) 2,3,3,5,7-pentamethyl-*N*-(4-sulfobutyl)indolenyne (compound 32) (64.7 mg; 0.2 mmole), malonic dialdehyde dianil, hydrochloride (compound 37) (51.8 mg; 0.2 mmole), acetic anhydride (700 µL), and acetic acid (200 µL); (2^{nd} step) 2,3-dimethyl-3-(4-carboxybutyl)-*N*-(4-sulfobutyl)-indolenyne (compound 22) (80.1 mg; 0.21 mmole), anhydrous potassium acetate (200 mg; 2 mole), acetic anhydride (700 µL), and acetic acid (350 µL). *N,N'*-Di(4-sulfobutyl)-3-(4-carboxybutyl)-3,3',3',5',7'-pentamethylindodicarbocyanine, sodium salt (compound A13) was obtained with yield of 8.2 mg (5.4%). For spectral characteristics see Table. Mass-spectrum (MALDI) (C₃₉H₅₁N₂O₈S₂): found m/z 741.2, calculated m/z 739.96.

¹H-NMR (DMSO-d6), δ (ppm): 0.48 (1H, m, CH₂CH₂CH₂CH₂COOH); 0.79 (1H, m, CH₂CH₂CH₂CH₂COOH); 1.32 (2H, m, CH₂CH₂CH₂CH₂COOH); 1.67-1.81 (17H, m, 3,3',3'-CH₃, CH₂CH₂CH₂CH₂SO₃); 2.05 (2H, m, CH₂CH₂CH2CH₂COOH); 2.18 (1H, m, CH₂CH₂CH₂CH₂COOH); 2.36 (4H, s, 5'-CH₃, CH₂CH₂CH₂CH₂COOH); 2.47 (4H, m, CH₂CH₂CH₂CH₂SO₃); 2.61 (3H, s, 7'-CH₃); 4.12 (4H, m, CH₂CH₂CH₂CH₂SO₃); 6.37 (2H, t, J=14, α,α'-CH); 6.59 (1H, t. J=12.5, γ-CH); 7.21-7.55 (6H, m, ArH); 8.33 (2H, m, β,β'-CH)

### Example 51. Preparation of 3-(4-carboxybutyl)-5-sulfo-N-(4-sulfobutyl)-3,3',3'-trimethyl-N'-ethylindodicarbocyanine, sodium salt (compound A14)

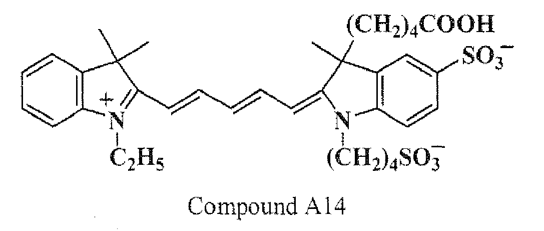

3-(4-Carboxybutyl)-5-sulfo-*N*-(4-sulfobutyl)-3,'3',3'-trimethyl-*N*-ethylindodicarbocyanine, sodium salt (compound A14) was prepared following the synthesis of *N,N*'-diethyl-3-(4-carboxybutyl)-5-sulfo-3,3',3'-trimethylindodicarbocyaninea (compound A9) using the reagents ratio as follows: (1^{st} step) 2,3-dimethyl-3-(4-carbnxybutyl)-*N*-(4-sulfobutyl)-5-sulfaindolenyne, potassium salt (compound 25) (99.9 mg; 0.2 mmole), malonic dialdehyde dianil, hydrochloride (compound 37) (51.8 mg; 0.2 mmole), acetic anhydride (700 µL), and acetic acid (350 µL); (2^{nd} step) 2,3,3-trimethyl-*N*-ethylindolenyneum iodide (compound 27) (69.1 mg; 0.21 mmole), anhydrous potassium acetate (98 mg; 1 mmole), acetic anhydride (350 µL), and acetic acid (700 µL). 3-(4-Carboxybutyl)-5-sulfo-*N*-(4-sulfobutyl)-3,3',3'-trimethyl-*N*'-ethylindodicarbocyanine, sodium salt (compound A14) was obtained with yield of 10 mg (7,1%). For spectral characteristics see Table. Mass-spectrum (MALI) (C₃₅H₄₃N₂O₈S₂): found m/z 683.1, calculated m/z 683.86.

¹H-NMR (DMSO-d6), δ (ppm): 0.48 1H, m, CH₂CH₂CH₂CH₂COOH); 0.78 (1H, m, CH₂CH₂CH₂CH₂COOH); 1.25-1.39 (5H, m, CH₂CH₃, CH₂CH₂CH₂CH₂COOH); 1.7 (13H, m. 3,3',3'-CH₃, CH₂CH₂CH₂CH₂SO₃); 2.04 (2H, m, CH₂CH₂CH₂CH₂COOH); 2.21 (1H, m, CH₂CH₂CH₂CH₂COOH); 2.4 (3H, m, CH₂CH₂CH₂CH₂COOH, CH₂CH₂CH₂CH₂SO₃); 4.12 (4H, m, CH₂CH₃, CH₂CH₂CH₂CH₂SO₃); 6.28 (1H, d, J=13.5. α'-CH); 6.45 (1H, d, J=13.5, α-CH); 6.57 (1H, t, J=12.0, γ-CH); 7.25-7.8 (7H, m, ArH); 8.35 (2H, t, J=13, β,β'-CH)

### Example 52. Preparation of 3-(4-carboxybutyl)-5-sulfo-N-(4-sulfobutyl)-3,3',3',5'-tetrarnethyl-N'-ethylindodicarbocyanine, sodium salt (compound A15)

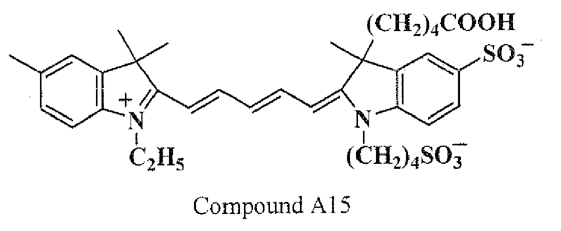

3-(4-Carboxybutyl)-5-sulfo-*N*-(4-sulfobutyl)-3,3',3',5'-tetramethyl-*N*'-ethylindodicarbocyanine, sodium salt (compound A15) was prepared following the synthesis of *N,N'*-diethyl-3-(4-carboxybutyl)-5-sulfo-3,3',3'-trimethylindodicarbocyanine (compound A9) using the reagents ratio as follows: (1^{st} step) 2,3-dimethyl-3-(4-carboxybutyl)-*N*-(4-sulfobutyl)-5-sulfoindolenyne, potassium salt (compound 25) (99.9 mg; 0.2 mmole), malonic dialdehyde dianil, hydrochloride (compound 37) (51.8 mg; 0.2 mmole), acetic anhydride (700 µL), and acetic acid (350 µL); (2^{nd} step) 2,3,3.5-tetramethyl-*N-*ethylindolenyneum iodide (compound 31) (69.1 mg; 0.21 mmole), anhydrous potassium acetate (118 mg; 1.2 mmole), acetic anhydride (3 50 µL), and acetic acid (700 µL). 3-(4-Carboxybutyl)-5-sulfo-*N*-(4-sulfobutyl)-3,3',3',5'-tetramethyl-*N*'-ethylindodicarbo cyanine, sodium salt (compound A15) was obtained with yield of 13.3 mg (9.2%). For spectral characteristics see Table. Mass-spectrum (MALDI) (C₃₆H₄₅N₂O₈S₂⁻): found m/z 697.1, calculated m/z 697.88.

¹H-NMR (DMSO-d6), δ (ppm): 0.48 (1H, m, CH₂CH₂CH₂CH₂COOH); 0.78 (1H, m, CH₂CH₂CH₂CH₂COOH); 1.24 (3H, t, CH₂CH₃); 1.37 (2H, m, CH₂CH₂CH₂CH₂COOH); 1.66 (9H, s, 3,3',3'-CH₃); 1.74 (4H, m, CH₂CH₂CH₂CH₂SO₃); 2.01 (2H, m, CH₂CH₂CH₂CH₂COOH); 2.18 (1H, m, CH₂CH₂CH₂CH₂COOH); 2.4 (4H, s, 5'-CH₃, CH₂CH₂CH₂CH₂COOH); 2.59 (2H, m, CH₂CH₂CH₂CH₂SO₃); 4.12 (4H, m, CH₂CH₃, CH₂CH₂CH₂CH₂SO₃); 6.23 (1H, d, J=13.5, α'-CH); 6.42 (1H, d, J=13.5, α-CH); 6.57 (1H, t, J=12.0, γ-CH); 7.23-7.75 (6H, m, ArH); 8.3 (2H, t, J=13, β,β'-CH)

### Example 53. Preparation of 3-(4-carboxybutyl)-3,3',3',5',7'-pentamethyl-5-sulfo-N-(4-sulfobutyl)-N'-ethylindodicarbocyanine, sodium salt (compound A16)

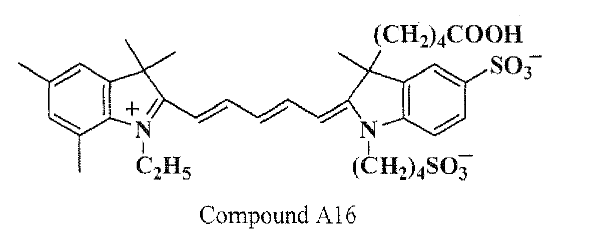

3-(4-Carboxybutyl)-3,3',3',5',7-pentamethyl-5-sulfo-*N*-(4-sulfobutyl)-*N*"ethylindodicarbo cyanine, sodium salt (compound A16) was prepared following the synthesis of *N*,*N*-diethyl-3-(4-carboxybutyl)-5-sulfo-3,3',3'-trimethylindodicarbocyanine (compound A9) using the reagents ratio as follows: (1^{st} step) 2,3-dimethyl-3-(4-carboxybutyl)-*N*-(4-sulfobutyl)-5-sulfoindolenyne, potassium salt (compound 25) (99.9 mg; 0.2 mmole), malonic dialdehyde dianil, hydrochloride (compound 37) (51.8 mg; 0.2 mmole), acetic anhydride (700 µL), and acetic acid (350 µL); (2^{nd} step) 2,3,3,5,7-pentamethyl-*N*-ethylindolenyneum iodide (compound 33) (68.7 mg; 0.21 mmole), anhydrous potassium acetate (98.1 mg; 1 mmole), acetic anhydride (350 µL), and acetic acid (700 µL). 3-(4-Carboxybutyl)-3,3',3',5',7'-pentamethyl-5-sulfo-*N*-(4-sulfobutyl)-*N'-*ethylindodicarbocyanine, sodium salt (compound A16) was obtained with yield of 8.5 mg (5.8%). For spectral characteristics see Table. Mass-spectrum (MALDI) (C₃₇H₄₇N₂O₈S2⁻): found m/z 711.3, calculated m/z 711.91.

¹H-NMR (DMSO-d6), δ (ppm): 0.5 (1H, m, CH₂CH₂CH₂CH₂COOH); 0.79 (1H, m, CH₂CH₂CH₂CH₂COOH); 1.35 (5H, m, CH₂CH₃, CH₂CH₂CH₂CH₂COOH); 1.65 (9H, s, 3,3',3'-CH₃); 1.75 (4H, m, CH₂CH₂CH₂CH₂SO₃); 2.05 (2H, m, CH₂CH₂CH₂CH₂COOH); 2.18 (1H, m, CH₂CH₂CH₂CH₂COOH); 2.32 (6H, s, 5.5'-CH₃); 2.42 (3H, m, CH₂CH₂CH₂CH₂COOH, CH₂CH₂CH₂CH₂SO₃); 2.6 (3H, s, 7'-CH₃); 4.08 (2H, m, CH₂CH₃); 4.28 (2H, m, CH₂CH₂CH₂CH₂SO₃); 6.37 (2H, m, α,α'-CH); 6.59 (1H, t, J=12.0, γ-CH); 7.1-7.59 (5H, m, ArH); 8.3 (2H, m, β,β'-CH)

### Example 54. Preparation of N,N'-di(4-sulfobutyl)-3-(4-carboxybutyl)-3,3',3',5-tetramethylindodicarbocyanine, sodium salt (compound A17)

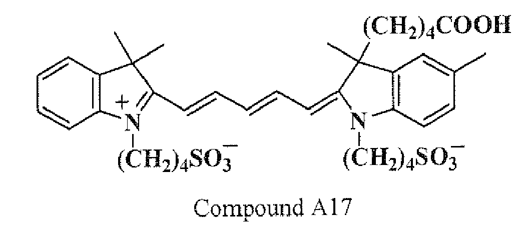

*N,N'*-Di(4-suifobutyl)-3-(4-carboxybutyl)-3,3',3',5-tetramethylindodicarbocyanine, sodium salt (compound A17) was prepared following the synthesis of 3-(4-carboxybutyl)-*N*-(4-sulfobutyl)-3,3',3'-trimethyl-*N*'-ethylindodicarbocyanine (compound A1) using the reagents ratio as follows: (1^{st} step) *N*-(4-sulfobutyl)-2,3,3-trimethylindolenyne (compound 26) (59.1 mg; 0.2 mmole), malonic dialdehyde dianil, hydrochloride (compound 37) (51.8 mg; 0.2 mmole), acetic anhydride (700 µL), and acetic acid (150 µL); (2^{nd} step) acetic anhydride (1.7 mL), 3-(4-carboxybutyl)-*N-*(4-sulfobutyl)-2,3,5-trimethylindolenyne (compound 23) (83.1 mg, 0.21 mmole), and a mixture of diisopropylethyl amine (200 µL; 269.5 mg; 2.1 mmole) and acetic anhydride (800 µL; 739.4 mg; 7.2 mole). *N*,*N*'-Di(4-sulfobutyl)-3-(4-carboxybutyl)-3,3',3',5-tetramethylindodicarbo cyanine, sodium salt (compound A17) was obtained with yield of 42.8 mg (28.6%). For spectral characteristics see Table. Mass-spectrum (MALDI) (C₃₈H₄₉N₂O₈S₂⁻): found m/z 725.1, calculated m/z 725.94.

¹H-NMR (DMSO-d6), δ (ppm): 0.48 (1H, m, CH₂CH₂CH₂CH₂COOH); 0.77 (1H, m, CH₂CH₂CH₂CH₂COOH); 1.32 (2H, m, CH₂CH₂CH₂CH₂COOH); 1.65-1.8 (17H, m, 3,3',3'-CH₃, CH₂CH₂CH₂CH₂SO₃); 2.00 (2H, m, CH₂CH₂CH₂CH₂COOH); 2.15 (1H, m, CH₂CH₂CH₂CH₂COOH); 2.37 (4H, s, 5'-CH₃, CH₂CH₂CH₂CH₂COOH); 2.43 (4H, m, CH₂CH₂CH₂CH₂SO₃); 4.08 (4H, m, CH₂CH₂CH₂SO₃); 6.28 (1H, d, J=13.5, α'-CH); 6.39 (1H, d, J=13.5, α-CH); 6.57 (1H, t, J=12.0, γ-CH); 7.19-7.59 (7H, m, ArH); 8.28 (2H, t, J=13, β,β'-CH).

### Example 55. Preparation of 3-(4-carboxybutyl)-N-(4-sulfobutyl)-5'sulfo-3,3',3'.5-tetramethyl-N'-ethylindodicaxbocyanine, sodium salt (compound A18)

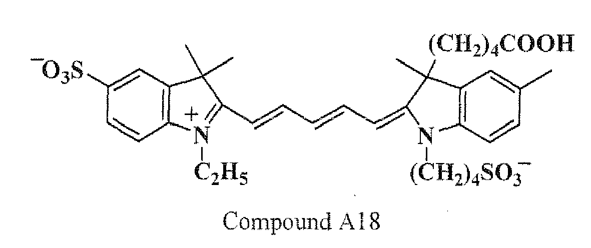

3-(4-Carboxybutyl)-*N*-(4-sulfobutyl)-5'-sulfo-3,3',3'.5-tetramethyl-*N*'-ethylindodicarbocyanine, sodium salt (compound A18) was prepared following the synthesis of *N*,*N'*-diethyl-3-(4-carboxybutyl)-5-sulfo-3,3',3'-trimethylindodicarbocyanine (compound A9) using the reagents ratio as follows: (1^{st} step) 5-sulfo-2,3,3-trimethyl-*N*-ethylindolenyne (compound 29) (53.3 mg; 0.2 mmole), malonic dialdehyde dianil, hydrochloride (compound 37) (51.8 mg; 0.2 mmole), acetic anhydride (700 µL), and acetic acid (200 µL); (2^{nd} step) 3-(4-carboxybutyl)-*N*-(4-sulfobutyl)-2,3,5-trimethylindolenyn (compound 23) (84 mg; 0.21 mmole), anhydrous potassium acetate (236 mg; 2.4 mmole), acetic anhydride (500 µL), and acetic acid (700 µL). 3-(4-Carboxybutyl)-*N*-(4-sulfobutyl)-5'-sulfo-3,3',3'.5-tetramethyl-*N*'-ethylindodicarbocyanine, sodium salt (compound A18) was obtained with yield of 25.5 mg (17.7%). For spectral characteristics see Table. Mass-spectrum (MALDI) (C₃₆H₄₅N₂O₈S₂⁻): found m/z 698.5, calculated m/z 697.88.

¹H-NMR (DMSO-d6), δ (ppm): 0.49 (1H, m, CH₂CH₂CH₂CH₂COOH); 0.77 (1H, m, CH₂CH₇CH₂CH₂COOH); 1.22 (3H, t, CH₂CH₃); 1.36 (2H, m, CH₂CH₂CH₂CH₂COOH); 1.64 (9H, s, 3,3',3'-CH₃); 1.74 (4H, m, CH₂CH₂CH₂CH₂SO₃); 2.02 (2H, m, CH₂CH₂CH₂CH₂COOH); 2.17 (1H, m, CH₂CH₂CH₂CH₂COOH); 2.38 (4H, s, 5-CH₃, CH₂CH₂CH₂CH₂COOH); 2.59 (2H, m, CH₂CH₂CH₂CH₂SO₃); 4.1 (4H, m, CH₂CH₃, CH₂CH₂CH₂CH₂SO₃); 6.21 (1H, d, J=13.5, α'-CH); 6.43 (1H, d, J=13.5, α-CH); 6.56 (1H, t, J=12.0, γ-CH); 7.2-7.77 (6H, m, ArH); 8.29 (2H, t, J=13, β,β'-CH).

### Example 56. Preparation of N,N'-di(4-sulfobutyl)-3-(4-carboxybutyl)-3,3',3',5,5'-pentamethylindodicarbocyanine, sodium salt (compound A19)

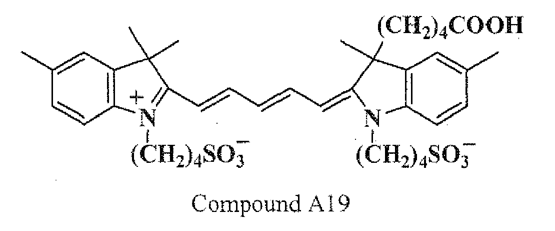

*N*,*N'*-Di(4-sulfobutyl)-3-(4-carboxybutyl)-3,3',3',5,5'-pentamethylindodicarbocyanine, sodium salt (compound A19) was prepared following the synthesis of 3-(4-carboxybutyl)-*N*-(4-sulfobutyl)-3,3',3'-trimethyl-*N'*-ethylindodicarbocyanine (compound A1) using the reagents ratio as follows: (1^{st} step) *N*-(4-sulfobutyl)-2,3,3.5-tetramethylindolenyne (61.9 mg; 0.2 mmole) (compound 30) (62 mg; 0.2 mmole), malonic dialdehyde dianil, hydrochloride (compound 37) (51.8 mg; 0.2 mmole), acetic anhydride (700 µL), and acetic acid (200 µL); (2^{nd} step) acetic anhydride (1.7 mL), 3-(4-carboxybutyl)-*N*-(4-sulfobutyl)-2,3,5-trimethylindolenyne (compound 23) (80.1 mg; 0.21 mmole), and a mixture of diisopropylethyl amine (200 µL; 269.5 mg; 2.1 mmole) and acetic anhydride (800 µL; 739.4 mg; 7.2 mmole). *N,N'*-Di(4-sulfobutyl)-3-(4-carboxybutyl)-3,3',3',5,5'-pentamethylindodicarbocyanine, sodium salt (compound A19) was obtained with yield of 34.6 mg (22.6%). For spectral characteristics see Table. Mass-spectrum (MALDI) (C₃₉H₅₁N₂O₈S₂⁻): found m/z 741.0, calculated m/z 739.96.

¹H-NMR (DMSO-d6), δ (ppm): 0.48 (1H, m, CH₂CH₂CH₂CH₂COOH); 0.76 (1H, m, CH₂CH₂CH₂CH₂COOH); 1.32 (2H, m, CH₂CH₂CH₂CH₂COOH); 1.63 (17H, m, 3,3',3'-CH₃, CH₂CH₂CH₂CH₂SO₃); 1.95 (2H, m, CH₂CH₂CH₂CH₂COOH); 2.13 (1H, m, CH₂CH₂CH₂CH₂COOH); 2.36 (6H, s, 5.5'-CH₃); 2.41 (9H, m, CH₂CH₂CH₂CH₂COOH, CH₂CH₂CH₂CH₂SO₃); 4.07 (4H, m, CH₂CH₂CH₂CH₂SO₃); 6.32 (2H, m, α,α'-CH); 6.54 (1H, t, J=12.0, γ-CH); 7.18-7.42 (6H, m, ArH); 8.26 (2H, m, β,β'-CH)

### Example 57. Preparation of 3.3',3',5,3',7'-hexamethyl-N,N'-di(4-sulfobutyl)-3-(4-carboxybutyl)-indodicarbocyanine, sodium salt (compound A20)

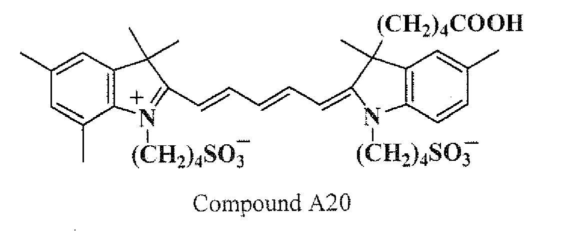

3,3',3',5,5',7'-Hexamethyl-*N*,*N'*-di(4-sulfobutyl)-3-(4-carboxybutyl)-indodicarbocyanine, sodium salt (compound A20) was prepared following the synthesis of *N,N'*-diethyl-3-(4-carboxybutyl)-5-sulfo-3,3',3'-trimethylindodicarbocyanine (compound A9) using the reagents ratio as follows: (1^{st} step) 2,3,3,5,7-pentamethyl-*N*-(4-sulfobutyl)-indolenyne (compound 30) (64.7 mg; 0.2 mmole), malonic dialdehyde dianil, hydrochloride (compound 35) (51.8 mg; 0.2 mmole), 700 µL acetic anhydride, and 200 µL acetic acid; (2^{nd} step) 3-(4-carboxybutyl)-*N-*(4-sulfobutyl)-2,3,5-trimethyl-indolenyne (compound 22) (84 mg; 0.21 mmole), anhydrous potassium acetate (200 mg; 2 mmole), acetic anhydride (700 µL), and acetic acid (200 µL). 3,3',3',5,5',7'-Hexamethyl-*N*,*N'*-di(4-sulfobutyl)-3-(4-carboxybutyl)indodicarbocyanine, sodium salt (compound A20) was obtained with yield of 7 mg (4.5%). For spectral characteristics see Table. Mass-spectrum (MALDI) (C₄₀H₅₃N₂O₈S₂⁻): found m/z 755.4, calculated m/z 753.99.

¹H-NMR (DMSO-d6), δ (ppm): 0.49 (1H, m, CH₂CH₂CH₂CH₂COOH); 0.78 (1H, m, CH₂CH₂CH₂CH₂COOH); 1.31 (2H, m, CH₂CH₂CH₂CH₂COOH); 1.65-1.79 (17H, m, 3,3',3'-CH₃, CH₂CH₂CH₂CH₂SO₃); 2.07 (2H, m, CH₂CH₂CH₂CH₂COOH); 2.19 (1H, m, CH₂CH₂CH₂CH₂COOH); 2.37 (7H, s, 5.5'-CH₃, CH₂CH₂CH₂CH₂COOH); 2.47 (4H, m, CH₂CH₂CH₂CH₂SO₃); 2.6 (3H, s, 7'-CH₃); 4.1 (4H, m, CH₂CH₂CH₂CH₂SO₃); 6.39 (2H, t, J=14, α,α'-CH); 6.6 (1H, t, J=12.5, γ-CH); 7.24-7.54 (5H, m, ArH); 8.32 (2H, m, β,β'-CH)

### Example 58. Preparation of 3-(4-carboxybutyl)-N'-(4-sulfobutyl)-5-sulfo-3,3',3'-trimethyl-N-ethylindodicarbocyanine, sodium salt (compound A21)

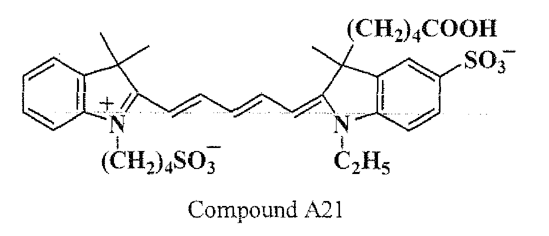

3-(4-Carboxybutyl)-*N*'-(4-sulfobutyl)-5-sulfo-3,3',3'-trimethyl-*N*-ethylindodicarbocyanine, sodium salt (compound A21) was prepared following the synthesis of *N*,*N'*-diethyl-3-(4-carboxybutyl)-5-sulfo-3,3',3'-trimethylindodicarbocyanine (compound A9) using the reagents ratio as follows: (1^{st} step) 2,3'-dimethyl-3-(4-carboxybutyl)-5-sulfo-*N*-ethylindolenyne (compound 24) (70.7 mg; 0.2 mmole), malonic dialdehyde dianil, hydrochloride (compound 37) (51.8 mg; 0.2 mmole), acetic anhydride (700 µL), and acetic acid (350 µL); (2^{nd} step) *N*-(4-sutfobutyl)-2,3,3-trimethylindolenyne (compound 26) (62.0 mg; 0.21 mmole), anhydrous potassium acetate (196 mg; 2 mmole), acetic anhydride (700 µL), and acetic acid (350 µL). 3-(4-Carboxybutyl)-*N'-*(4-sulfobutyl)-5-sulfo-3,3',3'-trimethyl-*N*-ethylindodicarbocyanine, sodium salt (compound A21) was obtained with yield of 28.5 mg (20.2%). For spectral characteristics see Table. Mass-spectrum (MALDI) (C₃₅H₄₃N₂O₈S₂⁻): found m/z 684.2, calculated m/z 683.86.

¹H-NMR (DMSO-d6), δ (ppm): 0.5 (1H, m, CH₂CH₂CH₂CH₂COOH); 0.77 (1H, m, CH₂CH₂CH₂CH₂COOH); 1.21 (3H, t, CH₂CH₃); 1.34 (2H, m, CH₂CH₂CH₂CH₂COOH); 1.66-1.79 (13H, m, 3,3',3'-CH₃, CH₂CH₂CH₂CH₂SO₃); 2.02 (2H, m, CH₂CH₂CH₂CH₂COOH); 2.19 (1H, m, CH₂CH₂CH₂COOH); 2.43 (3H, m, CH₂CH₂CH₂CH₂COOH, CH₂CH₂CH₂CH₂SO₃); 4.12 (4H, m, CH₂CH₃, CH₂CH₂CH₂CH₂SO₃); 6.3 (1H, d, J=13.5, α'-CH); 6.4 (1H, d, J=13.5, α-CH); 6.58 (1H, t, J=12.0, γ-CH); 7.24-7.75 (7H, m, ArH); 8.35 (2H, m, β,β'-CH)

### Example 59. Preparation of 5,5'-disulfo-N,N'-diethyl-3-(4-carboxybutyl)-3,3',3'-trimethylindodicarbocyanine, sodium salt (compound A22)

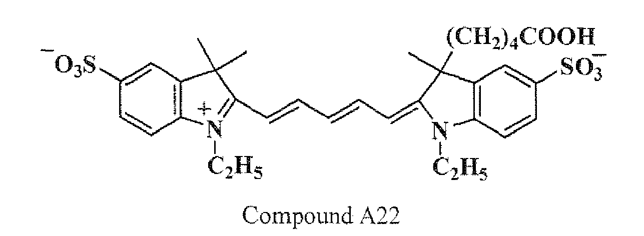

5,5'-Disulfo-*N,N'*-diethyl-3-(4-carboxybutyl)-3,3',3'-trimethylindodicarbocyanine, sodium salt (compound A22) was prepared following the synthesis of *N,N'*-diethyl-3-(4-carboxybutyl)-5-sulfo-3,3',3'-trimethylindodicarbocyanine (compound A9) using the reagents ratio as follows: (1^{st} step) 2,3-dimethyl-3-(4-carboxybutyl)-5-sulfo-*N*-ethylindolenyne (compound 24) (70.7 mg; 0.2 mmole), malonic dialdehyde dianil, hydrochloride (compound 37) (51.8 mg; 0.2 mmole), acetic anhydride (700 µL), and acetic acid (350 µL); (2^{nd} step) 5-sulfo-2,3,3-trimethyl-*N-*ethylindolenyne (compound 29) (56.1 mg; 0.21 mmole), anhydrous potassium acetate (196 mg; 2 mmole), acetic anhydride (700 µL), and acetic acid (350 µL). 5,5'-Disulfo-*N,N'*-diethyl-3-(4-carboxybutyl)-3,3',3'-trimethylindodicarbocyanine, sodium salt (compound A22) was obtained with yield of 18.3 mg (13.5%). For spectral characteristics see Table. Mass-spectrum (MALDI) (C₃₃H₃₉N₂O₈S₂⁻): found m/z 656.5, calculated m/z 655.80.

¹H-NMR (DMSO-d6), δ (ppm): 0.51 (1H, m, CH₂CH₂CH₂CH₂COOH); 0.75 (1H, m, CH₂CH₂CH₂CH₂COOH); 1.22-1.38 (8H, m, CH₂CH₃, CH₂CH₂CH₂CH₂COOH); 1.69 (9H, s, 3,3',3'-CH₃); 2.04 (2H, m, CH₂CH₂CH₂CH₂COOH); 2.21 (1H, m, CH₂CH₂CH₂CH₂COOH); 2.43 (1H, m, CH₂CH₂CH₂CH₂COOH); 4.14 (4H, m, CH₂CH₃); 6.33 (2H, m, α,α'-CH); 6.57 (1H, t, J=12.0, γ-CH); 7.28-7.82 (6H, m, ArH); 8.35 (2H, m, β,β'-CH); 11.88 (1H, s, COOH)

### Example 60. Preparation of N,N'-di(4-sulfobutyl)-3-(4-carboxybutyl)-5'-sulfo-3,3',3'-trimethylindodicarbocyanine, disodium salt (compound A23)

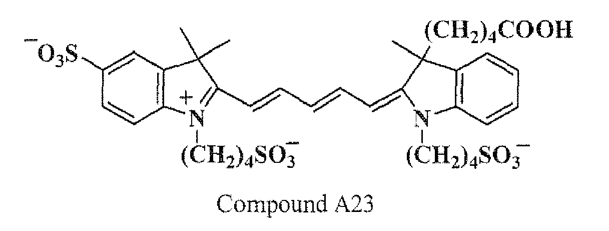

*N,N'*-Di(4-sulfobutyl)-3-(4-carboxybutyl)-5'-sulfo-3,3',3'-trimethylindodicarbocyanine, disodium salt (compound A23) was prepared following the synthesis of *N*,*N'*-diethyl-3-(4-carboxybutyl)-5-sulfo-3,3',3'-trimethylindodicarbocyanine (compound A9) using the reagents ratio as follows: (1^{st} step) 5-sulfo-*N*-(4-sulfobutyl)-2,3,3-trimethylindolenyne (compound 28) (82.7 mg; 0.2 mole), malonic dialdehyde dianil, hydrochloride (compound 37) (51.8 mg; 0.2 mmole), acetic anhydride (700 µL), and acetic acid (350 µL); (2^{nd} step) 2,3-dimethyl-3-(4-carboxybutyl)-*N*-(4-sulfobutyl)-indolenyne (compound 22) (80.1 mg; 0.21 mmole), anhydrous potassium acetate (117 mg; 1.2 mole), acetic anhydride (700 µL), and acetic acid (700 µL). *N,N'*-Di(4-sulfobutyl)-3-(4-carboxybutyl)-5'-sulfo-3,3',3'-trimethylindodicarbocyanine, disodium salt (compound A23) was obtained with yield of 53.8 mg (32.1%). For spectral characteristics see Table. Mass-spectrum (MALDI) (C₃₇H₄₆N₂O₁₁S₃²⁻): found m/z 789.6, calculated m/z 790.97.

¹H-NMR (DMSO-d6), δ (ppm): 0.46 (1H, m, CH₂CH₂CH₂CH₂COOH); 0.78 (1H, m, CH₂CH₂CH₂CH₂COOH); 1.33 (2H, m, CH₂CH₂CH₂CH₂COOH); 1.65-1.75 (17H, m, 3,3',3'-CH₃, CH₂CH₂CH₂CH₂SO₃); 2.02 (2H, m, CH₂CH₂CH₂CH₂COOH); 2.16 (1H, m, CH₂CH₂CH₂CH₂COOH); 2.39 (5H, m, CH₂CH₂CH₂CH₂COOH, CH₂CH₂CH₂CH₂SO₃); 4.09 (4H, m, CH₂CH₂CH₂CH₂SO₃); 6.38 (2H, m, α,α'-CH); 6.59 (1H, t, J=12.0, γ-CH); 7.22-7.79 (7H, m, ArH); 8.32 (2H, m, β,β'-CH); 11.9 (1H, s, COOH)

### Example 61. Preparation of N,N'-di(4-sulfobutyl)-3-(4-carboxybutyl)-5-sulfo-3,3',3'-trimethylindodicarbocyanine, disodium salt (compound A24)

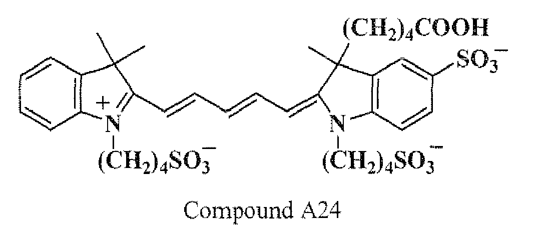

*N*,*N'*-Di(4-sulfobutyl)-3-(4-caxboxybutyl)-5-sulfo-3,3',3'-trimethylindodicarbocyanine, disodium salt (compound A24) was prepared following the synthesis of *N*,*N'*-diethyl-3-(4-carboxybutyl)-5-sulfo-3,3',3'-trimethylindodicarbocyanine (compound A9) using the reagents ratio as follows: (1^{st} step) 2,3-dimethyl-3-(4-carboxybutyl)-*N*-(4-sulfobutyl)-5-sulfoindolenyne, potassium salt (compound 25) (99.9 mg; 0.2 mmole), malonic dialdehyde dianil, hydrochloride (compound 37) (51.8 mg; 0.2 mmole), acetic anhydride (700 µL), and acetic acid (350 µL); (2^{nd} step) N-(4-sulfobutyl)-2,3,3-trimethylindolenyne (compound 26) (62 mg; 0.21 mmole), anhydrous potassium acetate (118 mg; 1.2 mmole), acetic anhydride (350 µL), and acetic acid (700 µL). *N*,*N'*-Di(4-sulfobutyl)-3-(4-carboxybutyl)-5-sulfo-3,3',3'-trimethylindodicarbocyanine, disodium salt (compound A24) was obtained with yield of 40.5 mg (24.1%). For spectral characteristics see Table. Mass-spectrum (MALDI) (C₃₇H₄₆N₂O₁₁S₃²⁻): found m/z 790.5, calculated m/z 790.97.

¹H-NMR (DMSO-d6), δ (ppm): 0.52 (1H, m, CH₂CH₂CH₂CH₂COOH); 0.78 (1H, m, CH₂CH₂CH₂CH₂COOH); 1.38 (2H, m, CH₂CH₂CH₂CH₂COOH); 1.68-1.79 (21H, m, 3,3',3'-CH₃, CH₂CH₂CH₂CH₂SO₃); 1.91 (1H, m, CH₂CH₂CH₂CH₂COOH); 2.15 (2H, m, CH₂CH₂CH₂CH₂COOH); 2.39 (1H, m, CH₂CH₂CH₂CH₂COOH); 4.09 (4H, m, CH₂CH₂CH₂CH₂SO₃); 6.37 (2H, m, α,α'-CH); 6.63 (1H, m, γ-CH); 7.22-7.78 (7H, m, ArH); 8.31 (2H, m, β,β'-CH).

### Example 62. Preparation of 5,5'-disulfo-3-(4-carboxybutyl)-N-(4-sulfobutyl)-3,3',3'-trimethyl-N'-ethylindodicarbocyanine, disodium salt (compound A25)

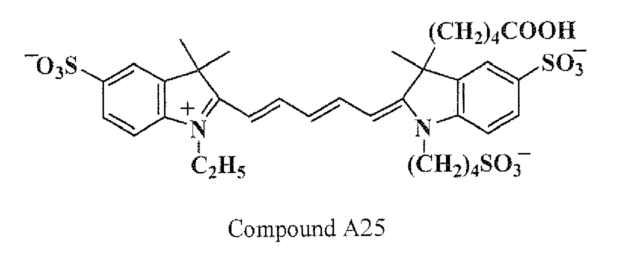

5,5'-Disulfo-3-(4-carboxybutyl)-*N*-(4-sulfobutyl)-3,3',3'-trimethyl-*N'*-ethylindodicarbocyanine, disodium salt (compound A25) was prepared following the synthesis of *N*,*N'*-diethyl-3-(4-carboxybutyl)-5-sulfo-3,3',3'-trimethylindodicarbocyaninea (compound A9) using the reagents ratio as follows: (1^{st} step) 2,3-dimethyl-3-(4-carboxybutyl)-*N*-(4-sulfobutyl)-5-sulfloindolenyne, potassium salt (compound 25) (99.9 mg; 0.2 mmole), malonic dialdehyde dianil, hydrochloride (compound 37) (51.8 mg; 0.2 mmole), acetic anhydride (700 µL), and acetic acid (350 µL); (2^{nd} step) 5-sulfo-2,3,3-trimethyl-*N*-ethylindolenyne (compound 29) (56.1 mg; 0.21 mmole), anhydrous potassium acetate (176 mg; 1.8 mmole), acetic anhydride (350 µL), and acetic acid (700 µL). 5,5'-Disulfo-3-(4-carboxybutyl)-*N*-(4-sulfobutyl)-3,3',3'-trimethyl-*N'*-ethylindodicarbo cyanine, disodium salt (compound A25) was obtained with yield of 46.4 mg (29.5%). For spectral characteristics see Table. Mass-spectrum (MALDI) (C₃₅H₄₂N₂O₁₁S₃²⁻): found m/z 762.0, calculated m/z 762.91.

¹H-NMR (DMSO-d6), δ (ppm): 0.51 (1H, m, CH₂CH₂CH₂CH₂COOH); 0.76 (1H, m, CH₂CH₂CH₂CH₂COOH); 1.21-1.36 (5H, m, CH₂CH₃, CH₂CH₂CH₂CH₂COOH); 1.65-1.78 (13H, m, 3,3',3'-CH₃, CH₂CH₂CH₂CH₂SO₃); 2.13 (2H, m, CH₂CH₂CH₂CH₂COOH); 2.22 (1H, m, CH₂CH₂CH₂CH₂COOH); 2.37 (3H, m, CH₂CH₂CH₂CH₂COOH, CH₂CH₂CH₂CH₂SO₃); 4.12 (4H, m, CH₂CH₃, CH₂CH₂CH₂CH₂SO₃); 6.31 (1H, d, J=13.5, α'-CH); 6.42 (1H, d, J=13.5, α-CH); 6.6 (1H, t, J=12.0, γ-CH); 7.3-7.8 (6H, m, ArH); 8.35 (2H, m, β,β'-CH)

### Example 63. Preparation of N,N'-di(4-sulfobutyl)-3-(4-carboxybutyl)-5-sulfo-3,3',3',5'-tetramethylindodicarbocyanine, disodium salt (compound A26)

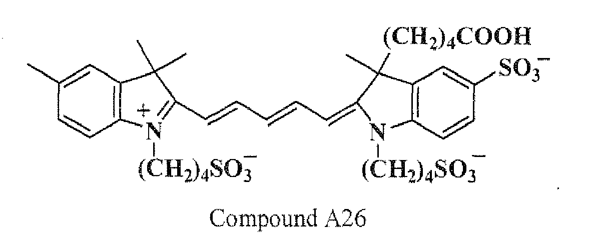

*N*,*N'*-di(4-Sulfobutyl)-3-(4-carboxybutyl)-5-sulfo-3,3',3',5'-tetramethylindodicarbocyanine, disodium salt (compound A26) was prepared following the synthesis of *N*,*N'*-diethyl-3-(4-carboxybutyl)-5-sulfo-3,3',3'-trimethylindodicarbocyanine (compound A9) using the reagents ratio as follows: (1^{st} step) 2,3-dimethyl-3-(4-carboxybutyl)-*N-*(4-sulfobutyl)-5-sulfoindolenyne, potassium salt (compound 25) (99.9 mg, 0.2 mmole), malonic dialdehyde dianil, hydrochloride (compound 37) (51.8 mg; 0.2 mmole), acetic anhydride (700 µL), and acetic acid (350 µL); (2^{nd} step) *N*-(4-sulfobutyl)-2,3,3.5-tetramethylindolenyne (compound 30) (80.1 mg; 0.21 mmole), anhydrous potassium acetate (176 mg, 1.8 mmole), acetic anhydride (300 µL), and acetic acid (700 µL). *N*,*N'*-Di(4-sulfobutyl)-3-(4-carboxybutyl)-5-sulfo-3,3',3,5-tetramethylindodicarbo cyanine, disodium salt (compound A26) was obtained with yield of 68.8 mg (40.4%). For spectral characteristics see Table. Mass-spectrum (MALDI) (C₃₈H₄₈N₂O₁₁S₂⁻): found m/z 804.2, calculated m/z 804.99.

¹H-NMR (DMSO-d6), δ (ppm): 0.52 (1H, m, CH₂CH₂CH₂CH₂COOH); 0.77 (1H, m, CH₂CH₂CH₂CH₂COOH); 1.39 (2H, m, CH₂CH₂CH₂CH₂COOH); 1.67 (18H, m, 3,3',3'-CH₃, CH₂CH₂CH₂CH₂SO₃, CH₂CH₂CH₂CH₂COOH); 2.17 (2H, m, CH₂CH₂CH₂CH₂COOH); 2.37 (8H, m, 5'-CH₃, CH₂CH₂CH₂CH₂COOH, CH₂CH₂CH₂CH₂SO₃); 4.09 (4H, m, CH₂CH₂CH₂CH₂SO₃); 6.38 (2H, m, α,α'-CH); 6.59 (1H, m, γ-CH); 7.2-7.72 (6H, m, ArH); 8.32 (2H, m, β,β'-CH)

### Example 64. Preparation of N,N'-di(4-sulfobutyl)-3-(4-carboxybutyl)-3,3',3',5',7'-pentamethyl-5-sulfoindodicarbocyanine, disodium salt (compound A27)

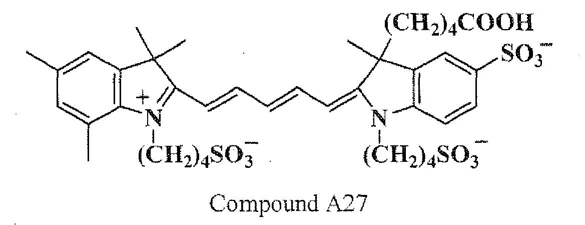

*N*,*N'*-Di(4-sulfobutyl)-3-(4-carboxybutyl)-3,3',3',5',7'-pentamethyl-5-sulfoindodicarbocyanine, disodium salt (compound A27) was prepared following the synthesis of *N*,*N'*-diethyl-3-(4-carboxybutyl)-5-sulfo-3,3',3'-trimethylindodicarbocyanine (compound A9) using the reagents ratio as follows: (1^{st} step) 2,3-dimethyl-3-(4-carboxybutyl)-*N*-(4-sulfobutyl)-5-sulfoindolenyne, potassium salt (compound 25) (99.9 mg; 0.2 mmole), malonic dialdehyde dianil, hydrochloride (compound 37) (51.8 mg; 0.2 mole), acetic anhydride (700 µL), and acetic acid (200 µL); (2^{nd} step) *N*-(4-sulfobutyl)-2,3,3,5,7-pentamethylindolenyne (compound 32) (64.7 mg; 0.2 mmole), anhydrous potassium acetate (98.1 mg; 1 mmole), acetic anhydride (700 µL), and acetic acid (700 µL). *N*,*N'*-Di(4-sulfobutyl)-3-(4-carboxybutyl)-3,3',3',5',7'-pentamethyl-5-sulfoindodicarbo cyanine, disodium salt (compound A27) was obtained with yield of 7 mg (4%). For spectral characteristics see Table. Mass-spectrum (MALDI) (C₃₉H₅₀N₂O₁₁S₃²⁻): found m/z 820.5, calculated m/z 819.02.

¹H-NMR (DMSO-d6), δ (ppm): 0.48 (1H, m, CH₂CH₂CH₂CH₂COOH); 0.77 (1H, m, CH₂CH₂CH₂CH₂COOH); 1.3 (2H, m, CH₂CH₂CH₂CH₂COOH); 1.68-1.83 (17H, m, 3,3',3'-CH₃, CH₂CH₂CH₂CH₂SO₃); 2.12 (2H, m, CH₂CH₂CH₂CH₂COOH); 2.21 (1H, m, CH₂CH₂CH₂CH₂COOH); 2.4 (4H, s, 5'-CH₃, CH₂CH₂CH₂CH₂COOH); 2.48 (4H, m, CH₂CH₂CH₂CH₂SO₃); 2.62 (3H, s, 7'-CH₃); 4.12 (4H, m, CH₂CH₂CH₂CH₂SO₃); 6.41 (2H, t, J=14, α,α'-CH); 6.58 (1H, t, J=12.5, γ-CH); 7.2-7.56 (5H, m, ArH); 8.35 (2H, m, β,β'-CH)

### Example 65. Preparation of N,N'-di(4-sulfobutyl)-3-(4-carboxybutyl)-5'-sulfo-3,3',3',5-tetramethylindodicarbocyanine, disodium salt (compound A28)

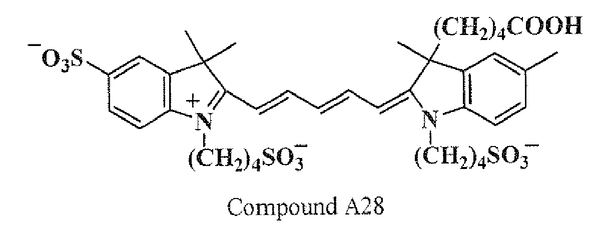

*N*,*N'*-Di(4-sulfobutyl)-3-(4-carboxybutyl)-5'-sulfo-3,3',3'.5-tetramethylindodicarbocyanine, disodium salt (compound A28) was prepared following the synthesis of *N,N'*-diethyl-3-(4-carboxybutyl)-5-sulfo-3,3',3'-trimethylindodicarbocyanine (compound A9) using the reagents ratio as follows: (1^{st} step) 5-sulfo-*N*-(4-sulfobutyl)-2,3,3-trimethylindolenyne (compound 28) (82.7 mg; 0.2 mmole), malonic dialdehyde dianil, hydrochloride (compound 37) (51.8 mg; 0.2 mmole), acetic anhydride (700 µL), and acetic acid (200 µL); (2^{nd} step) 3-(4-carboxybutyl)-*N*-(4-sulfobutyl)-2,3,5-trimethylindolenyne (compound 23) (84 mg; 0.21 mmole), anhydrous potassium acetate (350 mg; 3.6 mmole), acetic anhydride (500 µL), and acetic acid (700 µL). *N*,*N'*-Di(4-sulfobutyl)-3-(4-carboxybutyl)-5'-sulfo-3,3',3'.5-tetramethylindodicarbocyanine, disodium salt (compound A28) was obtained with yield of 85.6 mg (50.3%). For spectral characteristics see Table. Mass-spectrum (MALDI) (C₃₈H₄₈N₂O₁₁S₃²⁻): found m/z 805.5, calculated m/z 804.99.

¹H-NMR (DMSO-d6), δ (ppm): 0.45 (1H, m, CH₂CH₂CH₂CH2COOH); 0.77 (1H, m, CH₂CH₂CH₂CH₂COOH); 1.32 (2H, m, CH₂CH₂CH₂CH₂COOH); 1.67 (17H, m, 3,3',3'-CH₃, CH₂CH₂CH₂CH₂SO₃); 1.92 (1H, m, CH₂CH₂CH₂CH₂COOH); 2.17 (2H, m, CH₂CH₂CH₂CH₂COOH); 2.37 (8H, m, 5-CH₃, CH₂CH₂CH₂CH₂COOH, CH₂CH₂CH₂CH₂SO₃); 4.1 (4H, m, CH₂CH₂CH₂CH₂SO₃); 6.27 (1H, d, J=13.5, α'-CH); 6.41 (1H, d, J=13.5, α-CH); 6.58 (1H, t, J=12.0, γ-CH); 7.2-7.76 (6H, m, ArH); 8.28 (2H, m, β,β'-CH)

### Example 66. Preparation of 5,5'-disulfo-3-(4-carboxybutyl)-N'-(4-sulfobutyl)-3,3';3'-trimethyl-N-ethylindodicarbocyanine, disodium salt (compound A29)

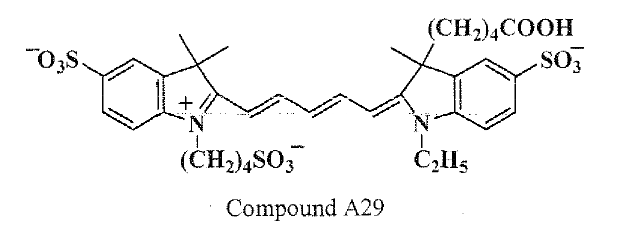

5,5'-Disulfo-3-(4-carboxybutyl)-*N*'-(4-sulflobutyl)-3,3',3'-trimethyl-*N*-ethylindodicarbocyanine, sodium salt (compound A29) was prepared following the synthesis of *N*,*N'*-diethyl-3-(4-carboxybutyl)-5-sulfo-3,3',3'-trimethylindodicarbocyanine (compound A9) using the reagents ratio as follows: (1^{st} step) 2,3-dimethyl-3-(4-carboxybutyl)-5-sulfo-*N*-ethylindolenyne (compound 24) (70.7 mg; 0.2 mmole), malonic dialdehyde dianil, hydrochloride (compound 37) (51.8 mg; 0.2 mmole), acetic anhydride (700 µL), and acetic acid (350 µL); (2^{nd} step) 5-sulfo-*N-*(4-sulfobutyl)-2,3,3-trimethylindolenyne (compound 28) (86.8 mg; 0.21 mmole), anhydrous potassium acetate (294 mg; 3 mmole), acetic anhydride (700 µL), and acetic acid (350 µL). 5,5'-Disulfo-3-(4-carboxybutyl)-*N'*-(4-sulfobutyl)-3,3',3'-trimethyl-*N*-ethylindodicarbocyanine, sodium salt (compound A29) was obtained with yield of 29.7 mg (18.8%). For spectral characteristics see Table. Mass-spectrum (MALDI) (C₃₅H₄₂N₂O₁₁S₃²⁻): found m/z 763.2, calculated m/z 762.91.

¹H-NMR (DMSO-d6), δ (ppm): 0.52 (1H, m, CH₂CH₂CH₂CH₂COOH); 0.73 (1H, m, CH₂CH₂CH₂CH₂COOH); 1.22-1.35 (5H, m, CH₂CH₃, CH₂CH₂CH₂CH₂COOH); 1.68 (15H, m, 3,3',3'-CH₃, CH₂CH₂CH₂CH₂SO₃); 2.02 (2H, m, CH₂CH₂CH₂CH₂COOH); 2.18 (1H, m, CH₂CH₂CH₂CH₂COOH); 2.37 (1H, m, CH₂CH₂CH₂CH₂COOH); 4.12 (4H, m, CH₂CH₃, CH₂CH₂CH₂CH₂SO₃); 6.36 (2H, m, α,α'-CH), 6.59 (1H, m, γ-CH); 7.0-7.79 (6H, m, ArH); 8.35 (2H, m, β,β'-CH)

### Example 67. Preparation of 5,5'-disulfo-N,N'-di(4-sulfobutyl)-3-(4-carboxybutyl)-3,3',3'-trimethylindodicarbocyanine, trisodium salt (compound A30)

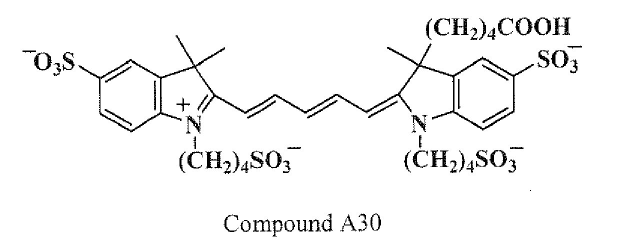

5,5'-Disulfo-*N*,*N'*-di(4-sulfobutyl)-3-(4-carboxybutyl)-3,3',3'-trimethylindodicarbocyanine, trisodium salt (compound A30) was prepared following the synthesis of *N*,*N'*-diethyl-3-(4-carboxybutyl)-5-sulfo-3,3',3'-trimethylindodicarbocyanine (compound A9) using the reagents ratio as follows: (1^{st} step) 2,3-dimethyl-3-(4-carboxybutyl)-*N*-(4-sulfobutyl)-5-sulfoindolenyne, potassium salt (compound 25) (99.9 mg; 0.2 mmole), malonic dialdehyde dianil, hydrochloride (compound 37) (51.8 mg; 0,2 mmole), acetic anhydride (700 µL), and acetic acid (350 µL); (2^{nd} step) 5-sulfo-*N*-(4-sulfobutyl)-2,3,3-trimethylindolenyne (compound 28) (86.8 mg; 0.21 mmole), anhydrous potassium acetate (236 mg; 2.4 mmole), acetic anhydride (350 µL), and acetic acid (700 µL). 5,5'-Disulfo-*N*,*N'*-di(4-sulfobutyl)-3-(4-carboxybutyl)-3,3',3'-trimethylindodicarbocyanine, trisodium salt (compound A30) was obtained with yield of 77.8 mg (41.4%). For spectral characteristics see Table. Mass-spectrum (MALDI) (C₃₇H₄₅N₂O₁₄S₄³⁻): found m/z 870.0, calculated m/z 870.02.

¹H-NMR (DMSO-d6), δ (ppm): 0.53 (1H, m, CH₂CH₂CH₂CH₂COOH); 0.78 (1H, m, CH₂CH₂CH₂CH₂COOH); 1.39 (2H, m, CH₂CH₂CH₂CH₂COOH); 1.67-1.81 (21H, m, 3,3',3'-CH₃, CH₂CH₂CH₂CH₂SO₃); 1.92 (1H, m, CH₂CH₂CH₂CH₂COOH); 2.17 (2H, m, CH₂CH₂CH₂CH₂COOH); 2.41 (1H, m, CH₂CH₂CH₂CH₂COOH); 4.12 (4H, m, CH₂CH₂CH₂CH₂SO₃); 6.39 (2H, m, α,α'-CH); 6.64 (1H, m, γ-CH); 7.25-7.81 (6H, m, ArH); 8.33 (2H, m, β,β'-CH)

### Example 68. Preparation of 5-carboxyethyl-N-sulfobutyl-3.3,3',3'-tetramethyl-N'-ethylindodicarbocyanine (compound B1)

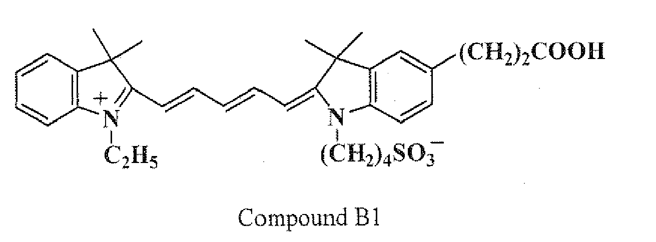

5-Carboxyethyl-*N*-sulfobutyl-3.3,3',3'-tetramethyl-*N'*-ethylindodicarbocyanine (compound B1) was prepared following the synthesis of 3-(4-carboxybutyl)-*N*-(4-sulfobutyl),3,3',3'-trimethyl-*N*'-ethylindodicarbocyanine (compound A1) using the reagents ratio as follows: (1^{st} step) 2,3,3-trimethyl-*N*-ethylindolenyneum iodide (compound 27) (53.5 mg; 0.2 mmole), malonic dialdehyde dianil, hydrochloride (compound 37) 58.8 mg; 0.23 mmole), acetic anhydride (1 mL), and acetyl chloride (64 µL); (2^{nd} step) acetic anhydride (2 mL), 5-carboxyethyl-*N-*sulfobutyl-2,3,3-trimethylindolenyne (compound 34) (80.7 mg; 0.22 mmole), and a mixture (545 µL) of diisopropylethyl amine (200 µL; 269.5 mg; 2.1 mmole) and acetic anhydride (800 µL; 739.4 mg; 7.2 mmole). 5-Carboxyethyl-*N*-sulfobutyl-3.3,3',3'-tetramethyl-*N*'-ethylindodicarbocyanine (compound B1) was obtained with yield of 57 mg (48%). For spectral characteristics see Table. Mass-spectrum (MALDI) (C₃₄H₄₂N₂O₅S): found m/z 591.0, calculated m/z 590.77.

¹H-NMR (DMSO-d6), δ (ppm): 1.26 (3H, t, J=7.0, CH₂CH₃), 1.63 (12H, s, C(CH₃)₂), 1.73 (4H, m, CH₂CH₂CH₂CH₂SO₃), 2.48 (2H, m, CH₂CH₂CH₂CH₂SO₃), 2.58 (2H, t, J=7.5, CH₂CH₂COOH), 2.88 (2H, t, J=7.5, CH₂CH₂COOH), 4.10 (4H, m, NCH₂), 6.37 (2H, d, J=13.5, a,a' -CH), 6.56 (1H, t, J=12.0, γ-CH), 7.19-7.63 (6H, m, ArH), 8.31 (2H, m, β,β'-CH)

### Example 69. Preparation of N,N'-diethyl-5-carboxyethyl-5'-sulfo-3.3,3',3'-tetramethylindodicarbocyanine (compound B2)

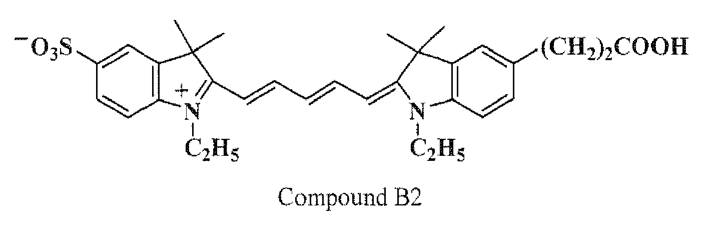

*N,N*'-Diethyl-5-carboxyethyl-5'-sulfoindodicarbocyanine (compound B2) was prepared following the synthesis of *N,N*'-diethyl-3-(4-carboxybutyl)-5-sulfo-3,3',3',-trimethylindodicarbo cyanine (compound A9) using the reagents ratio as follows: (1^{st} step) 5-sulfo-2,3,3-trimethyl-*N-*ethylindolenyne (compound 29) (53.3 mg; 0.2 mmole), malonic dialdehyde dianil, hydrochloride (compound 37) (58.7 mg; 0.23 mmole), acetic anhydride (1.5 mL), and acetic acid (0.5 mL); (2^{nd} step) 5-carboxyethyl-2,3,3-trimethyl-*N*-ethylindolenyneum iodide (compound 35) (85.0 mg; 0.22 mmole), anhydrous potassium acetate (120 mg; 1.2 mmole), acetic anhydride (1.5 mL), and acetic acid (0.5 mL). *N,N*'-diethyl-5-carboxyethyl-5'-sulfo-3.3,3',3'-tetramethylindodicarbo cyanine (compound B2) was obtained with yield of 60 mg (53%). For spectral characteristics see Table. Mass-spectrum (MALDI) (C₃₂H₃₈N₂O₅S): found m/z 563.2, calculated m/z 562.72.

¹H-NMR (DMSO-d6), δ (ppm): 1.25 (6H, t, J=7.0, CH₂CH₃), 1.69 (2H, s, 1C(CH₃)₂), 2.32 (2H, t, J=7.5, CH₂CH₂COOH), 2.86 (2H, t, J=7.5, CH₂CH₂COOH), 4.95 (4H, m, NCH₂), 6.30 (2H, d, J=13.5, α,α'-CH), 6.58 (1H, t, J=12.0, γ-CH), 7.21-7.63 (6H, m, ArH), 8.31 (2H, m, β,β'-CH)

### Example 70. Preparation of 5-carboxyethyl-N'-sulfobutyl-3.3,3',3'-tetramethyl-N-ethylindodicarbocyanine (compound B3)

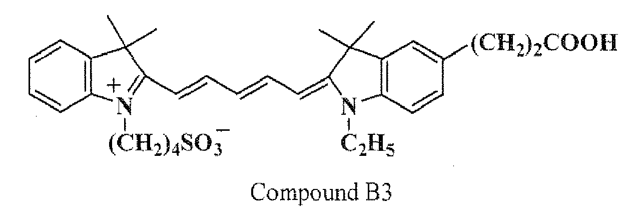

5-Carboxyethyl-*N*'-sulfobutyl-3,3,3',3'-tetramethyl-*N*-ethylindodicarbocyanine (compound B3) was prepared following the synthesis of *N,N*'-diethyl-3-(4-carboxybutyl)-5-sulfo-3,3',3',-trimethylindodicarbocyanine (compound A9) using the reagents ratio as follows: (1^{st} step) *N*-(4-sulfobutyl)-2,3,3-trimethylindolenyne (compound 26) (59.1 mg; 0.2 mmole), malonic dialdehyde dianil, hydrochloride (compound 37) (58.7 mg; 0.23 mmole), acetic anhydride (1.5 mL), and acetic acid (0.5 mL); (2^{nd} step) 5-carboxyethyl-2,3,3-trimethyl-*N*-ethylindolenyneum iodide (compound 35) (85.0 mg, 0.22 mmole), anhydrous potassium acetate (120 mg; 1.2 mmole), acetic anhydride (1.5 mL), and acetic acid (0.5 mL). 5-Carboxyethyl-*N*'-sulfobutyl-3,3,3',3'-tetramethyl-*N*-ethylindodicarbocyanine (compound B3) was obtained with yield of 59 mg (50%). For spectral characteristics see Table. Mass-spectrum (MALDI) (C₃₄H₄₂N₂O₅S): found m/z 592.8, calculated m/z 590.77.

¹H-NMR (DMSO-d6), δ (ppm): 1.28 (3H, s, CH₂CH₃), 1.67 (12H, s, C(CH₃)₂), 1.74 (4H, m, CH₂CH₂CH₂CH₂SO₃), 2.27 (2H, t, J=7.5, CH₂CH₂COOH), 2.48 (2H, m, CH₂CH₂CH₂CH₂SO₃), 2.82 (2H, t, J=7.5, CH₂CH₂COOH), 4.13 (4H, m, NCH₂), 6.30 (2H, d, J=13.5, α,α' -CH), 6.56 (1H, t, J-12.0, γ-CH), 7.24-7.58 (7H, m, ArH), 8.28 (2H, m, β,β'-CH).

### Example 71. Preparation of N,N'-disulfobutyl-5-carboxyethyl-3,3,3',3',5'-pentamethylindodicarbocyanine, sodium salt (compound B4)

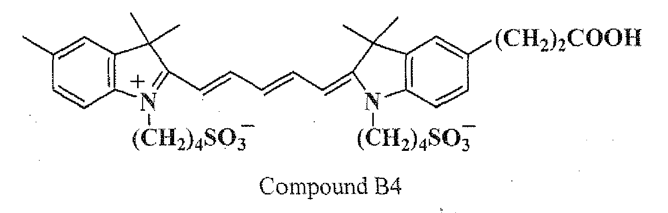

*N,N*'-Disulfobutyl-5-carboxyethyl-3,3,3',3',5'-pentamethylindodicarbocyanine, sodium salt (compound B4) was prepared following the synthesis of 3-(4-carboxybutyl)-*N*-(4-sulfobutyl)-3,3',3'-trimethyl-*N*'-ethylindodicarbocyanine (compound A1) using the reagents ratio as follows: (1^{st} step) *N*-(4-sulfobutyl)-2,3,3.5-tetramethylindolenyne (61.9 mg; 0.2 mmole) (compound 30), malonic dialdehyde dianil, hydrochloride (compound 37) (58.8 mg; 0.23 mmole), acetic anhydride (1 mL), and acetyl chloride (64 µL); (2^{nd} step) 3-(4-carboxybutyl)-*N*-(4-sulfobutyl)-2,3,5-trimethylindolenyne (compound 34) (80.7 mg; 0.22 mmole), acetic anhydride (2 mL), and a mixture (545 µL) of diisopropylethyl amine (200 µL; 269.5 mg; 2.1 mmole) and acetic anhydride (800 µL; 739.4 mg; 7.2 mmole). *N,N*'-Disulfobutyl-5-carboxyethyl-3,3,3',3',5-pentamethylindodicarbocyanine, sodium salt (compound B4) was obtained with yield of 81 mg (55%). For spectral characteristics see Table. Mass-spectrum (MALDI) (C₃₇H₄₇N₂O₈S₂⁻): found m/z 713.0, calculated m/z 711.91.

¹H-NMR (DMSO-d6), δ (ppm): 1.66 (12H, s, C(CH₃)₂), 1.74 (8H, m, CH₂CH₂CH₂SO₃), 2.36 (3H, s, CH₃), 2.42 (4H, m, CH₂CH₂CH₂CH₂SO₃), 2.86(2H, t, J=7.5, CH₂CH₂COOH), 3.44 (2H, m, CH₂CH₂COOH), 4.06 (4H, s, NCH₂), 6.31 (2H, d, J=13.5, α,α'-CH), 6.56 (1H, t, J=12.0, γ-CH), 7.17-7.47 (6H, m, ArH), 8.26 (2H, t, β,β'-CH)

### Example 72. Preparation of N,N'-disulfobutyl-5-carboxyethyl-3,3,3',3'-tetramethylindodicarbocyanine, sodium salt (compound B5)

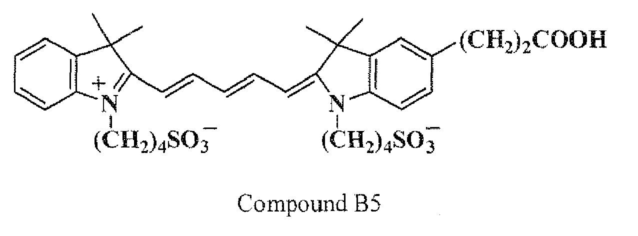

*N,N*'-Disulfobutyl-5-carboxyethyl-3,3,3',3'-tetramethylindodicarbocyanine, sodium salt (compound B5) was prepared following the synthesis of 3-(4-carboxybutyl)-*N*-(4-sulfobutyl)-3,3',3'-trimethyl-*N*'-ethylindodicarbocyanine (compound A1) using the reagents ratio as follows: (1^{st} step) *N*-(4-sulfobutyl)-2,3,3-trimethylindolenyne (compound 26) (59.1 mg; 0.2 mole), malonic dialdehyde dianil, hydrochloride (compound 37) (58.8 mg; 0.23 mmole), acetic anhydride (1 mL), and acetyl chloride (64 µL); (2^{nd} step) 3-(4-carboxybutyl)-*N*-(4-sulfobutyl)-2,3,5-trimethylindolenyne (compound 34) (80.7 mg; 0.22 mmole), acetic anhydride (2 mL), and a mixture (545 µL) of diisopropylethyl amine (200 µL; 269.5 mg,; 2.1 mmole) and acetic anhydride (800 µL; 739.4 mg; 7.2 mmole). *N,N*'-Disulfobutyl-5-carboxyethyl-3,3,3',3'-tetramethylindodicarbocyanine, sodium salt (compound B5) was obtained with yield of 104 mg (72%). For spectral characteristics see Table. Mass-spectrum (MALDI) (C₃₆H₄₅N₂O₈S₂⁻): found m/z 699.0, calculated m/z 697.88.

¹H-NMR (DMSO-d6), δ (ppm): 1.67 (12H, s, C(CH₃)₂), 1.75 (8H, m, CH₂CH₂CH₂CH₂SO₃), 2.36 (2H, t, J=7.5, CH₂CH₂COOH), 2.43 (4H, m, CH₂CH₂CH₂CH₂SO₃), 2.85 (2H, t, J=7.5, CH₂CH₂COOH), 4.06 (4H, m, NCH₂), 6.33 (2H, d, J=13.5, α,α'-CH), 6.58 (1H, t, J=12.0, γ-CH), 7.19-7.57 (7H, m, ArH), 8.28 (2H, m, β,β-CH)

### Example 73. Preparation of 5-carboxyethyl-5'-sulfo-N-sulfobutyl-3,3,3',3-tetramethyl-N'-ethylindodicarbocyanine, sodium salt (compound B6)

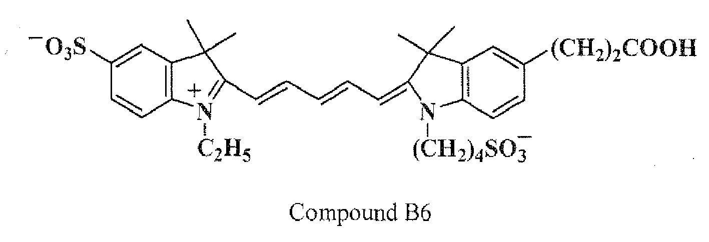

5-Carboxyethyl-5'sulfo-*N*-sulfobutyl-3,3,3',3'-tetramethyl-N'-ethylindodicarbocyanine, sodium salt (compound B6) was prepared following the synthesis of 3-(4-carboxybutyl)-*N*-(4-sulfobutyl)-3,3',3'-trimethyl-*N*'-ethylindodicarbocyanine (compound A1) using the reagents ratio as follows: (1^{st} step) 5-sulfo-2,3,3-trimethyl-*N*-ethylindolenyne (compound 29) (63.0 mg; 0.2 mmole), malonic dialdehyde dianil, hydrochloride (compound 37) (58.8 mg; 0.23 mole), acetic anhydride (1 mL), and acetyl chloride (64 µL); (2^{nd} step) 3-(4-carboxybutyl)-*N*-(4-sulfobutyl)-2,3,5-trimethylindolenyne (compound 34) (80.7 mg; 0.22 mmole), acetic anhydride (2 mL), and mixture (545 µL) of diisopropylethyl amine (200 µL; 269.5 mg; 2.1 mmole) and acetic anhydride (800 µL; 739.4 mg, 7.2 mmole). 5-Carboxyethyl-5'-sulfo-*N*-sulfobutyl-3,3,3',3'-tetramethyl-*N*'-ethylindodicarbocyanine, sodium salt (compound B6) was obtained with yield of 39 mg (28%). For spectral characteristics see Table. Mass-spectrum (MALDI) (C₃₄H₄₁N₂O₈S₂⁻): found m/z 670.8, calculated m/z 669.83.

¹H-NMR (DMSO-d6), δ (ppm): 1.23 (3H, t, J=7.0, CH₂CH₃), 1.67 (12H, s, C(CH₃)₂), 1.76 (4H, m, CH₂CH₂CH₂CH₂SO₃), 2.25 (2H, t, J=7.5, CH₂CH₂COOH), 2.47 (2H, m, CH₂CH₂CH₂CH₂SO₃), 2.83 (2H, t, J=7.5, CH₂CH₂COOH), 4.90 (4H, m, NCH₂), 6.32 (2H, d, J=13.5, α,α'-CH), 6.56 (1H, t, J=12.0, γ-CH), 7.23-7.62 (6H, m, ArH), 8.28 (2H, m, β,β'-CH).

### Example 74. Preparation of N,N'-disulfobutyl-5-carboxyethyl-5'-sulfo-3,3,3',3'-tetramethylindodicarbocyanine, disodium salt (compound B7)

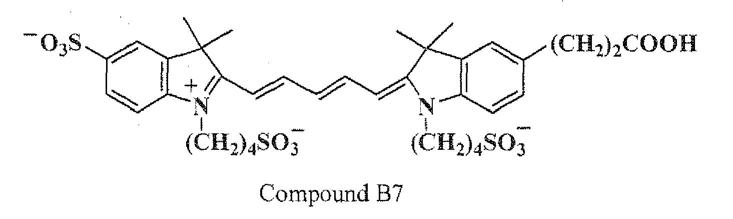

N,N'-Disulfobutyl-5-carboxyethyl-5'-sulfo-3.3,3',3'-tetramethylindodicarbocyanine, disodium salt (compound B7) was prepared following the synthesis of *N,N*'-diethyl-3-(4-carboxybutyl)-5-sulfo-3,3',3'.-trimethylindodiearbocyanine (compound A9) using the reagents ratio as follows: (1^{st} step) 5-sulfo-*N*-(4-sulfobutyl)-2,3,3-trimethylindolenyne, potassium salt (compound 28) (82.7 mg; 0.2 mmole), malonic dialdehyde dianil, hydrochloride (compound 37) (58.7 mg; 0.23 mmole), acetic anhydride (2 mL), and acetic acid (0.5 mL); (2^{nd} step) 3-(4-carboxybutyl)-*N*-(4-sulfobutyl)-2,3,5-trimethylindolenyne (compound 34) (80.7 mg; 0.22 mmole), anhydrous potassium acetate (120 mg; 1.2 mmole), acetic anhydride (1.5 mL), and acetic acid (1 mL). N,N'-Disulfobutyl-5-carboxyethyl-5'-sulfo-3.3,3',3'-tetramethylindodicarbocyanine, sodium salt (compound B7) was obtained with yield of 55 mg (33%). For spectral characteristics see Table. Mass-spectrum (MALDI) (C₃₆H₄₄N₂O₁₁S₃²⁻): found m/z 777.6, calculated m/z 776.94.

¹H-NMR (DMSO-d6), δ (ppm): 1.67 (12H, s, C(CH₃)₂), 1.75 (8H, m, CH₂CH₂CH₂CH₂SO₃), 2.28 (2H, t, J=7.5, CH₂CH₂COOH), 2.44 (4H, m, CH₂CH₂CH₂CH₂SO₃), 2.84 (2H, t, J=7.5, CH₂CH₂COOH), 4.09 (4H, m, NCH₂), 6.25-6.61 (2H, d, J=13.5, α,α'-CH), 6.58 (1H, t, J=12.0, γ-CH), 7.26-7.75 (6H, m, ArH), 8.25 (2H, m, β,β'-CH).

### Example 75. Preparation of sodium salt of indodicarbocyanine hydroxysuccinimide ester dyes (A1-A30)

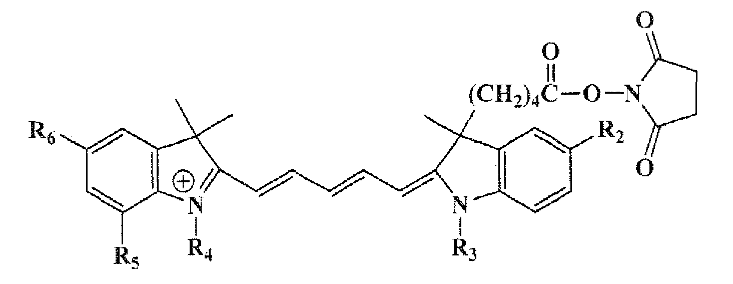

*N*-Hydroxysuccinimide (69.1 mg; 0.6 mole) was added to the solution of indodicarbocyanine, sodium salt (A1-A30) (0.15 mmole) in anhydrous dimethylformamide (DMF; 11 mL). The reaction mixture was cooled down to -18°C and the solution of dicyclohexyl carbodiimide (61.9 mg; 0.3 mmole) in anhydrous DMF (500 µL) was added dropwise. The mixture was stirred at room temperature for 5 days. Then, it was diluted with water and filtered off; the filtrate was applied to column RP-18, washed with water (100 mL), and the product was then eluted with a mixture acetonitrile-water (1:1). The solvent was removed and the residue was dried in vacuum desiccator over P₂O₅. Hydroxysuccinimide esters were obtained with yield of 75-80%.

### Example 76. Preparation of sodium salt of indodicarbocyanine hydroxysuccinimide ester dyes (B1-B7)

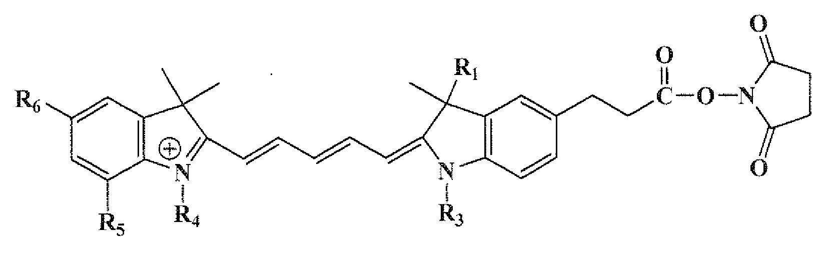

To solution of indodicarbocyanine, sodium salt (B1-B7) (0.1 mmole) in anhydrous dimethylformamide (10 mL), there were added di-(*N*-hydroxysuccinimidyl)carbonate (0.25 mmole) and pyridine (0.25 mmole). The reaction mixture obtained was stirred at room temperature for a period of day. Then, it was diluted with water and filtered off; the filtrate was applied to column RP-18, washed with water (100 mL), and the product was then eluted with a mixture acetonitrile-water (1:1). The solvent was removed under vacuum, and the residue was dried in vacuum desiccator over P₂O₅. Hydroxysuccinimide esters were obtained with yield about 75-80%.

### Example 77. Oligonucleotide labeling

To oligonucleotide 5-CTCAGTTT-NH₂-3' (200 nmole), there were added 0.1M carbonate-bicarbonate buffer (15 µL) and acetonitrile (15 µL); the mixture was thoroughly stirred and cooled down to -18°C for 5 min. Then, hydroxysuccinimide ester dye (A1-A30, B1-B7) (∼0.6 mg) was added, stirred, and allowed to hold for 24 h at room temperature. Whereupon 0.1 M TEAA (0.5 mL) was added and extracted with 1-butanol to discolour the upper layer completely. Oligonucleotide stained was isolated by means of HPLC, with control being performed by monitoring wavelength 254 nm; flow rate was 1 mL/min, gradient elution: from 20% of system B in system A to 100% B for 30 min, wherein A was 0.1M TEAA, B - 50% solution acetonitrile in A. Oligonucleotide stained was dried under vacuum at 40°C. Yield of labeled oligonucleotide was ∼70%.

## Claims

1. Compound of general formula (I) 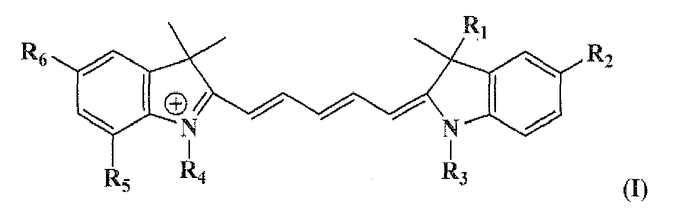 wherein:
R₁ is -CH₃; -(CH₂)₄COOH;
R₂ represents -CH₃; -(CH₂)₂COOH; -H; -SO₃⁻;
R₃ represents -C₂H₅; -(CH₂)₄SO₃⁻;
R₄ represents -C₂H₅; -(CH₂)₄SO₃⁻;
R₅ represents -H; -CH₃;
R₆ represents -H; -CH₃; -SO₃⁻; and
derivatives and salts thereof.

2. Compound according to claim 1 of general formula (A) 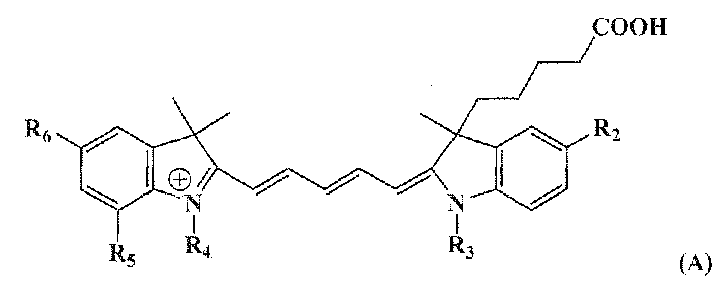 wherein:
R₁ is -(CH₂)₄COOH;
R₂ represents -H; -CH₃; -SO₃⁻;
R₃ represents -C₂H₅;-(CH₂)₄SO₃⁻
R₄ represents -C₂H₅; -(CH₂)₄SO₃⁻;
R₅ represents -H; -CH₃;
R₆ represents -H; -CH₃; -SO₃⁻; and
derivatives and salts thereof.

3. Compound according to claim 1 of general formula (B) as well as compounds being arbitrary considered as compounds of group **B** corresponding to general formula **(B)** 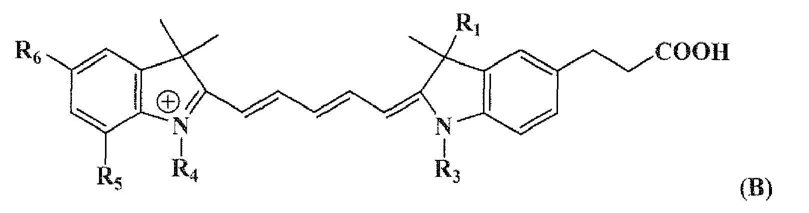 wherein:
R₁ is -CH₃;
R₂ is -(CH₂)₂COOH;
R₃ is -C₂H₅; -(CH₂)₄SO₃⁻;
R₄ is -C₂H₅; -(CH₂)₄SO₃⁻;
R₅ is -H;
R₆ is -H; -CH₃; -SO₃⁻; and
derivatives and salts thereof.

4. Compounds of general formula (I) according to claim 1 selected from the group consisting of:
3-(4-carboxybutyl)-*N*-(4-sulfobutyl)-3,3',3'-trimethyl-*N*'-ethylindodicarbocyanine (compound **A1**);
3-(4-carboxybutyl)-*N*-(4-sulfobutyl)-3,3',3',5'-tetramethyl-*N*'-ethylindodicarbocyanine (compound **A2**);
3-(4-carboxybutyl)-3,3',3',5',7'-pentamethyl-*N*-(4-sulfobutyl)-*N*'-ethylindodicarbocyanine (compound **A3**);
3-(4-carboxybutyl)-*N*-(4-sulfobutyl)-3,3',3',5-tetramethyl-*N*'-ethylindodicarbocyanine (compound **A4**);
3-(4-carboxybutyl)-3,3',3',5,5'-pentamethyl-*N*-(4-sulfobutyl)-*N*'-ethylindodicarbocyanine (compound **A5**);
3,3',3',5,5',7'-hexamethyl-3-(4-carboxybutyl)-*N*-(4-sulfobutyl)-*N*'-ethylindodicarbocyanine (compound **A6**);
*N,N*'-diethyl-3-(4-carboxybutyl)-5'-sulfo-3,3',3'-trimethylindodicarbocyanine (compound **A7**);
3-(4-carboxybutyl)-*N*'-(4-sulfobutyl)-3,3',3'-trimethyl-*N*-ethylindodicarbocyanine (compound **A8**);
*N,N*'-diethyl-3-(4-carboxybutyl)-5-sulfo-3,3',3'-trimethylindodicarbocyanine (compound **A9**);
*N,N*'-di(4-sulfobutyl)-3-(4-carboxybutyl)-3,3',3'-trimethylindodicarbocyanine (compound **A10**);
3-(4-carboxybutyl)-*N*-(4-sulfobutyl)-5'-sulfo-3,3',3'-trimethyl-*N*'-ethylindodicarbocyanine, sodium salt (compound **A11**);
*N,N*'-di(4-sulfobutyl)-3-(4-carboxybutyl)-3,3',3',5'-tetramethylindodicarbocyanine, sodium salt (compound **A12**);
*N,N*'-di(4-sulfobutyl)-3-(4-carboxybutyl)-3,3',3',5',7'-pentamethylindodicarbocyanine, sodium salt (compound **A13**);
3-(4-carboxybutyl)-5-sulfo-*N*-(4-sulfobutyl)-3,3',3'-trimethyl-*N*'-ethylindodicarbocyanine, sodium salt (compound **A14**);
3-(4-carboxybutyl)-5-sulfo-*N*-(4-sulfobutyl)-3,3',3',5'-tetramethyl-*N*'-ethylindodicarbocyanine, sodium salt (compound **A15**);
3-(4-carboxybutyl)-3,3',3',5',7'-pentamethyl-5-sulfo-*N*-(4-sulfobutyl)-*N*'-ethylindodicarbocyanine, sodium salt (compound **A16**);
*N,N*'-di(4-sulfobutyl)-3-(4-carboxybutyl)-3,3',3'.5-tetramethylindodicarbocyanine, sodium salt (compound **A17**);
3-(4-carboxybutyl)-*N*-(4-sulfobutyl)-5'-sulfo-3,3',3'.5-tetramethyl-*N*'-ethylindodicarbocyanine, sodium salt (compound **A18**);
*N,N*'-di(4-sulfobutyl)-3-(4-carboxybutyl)-3,3',3',5,5'-pentamethylindodicarbocyanine, sodium salt (compound **A19**);
3,3',3',5,5',7'-hexamethyl-*N,N*'-di(4-sulfobutyl)-3-(4-carboxybutyl)-indodicarbocyanine, sodium salt (compound **A20**);
3-(4-carboxybutyl)-*N*'-(4-sulfobutyl)-5-sulfo-3,3',3'-trimethyl-*N*-ethylindodicarbocyanine, sodium salt (compound **A21**);
5,5'-disulfo-*N,N*'-diethyl-3-(4-carboxybutyl)-3,3',3'-trimethylindodicarboeyaninea (compound **A22**);
*N,N*'-di(4-sulfobutyl)-3-(4-carboxybutyl)-5'-sulfo-3,3',3'-trimethylindodicarbocyanine, disodium salt (compound **A23**);
*N,N*'-di(4-sulfobutyl)-3-(4-carboxybutyl)-5-sulfo-3,3',3'-trimethylindodicarbocyanine, disodium salt (compound **A24**);
5,5'-disulfo-3-(4-carboxybutyl)-*N*-(4-sulfobutyl)-3,3',3'-trimethyl-*N*'-ethylindodicarbocyanine, disodium salt (compound **A25**);
*N,N*'-di(4-sulfobutyl)-3-(4-carboxybutyl)-5-sulfo-3,3',3',5'-tetramethylindodicarbocyanine, disodium salt (compound **A26**);
*N,N*'-di(4-sulfobutyl)-3-(4-carboxybutyl)-3,3',3',5',7'-pentamethyl-5-sulfoindodicarbocyanine, disodium salt (compound **A27**);
*N,N*'-di(4-sulfobutyl)-3-(4-carboxybutyl)-5'-sulfo-3,3',3'.*5*-tetramethylindodicarbocyanine, disodium salt (compound **A28**);
5,5'-disulfo-3-(4-carboxybutyl)-*N'*-(4-sulfobutyl)-3,3',3'-trimethyl-*N*-ethylindodicarbocyanine, disodium salt (compound **A29**);
5,5'-disulfo-*N,N*'-di(4-sulfobutyl)-3-(4-carboxybutyl)-3,3',3'-trimethylindodicarbocyanine, trisodium salt (compound **A30**);
5-carboxyethyl-*N*-sulfobutyl-3,3,3',3'-tetrarmethyl-*N*'-ethylindodicarbocyanine (compound **B1**);
*N,N*'-diethyl-5-carboxyethyl-5'-sulfo-3,3,3',3'-tetramethylindodicarbocyanine (compound **B2**);
5-carboxyethyl-*N*'-sulfobutyl-3,3,3',3'-tetramethyl-*N*-ethylindodicarbocyanine (compound **B3**);
*N,N*'-disulfobutyl-5-carboxyethyl-3,3,3',3',5'-pentamethylindodicarbocyanine, sodium salt (compound **B4**);
*N,N*'-disulfohutyl-5-carboxyethyl-3,3,3',3'-tetramethylindodicarbocyanine, sodium salt (compound **B5**);
5-carboxyethyl-5'-sulfo-*N*-sulfobutyl-3,3,3',3'-tetramethyl-N'-ethylindodicarbocyanine, sodium salt (compound **B6**);
N,N'-disulfobutyl-5-carboxyethyl-5'-sulfo-3,3,3',3'-tetramethylindodicarbocyanine, disodium salt (compound **B7**).

5. A method for producing compounds of general formula (I) comprising the following steps:
(a) heating indolenyne base and malonic dialdehyde dianil, hydrochloride in a mixture of acetic anhydride - acetic acid or in acetic anhydride with acetyl chloride being added;
(b) removal of solvents provided the second step being performed in the presence of diisopropylethyl amine;
(c) introduction of the second indolenyne base into the reaction mixture and carrying out the reaction on heating in a mixture of acetic anhydride-acetic acid matches the structure holding at room temperature for a period of day in a mixture of acetic anhydride-diisopropylethyl amine;
(d) isolation of the desired reaction product by reversed phase chromatography;
(e) preparation of indodicarbocyanines, sodium salts;
(f) preparation of appropriate *N*-hydroxysuccinimide or *para*-nitrophenyl esters of dyes.

6. A method according to claim 5 wherein step (a) heating and step (b) are performed at temperature 118°C for 2-3 hours.

7. A method according to claim 5 wherein said chromatography is performed using column RP-18 in a system acetonitrile-0.0 M triethylammonium acetate.

8. A use of compounds of general formula (I) as fluorescent labels for analysing biological macromolecules.

9. A use according to claim 8 wherein said biological macromolecule is the protein molecule (peptide, polypeptide).

10. A use according to claim 8 wherein said biological macromolecule is the nucleic acid (nucleotide sequence).

11. Intermediate (initial) compounds for synthesis of the compound according to the invention of formula (I) selected from the group consisting of:
2,3-dimethyl-3-(4-carboxybutyl)-indolenyne (compound **6**);
2,3-dimethyl-3-(4-carboxybutyl)-5-sulfoindolenyne, potassium salt (compound **8**);
3-(4-carboxybutyl)-2,3,5-trimethylindolenyne (compound **10**);
5-carboxyethyl-2,3,3-trimethylindolenyne (compound **20**);
2,3-dimethyl-3-(4-carboxybutyl)-*N*-ethylindolenyneum iodide (compound **21**);
2,3-dimethyl-3-(4-carboxybutyl)-*N*-(4-sulfobutyl)-indolenyne (compound **22**);
3-(4-carboxybutyl)-*N*-(4-sulfobutyl)-2,3,5-trimethylindolenyne (compound **23**);
2,3-dimethyl-3-(4-carboxybutyl)-5-sulfo-*N*-ethylindolenyne (compound **24**);
2,3-dimethyl-3-(4-carboxybutyl)-*N*-(4-sulfobutyl)-5-sulfoindolenyne, potassium salt (compound **25**);
5-carboxyethyl-*N*-sulfobutyl-2,3,3-trimethylindolenyne (compound **34**);
5-carboxyethyl-2,3,3-trimethyl-*N*-ethylindolenyneum iodide (compound **35**).
